(19) Europäisches Patentamt
European Patent Office
Office européen des brevets

(11) Publication number: **0 686 635 A1**

(12) **EUROPEAN PATENT APPLICATION**

(21) Application number: **95113591.2**

(22) Date of filing: **24.12.92**

(51) Int. Cl.[6]: **C07D 417/04**, C07D 417/14, A61K 31/44, //(C07D417/04, 277:00,213:00)

This application was filed on 30 - 08 - 1995 as a divisional application to the application mentioned under INID code 60.

(30) Priority: **27.12.91 JP 345736/91**
**13.03.92 JP 54698/92**

(43) Date of publication of application:
**13.12.95 Bulletin 95/50**

(60) Publication number of the earlier application in accordance with Art.76 EPC: **0 549 364**

(84) Designated Contracting States:
**AT BE CH DE DK ES FR GB GR IE IT LI LU MC NL PT SE**

(71) Applicant: **Sankyo Company Limited**
**5-1 Nihonbashi Honcho 3-chome**
**Chuo-ku**
**Tokyo (JP)**

(72) Inventor: **Fukumi, Hiroshi, c/o Sankyo Company Limited**
**2-58, Hiromachi 1-chome,**
**Shinagawa-ku**
**Tokyo (JP)**
Inventor: **Sakamoto, Toshiaki, c/o Sankyo Company Limited**
**2-58, Hiromachi 1-chome,**
**Shinagawa-ku**
**Tokyo (JP)**
Inventor: **Sugiyama, Mitsuo, c/o Sankyo Company Limited**
**2-58, Hiromachi 1-chome,**
**Shinagawa-ku**
**Tokyo (JP)**
Inventor: **Yamaguchi, Takeshi, c/o Sankyo Company Limited**
**2-58, Hiromachi 1-chome,**
**Shinagawa-ku**
**Tokyo (JP)**
Inventor: **Asai, Fumitoshi, c/o Sankyo Company Limited**
**2-58, Hiromachi 1-chome,**
**Shinagawa-ku**
**Tokyo (JP)**
Inventor: **Iijima, Yasuteru, c/o Sankyo Company Limited**
**2-58, Hiromachi 1-chome,**
**Shinagawa-ku**
**Tokyo (JP)**

(74) Representative: **Gibson, Christian John Robert**
**MARKS & CLERK,**
**57/60 Lincoln's Inn Fields**
**London WC2A 3LS (GB)**

(54) **Pyridylthiazolidinecarboxylic acid amide derivatives, their preparation and their therapeutic uses**

(57) New pyridylthiazolinecarboxylic acid amide compounds have been found to have a valuable combination of antiallergic and antiasthmatic activities with an antagonist activity against Platelet Activating Factor.

EP 0 686 635 A1

The present invention relates to a series of new pyridylthiazolinecarboxylic acid amide compounds which we have found to have a valuable combination of antiallergic and antiasthmatic activities with an antagonist activity against Platelet Activating Factor (PAF).

A number of compounds having anti-allergic activities are known, and it is also known that compounds having a heterocyclylalkylamide structure have anti-allergic activity [see, for example, US Patent No. 4 965 266, Chemical Pharmaceutical Bulletin, 37, p. 1256 (1989), etc.].

PAF (platelet activating factor) exhibits a strong platelet activating and aggregating effect, from which it derives its name. It has, however, in recent years been seen to be a potentially crucial mediator in a wide variety of pathological processes. Thus, it also has a hypotensive effect and increases vasopermeability; it is believed to be an active agent in the induction of the shock state (for example endotoxin-induced shock or anaphylactic shock) and to act as a mediator of inflammatory disease. It has also been found to play an important role in nephritis, myocardial infarction, angina pectoris, asthma, cardiac and systemic anaphylaxis, gastric and intestinal ulceration, psoriasis and immune and renal disorders. In addition, PAF inhibitors also inhibit eosinophile accumulation and could, therefore, be used for the treatment of late allergic reactions.

However, although it has recently appeared to us that it would be desirable to develop anti-allergic agents which not only have anti-allergic activity but also have activities which cooperate with the anti-PAF activity, no drugs satisfying this demand have yet been put on the market, and most of the known drugs of this type do not possess both kinds of activity.

For example, Japanese Patent Application Kokai No. Hei 2-179 (equivalent to Australian Patent No. 9214013) discloses a number of pyridylthiazolinecarboxylic acid amide compounds which are said to be PAF antagonists, such as the compound of formula (A):

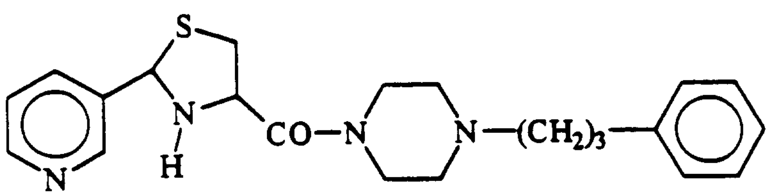

(A)

However, the antiallergic activity of these compounds is very weak.

However, the closest prior compounds are believed to be those disclosed in European Patent Publication No. 463 873, published after the priority date hereof, which are pyridylthiazolinecarboxylic acid amide compounds having both PAF antagonist and antiallergic activities. These prior compounds, however, differ from the compounds of the present invention in the nature of the group or groups attached to the carbonyl group of the carboxylic amide moiety.

The compounds of the present invention are those pyridylthiazolidinecarboxylic acid amide derivatives of formula (I):

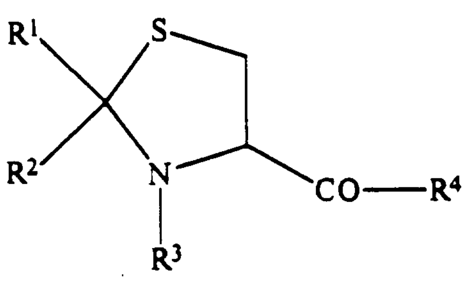

(I)

in which:

$R^1$ represents a pyridyl group which is unsubstituted or is substituted by at least one substituent selected from alkyl groups having from 1 to 4 carbon atoms and alkoxy groups having from 1 to 4 carbon atoms;

$R^2$ represents a hydrogen atom, or a pyridyl group which is unsubstituted or is substituted by at least one

substituent selected from alkyl groups having from 1 to 4 carbon atoms and alkoxy groups having from 1 to 4 carbon atoms;

$R^3$ represents a hydrogen atom or an alkyl group having from 1 to 4 carbon atoms;

$R^4$ represents a group of formula (II), (III), (IV), (V) or (VI):

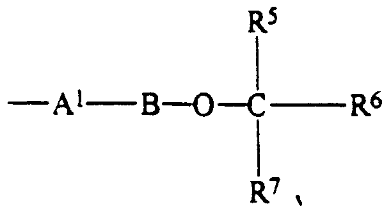

(II)

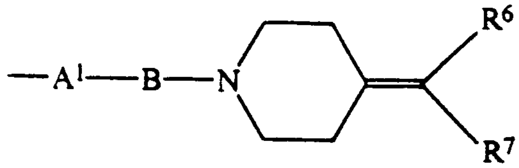

(III)

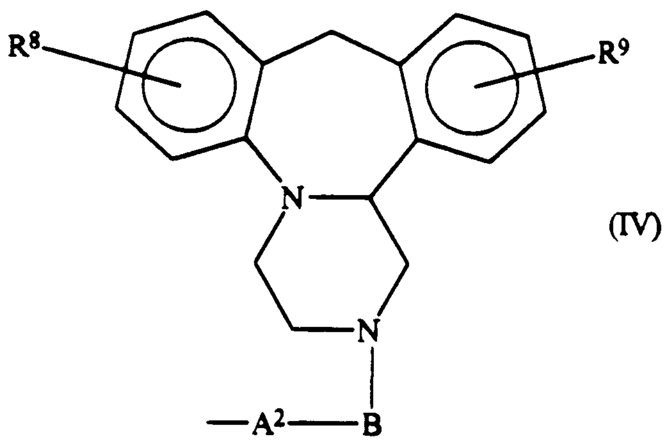

(IV)

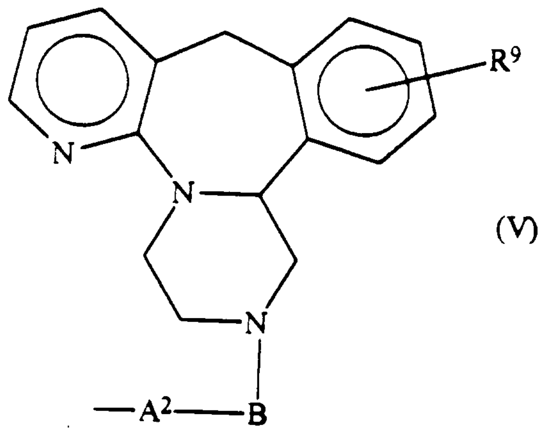

(V)

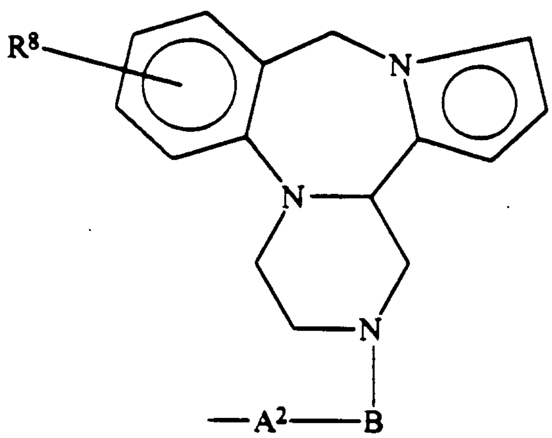

(VI)

in which:

$R^5$ represents a hydrogen atom or an alkyl group having from 1 to 4 carbon atoms;

$R^6$ and $R^7$ are the same or different and each represents:

an unsubstituted phenyl group,

a substituted phenyl group which is substituted by at least one substituent selected from alkyl groups having from 1 to 4 carbon atoms, haloalkyl groups having from 1 to 4 carbon atoms and having at least one halogen atom, alkoxy groups having from 1 to 4 carbon atoms and halogen atoms,

a cycloalkyl group having from 3 to 6 ring carbon atoms, and

an aromatic heterocyclic group having 5 or 6 ring atoms of which from 1 to 3 are nitrogen, and/or oxygen and/or sulphur hetero-atoms and the remainder are carbon atoms;

$R^8$ and $R^9$ are the same or different and each represents a hydrogen atom, an alkyl group having from 1 to 4 carbon atoms, a haloalkyl group having from 1 to 4 carbon atoms and having at least one halogen atom, an alkoxy group having from 1 to 4 carbon atoms, a hydroxy group or a halogen atom;

$A^1$ represents a group of formula (VII) or (VIII):

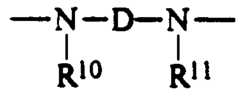 (VII)

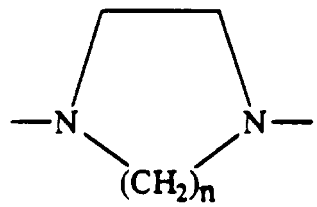 (VIII)

where:
$R^{10}$ and $R^{11}$ are the same or different and each represents a hydrogen atom or an alkyl group having from 1 to 4 carbon atoms;
D represents an alkylene or alkylidene group having from 2 to 5 carbon atoms; and
n is 2 or 3;
$A^2$ represents a group of formula (VIII), above, or (IX):

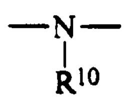 (IX)

where $R^{10}$ is as defined above; and
B represents an alkylene or alkylidene group having from 2 to 4 carbon atoms; or
$-A^2-B-$ represents a single bond;
and pharmaceutically acceptable salts thereof.

The invention also provides a composition for the treatment or prophylaxis of histamine- or PAF- related disorders, such as allergies or asthma, in a mammal, e.g. a human being, which comprises an effective amount of an anti-histamine or anti-PAF agent in admixture with a pharmaceutically acceptable carrier or diluent, in which the anti-histamine or anti-PAF agent is at least one compound selected from compounds of formula (I) and pharmaceutically acceptable salts thereof.

The invention also provides the use of at least one compound of formula (I) or a pharmaceutically acceptable salt thereof, as defined above, in therapy.

The invention also provides the use of at least one compound of formula (I) or a pharmaceutically acceptable salt thereof, as defined above, for the manufacture of a medicament for the treatment or prophylaxis of histamine-related disorders, such as allergies or asthma, in a mammal, e.g. a human being.

The invention also provides novel processes for the preparation of the compounds of the present invention, which processes are described in more detail hereafter.

In the compounds of the present invention, where the substituent on the pyridyl group represented by $R^1$ or $R^2$ is an alkyl group, or where $R^3$, $R^5$, $R^8$, $R^9$, $R^{10}$ or $R^{11}$ represents an alkyl group, or where the substituent on the substituted phenyl group represented by $R^6$ or $R^7$ is an alkyl group, this may be a straight or branched chain alkyl group having from 1 to 4 carbon atoms. Examples of such groups include the methyl, ethyl, propyl, isopropyl, butyl, isobutyl, sec-butyl and t-butyl groups, of which the methyl, ethyl, propyl, isopropyl, butyl and sec-butyl groups are preferred, the methyl and ethyl groups being more preferred, and the methyl group being most preferred.

Where the substituent on the pyridyl group represented by $R^1$ or $R^2$ is an alkoxy group, or where $R^8$ or $R^9$ represents an alkoxy group, or where the substituent on the substituted phenyl group represented by $R^6$ or $R^7$ is an alkoxy group, this may be a straight or branched chain alkoxy group having from 1 to 4 carbon atoms. Examples of such groups include the methoxy, ethoxy, propoxy, isopropoxy, butoxy, isobutoxy, sec-butoxy and t-butoxy groups, of which the methoxy, ethoxy, propoxy, isopropoxy, butoxy and isobutoxy groups are preferred, the methoxy and ethoxy groups being more preferred, and the methoxy group being most preferred.

Where $R^1$ or $R^2$ represents a substituted pyridyl group, the number of substituents is limited only by the number of positions capable of being substituted (i.e. 4), and possibly by steric constraints. In general, from 1 to 3 substituents are preferred, 1 or 2 substituents being more preferred.

The pyridyl groups may be 2-, 3- or 4- pyridyl groups.

$R^1$ is preferably an unsubstituted pyridyl group or a substituted pyridyl group having at least one alkyl substituent which has from 1 to 4 carbon atoms, and is more preferably a pyridyl group having 0 or 1 such substituent, and is most preferably an unsubstituted pyridyl group.

$R^2$ is preferably a hydrogen atom.

$R^3$ is preferably a hydrogen atom, a methyl group or an ethyl group, more preferably a hydrogen atom or a methyl group.

$R^5$ is preferably a hydrogen atom, a methyl group or an ethyl group, more preferably a hydrogen atom or a methyl group, and most preferably a hydrogen atom.

Where $R^8$ or $R^9$ or the substituent on the phenyl group represented by $R^6$ or $R^7$ is a haloalkyl group, the alkyl part may be a straight or branched chain alkyl group having from 1 to 4 carbon atoms. This is substituted by one or more halogen atoms, for example fluorine, chlorine, bromine or iodine atoms. There is no particular limitation on the number of halogen substituents, except such as may be imposed by the number of substitutable positions and possibly by steric constraints. Thus, the maximum number of halogen atoms is 3 for the methyl group, 5 for the ethyl group, 7 for the propyl groups and 9 for the butyl groups. However, in general, from 1 to 5 halogen atoms are preferred (except for halomethyl groups, where from 1 to 3 are preferred), from 1 to 3 halogen atoms being more preferred. Examples of such haloalkyl groups include the fluoromethyl, chloromethyl, bromomethyl, iodomethyl, 2-fluoroethyl, 2-chloroethyl, 3-fluoropropyl, 3-chloropropyl, 4-fluorobutyl, 4-chlorobutyl, trifluoromethyl, trichloromethyl, 2,2,2-trifluoroethyl and 2,2,2-trichloroethyl groups, of which we prefer the fluoromethyl, chloromethyl and trifluoromethyl groups, more preferably the trifluoromethyl group.

$R^{10}$ and $R^{11}$ are the same or different and each preferably represents a hydrogen atom, a methyl group or an ethyl group, more preferably a methyl group or an ethyl group.

Where $R^8$ or $R^9$ or the substituent on the phenyl group represented by $R^6$ or $R^7$ is a halogen atom, this may be, for example, a fluorine, chlorine, bromine or iodine atom, preferably a fluorine or chlorine atom.

$R^8$ and $R^9$ are the same or different and each preferably represents a hydrogen atom, a halogen atom, a methyl group, a methoxy group, an ethyl group, an ethoxy group, a trifluoromethyl group or a hydroxy group. Where $R^8$ or $R^9$ is other than a hydrogen atom, it is preferably at the 7-, 8-, 12- or 13- position of the group of formula (IV), (V) or (VI).

Where $R^6$ or $R^7$ represents a cycloalkyl group, this has from 3 to 6 ring carbon atoms, and examples include the cyclopropyl, cyclobutyl, cyclopentyl and cyclohexyl groups, of which we prefer the cyclopentyl and cyclohexyl groups.

Where $R^6$ or $R^7$ represents a heterocyclic group, this is an aromatic heterocyclic group containing 5 or 6 ring atoms, of which from 1 to 3, preferably 1 or 2, are nitrogen, and/or oxygen and/or sulphur hetero-atoms. Where there are three hetero-atoms, these may be the same or different and they may be selected from nitrogen, oxygen and sulphur atoms; however, more preferably one or two are nitrogen atoms and the other is a nitrogen, oxygen or sulphur atom. Where there are two hetero-atoms, these may be the same or different and they may be selected from nitrogen, oxygen and sulphur atoms; however, more preferably one is a nitrogen atom and the other is a nitrogen, oxygen or sulphur atom. Most preferably, there is a single hetero-atom. Examples of such groups include the furyl, thienyl, pyrrolyl, pyridyl, thiazolyl, isothiazolyl, oxazolyl, isoxazolyl, imidazolyl, pyrazolyl, pyrazinyl, pyridazinyl, pyrimidinyl and furazanyl groups, preferably the pyrrolyl, pyridyl, pyrimidinyl, pyrazinyl, furyl, thienyl, oxazolyl, isoxazolyl, thiazolyl, isothiazolyl or imidazolyl groups, and more preferably the furyl, thienyl and pyridyl groups.

B represents an alkylene or alkylidene group having from 2 to 4 carbon atoms, which may be a straight or branched chain group. The bonds of the alkylene group by which it is attached, on the one hand, to the group represented by $A^1$ or $A^2$ and, on the other hand, to remainder of the molecule may be on the same carbon atom or on different carbon atoms. Where the bonds are on the same carbon atom, the groups are sometimes referred to as "alkylidene groups". It is, however, conventional to use the general term "alkylene group" to include both those groups where the bonds are on the same carbon atom and those where they are on different carbon atoms. Examples of such groups include the ethylene, ethylidene, trimethylene, propylene, isopropylidene, tetramethylene and 2-methyltrimethylene groups, preferably the ethylene, trimethylene, propylene, tetramethylene and 2-methyltrimethylene groups, more preferably the ethylene and trimethylene groups, most preferably the ethylene group.

D represents an alkylene or alkylidene group having from 2 to 5 carbon atoms, which may be a straight or branched chain group. Examples of such groups include the ethylene, ethylidene, trimethylene,

propylene, isopropylidene, tetramethylene, 2-methyltrimethylene and pentamethylene groups, preferably the ethylene, trimethylene, propylene, tetramethylene, 2-methyltrimethylene and pentamethylene groups, more preferably the ethylene, trimethylene or tetramethylene group.

Where $R^4$ represents a group of formula (II):

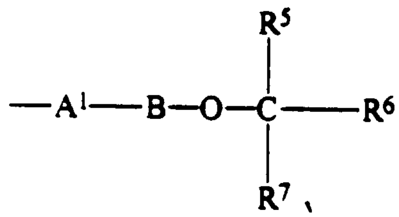

(II)

preferred examples of the group represented by the formula (X):

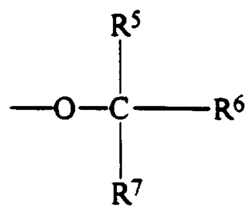

(X)

include the diphenylmethoxy, α-(o-, m- and p- fluorophenyl)benzyloxy, α-(o-, m- and p- chlorophenyl)-benzyloxy, bis(o-, m- and p- fluorophenyl)methoxy, α-(o-, m-and p- fluorophenyl)- o-, m- and p- chlorobenzyloxy, α-(o-, m- and p- fluorophenyl)-o-, m- and p- methylbenzyloxy, α-(o-, m- and p- fluorophenyl)-o-, m- and p-methoxybenzyloxy, bis(o-, m- and p- chlorophenyl)methoxy, α-(o-, m- and p- chlorophenyl)-o-, m- and p- methylbenzyloxy, α-(o-, m- and p- chlorophenyl)-o-, m- and p-methoxybenzyloxy, α-(o-, m- and p-methoxyphenyl)benzyloxy, bis(o-, m- and p- methoxyphenyl)methoxy, α-(o-, m-and p- methylphenyl)-benzyloxy, di(o-, m- and p- methylphenyl)methoxy, α-(2-, 3- or 4- pyridyl)benzyloxy, α-(2-, 3- or 4- pyridyl)-o-, m- and p- methylbenzyloxy, α-(2-, 3- or 4- pyridyl)-o-, m- and p- methoxybenzyloxy, α-(2-, 3- or 4-pyridyl)-o-, m- and p- trifluoromethylbenzyloxy, α-(2-, 3- or 4- pyridyl)-o-, m- and p-chlorobenzyloxy, α-(2-, 3- or 4- pyridyl)-o-, m- and p-fluorobenzyloxy, α-(2- or 3- thienyl)benzyloxy, α-(2-or 3- thienyl)-o-, m- and p-methylbenzyloxy, α-(2- or 3- thienyl)-o-, m- and p- methoxybenzyloxy, α-(2- or 3-thienyl)-o-, m- and p-trifluoromethylbenzyloxy, α-(2-or 3- thienyl)-o-, m- and p- chlorobenzyloxy, α-(2- or 3- thienyl)-o-, m- and p-fluorobenzyloxy, α-(2- or 3-furyl)benzyloxy, α-(2- or 3- furyl)-o-, m- and p-methylbenzyloxy, α-(2- or 3-furyl)-o-, m- and p-methoxybenzyloxy, α-(2- or 3- furyl)-o-, m- and p-trifluoromethylbenzyloxy, α-(2- or 3-furyl)-o-, m- and p- chlorobenzyloxy, α-(2- or 3- furyl)-o-, m- and p-fluorobenzyloxy, α-(2-, 3- or 4- pyridyl)-α-(2- or 3-thienyl)methoxy, α,α-di(2-, 3- or 4- pyridyl)methoxy, α-(2-, 3- or 4- pyridyl)-α-(2- or 3- furyl)-methoxy, α-cyclohexylbenzyloxy, α-cyclohexyl-o-, m- and p-methylbenzyloxy, α-cyclohexyl-o-, m- and p-methoxybenzyloxy, α-cyclohexyl-o-, m- and p- trifluoromethylbenzyloxy, α-cyclohexyl-o-, m- and p-chlorobenzyloxy, α-cyclohexyl-o-, m- and p- fluorobenzyloxy, α-cyclohexyl-α-(2- or 3- thienyl)methoxy, α-cyclohexyl-α-(2- or 3- furyl)methoxy, 4-(diphenylmethylene)piperidinyl, 4-[α-(o-, m- and p- fluorophenyl)-α-phenylmethylene]piperidinyl, 4-[α,α-bis(o-, m- and p-fluorophenyl)methylene]piperidinyl, 4-[α-(o-, m- and p-chlorophenyl)-α-phenylmethylene]piperidinyl, 4-[α,α-bis(o-, m- and p- chlorophenyl)methylene]piperidinyl, 4-[α-(o-, m- and p- methylphenyl)-α-phenylmethylene]-piperidinyl, 4-[α,α-di(o-, m- and p- methylphenyl)-methylene]piperidinyl, 4-[α-(o-, m- and p- methoxyphenyl)-α-(o-, m- and p- methylphenyl)methylene]-piperidinyl, 4-[α,α-bis(o-, m- and p- methoxyphenyl)methylene]piperidinyl, 4-[α-(o-, m- and p- methoxyphenyl)-α-phenylmethylene]piperidinyl, 4-[α-(2-, 3- or 4- pyridyl)-α-phenylmethylene]piperidinyl, 4-[α-(2-, 3- or 4- pyridyl)-α-(o-, m- and p- methylphenyl)methylene]piperidinyl, 4-[α-(2-, 3- or 4- pyridyl)-α-(o-, m- and p-methoxyphenyl)methylene]piperidinyl, 4-[α-(2-, 3- or 4- pyridyl)-α-(o-, m- and p-trifluoromethylphenyl)-methylene]piperidinyl, 4-[α-(2-, 3- or 4- pyridyl)-α-(o-, m- and p- chlorophenyl)methylene]piperidinyl, 4-[α-(2-, 3- or 4- pyridyl)α-(o-, m- and p- fluorophenyl)methylene]piperidinyl, 4-[α-(2- or 3- thienyl)-α-phenylmethylene]piperidinyl, 4-[α-(2- or 3- thienyl)-α-(o-, m- and p- methylphenyl)methylene]piperidinyl, 4-[α-(2- or

3- thienyl)-α-(o-, m- and p- methoxyphenyl)methylene]piperidinyl, 4-[α-(2- or 3- thienyl)-α-(o-, m- and p-trifluoromethylphenyl)methylene]piperidinyl, 4-[α-(2- or 3-thienyl)-α-(o-, m- and p- chlorophenyl)methylene]-piperidinyl, 4-[α-(2- or 3- thienyl)-α-(o-, m- and p-fluorophenyl)methylene]piperidinyl, 4-[α-(2- or 3-furyl)-α-phenylmethylene]piperidinyl, 4-[α-(2- or 3-furyl)-α-(o-, m- and p- methylphenyl)methylene]piperidinyl, 4-[α-(2- or 3- furyl)-α-(o-, m- and p-methoxyphenyl)methylene]piperidinyl, 4-[α-(2- or 3-furyl)-α-(o-, m- and p-trifluoromethylphenyl)methylene]piperidinyl, 4-[α-(2- or 3- furyl)-α-(o-, m-and p- chlorophenyl)methylene]-piperidinyl, 4-[α-(2- or 3- furyl)-α-(o-, m- and p- fluorophenyl)methylene]piperidinyl, 4-[α-(2-, 3- or 4-pyridyl)-α-(2- or 3-thienyl)methylene]piperidinyl, 4-[α,α-di(2-, 3- or 4-pyridyl)methylene]piperidinyl, 4-[α-(2-, 3- or 4-pyridyl)-α-(2- or 3- furyl)methylene]piperidinyl, 1,1-diphenylethoxy, 1,1-bis(o-, m- and p-fluorophenyl)ethoxy, α-(o-, m- and p- fluorophenyl)-α-methylbenzyloxy, α-(o-, m- and p- chlorophenyl)-α-methylbenzyloxy, α-(o-, m- and p- chlorophenyl)-α-methyl-o-, m- and p- chlorobenzyloxy, α-(o-, m- and p-methylphenyl)-α-methylbenzyloxy, α-(o-, m- and p- methylphenyl)-α-methyl-o-, m- and p- methylbenzyloxy, α-(o-, m- and p- methoxyphenyl)-α-methylbenzyloxy, α-(2-, 3-or 4- pyridyl)-α-methylbenzyloxy, α-(2-, 3- or 4-pyridyl)-α-methyl-o-, m- and p- chlorobenzyloxy, α-(2-, 3- or 4- pyridyl)-α-methyl-o-, m- and p-fluorobenzyloxy, α-(2- or 3- thienyl)-α-methylbenzyloxy, α-(2- or 3- thienyl)-α-methyl-o-, m- and p-chlorobenzyloxy, α-(2- or 3- thienyl)-α-methyl-o-, m-and p- fluorobenzyloxy, α-(2- or 3- furyl)-α-methylbenzyloxy, α-(2- or 3-furyl)-α-methyl-o-, m- and p-chlorobenzyloxy and α-(2- or 3- furyl)-α-methyl-o-, m-and p- fluorobenzyloxy groups.

More preferred groups which may be represented by the formula (X) include the diphenylmethoxy, bis-(o-, m- and p-fluorophenyl)methoxy, α-(o-, m- and p- fluorophenyl)-o-, m- and p- chlorobenzyloxy, bis(o-, m- and p- chlorophenyl)methoxy, α-(o-, m- and p- chlorophenyl)benzyloxy, α-(o-, m- or p- fluorophenyl)-benzyloxy, α-(o-, m- or p-methylphenyl)benzyloxy, α-(2-, 3- or 4- pyridyl)benzyloxy, (2-, 3- or 4- pyridyl)-o-, m- and p- chlorobenzyloxy, (2-, 3- or 4- pyridyl)-o-, m- and p- fluorobenzyloxy, 4-(diphenylmethylene)-piperidinyl, 4-[α-(o-, m- and p-fluorophenyl)-α-phenylmethylene]piperidinyl, 4-[α,α-bis(o-, m- and p-fluorophenyl)-methylene]piperidinyl, 4-[α-(o-, m- and p- chlorophenyl)-α-phenylmethylene]-piperidinyl, 4-[α-(2- or 3- thienyl)-α-phenylmethylene]piperidinyl, 4-(2- or 3- thienyl)-α-(o-, m-and p- fluorophenyl)methylene]-piperidinyl, 1,1-diphenylethoxy, 1,1-bis(o-, m- and p- fluorophenyl)ethoxy, 1-(o-, m- and p- fluorophenyl)-1-phenylethoxy and α-(o-, m- and p- chlorophenyl)-α-methylbenzyloxy groups.

Still more preferred groups which may be represented by the formula (X) include the diphenylmethoxy, bis(o-, m-and p- fluorophenyl)methoxy, α-(o-, m- and p- fluorophenyl)-o-, m- and p- chlorobenzyloxy, bis(o-, m- and p-chlorophenyl)methoxy, α-(o-, m- and p- chlorophenyl)benzyloxy, α-(o-, m- or p- fluorophenyl)-benzyloxy, α-(o-, m- or p- methylphenyl)benzyloxy, α-(2-, 3- or 4- pyridyl)benzyloxy, α-(2-, 3- or 4- pyridyl)-o-, m-and p- chlorobenzyloxy and α-(2-, 3- or 4- pyridyl)-o-, m- and p- fluorobenzyloxy groups.

The compounds of the present invention contain at least one basic nitrogen atom in their molecules, and can, therefore, form acid addition salts. There is no particular limitation on the nature of the acid employed to form such salts, provided that, where the salt is to be used for therapeutic purposes, it is pharmaceutically acceptable, that is it is not more toxic (or unacceptably more toxic) or less active (or unacceptably less active) than the parent compound. On the other hand, where the salt is to be used for other purposes, e.g. for the preparation of other, and possibly more active, compounds, even this restriction does not apply. Examples of such acid addition salts include: salts with mineral acids, especially hydrohalic acids (such as hydrofluoric acid, hydrobromic acid, perchloric acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid. Of these salts, we prefer the hydrochlorides, fumarates, oxalates and maleates.

The compounds of the present invention necessarily contain several asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques. We particularly prefer those compounds in which the carbon atom at the 4-position of the thiazolidine ring has the R-configuration.

A preferred class of compounds of the present invention are those compounds of formula (I) in which:
$R^1$, $R^2$ and $R^3$ are as defined above;

$R^4$ represents a group of formula (II) or (III), in which $R^5$, $R^6$, $R^7$, $A^1$ and B are as defined above; and pharmaceutically acceptable salts thereof.

An alternative preferred class of compounds of the present invention are those compounds of formula (I) in which:

$R^1$, $R^2$ and $R^3$ are as defined above;

$R^4$ represents a group of formula (IV), (V) or (VI), in which $R^8$, $R^9$, $A^2$ and B are as defined above; and pharmaceutically acceptable salts thereof.

More preferred compounds of the present invention are those compounds of formula (I) and salts thereof in which:

(A) $R^1$ represents a pyridyl group.

(B) $R^2$ represents a hydrogen atom, a methyl group or a pyridyl group (particularly a hydrogen atom);

(C) $R^3$ represents a hydrogen atom or a methyl group (particularly a hydrogen atom).

(D) $A^1$ represents a group of formula (VII) or (VIII), in which $R^{10}$ and $R^{11}$ are the same or different, and each represent a methyl group or an ethyl group; D represents an ethylene, trimethylene or tetramethylene group; and n is 2 or 3. More preferably: $R^{10}$ and $R^{11}$ are the same and each represents a methyl group; D represents an ethylene group or a trimethylene group; and n is 2.

(E) B represents an ethylene group or a trimethylene group, particularly an ethylene group.

(F) The group of formula (X) represents a bis(o-, m- or p- fluorophenyl)methoxy, α-(o-, m- or p-chlorophenyl)benzyloxy, bis(o-, m- or p- fluorophenyl)methoxy, α,α-diphenylmethoxy, α-(o-, m- or p-fluorophenyl)benzyloxy, α-(o-, m- or p- methylphenyl)benzyloxy, α-(2-, 3- or 4- pyridyl)benzyloxy, 4-[α-(2- or 3- thienyl)-α-phenylmethylene]piperidinyl or 4-[α,α-bis(o-, m- or p- fluorophenyl)methylene]piperidinyl group, particularly, a bis(4-fluorophenyl)methoxy, α-(4-chlorophenyl)benzyloxy, bis(4-fluorophenyl)-methoxy, diphenylmethoxy or α-(2-pyridyl)benzyloxy group.

(G) $R^4$ represents a group of formula (IV) or (VI), in which $R^8$ and $R^9$ are the same or different, and each represents a hydrogen atom or a halogen atom, particularly a group of formula (IV), in which $R^8$ and $R^9$ are the same or different and each represents a hydrogen atom, a fluorine atom or a chlorine atom.

(H) $A^2$ represents a group of formula (IX) or (XI):

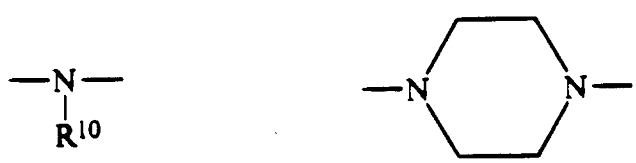

(IX) (XI)

in which $R^{10}$ represents a hydrogen atom or an alkyl group having from 1 to 4 carbon atoms, particularly a group of formula (IX), in which $R^{10}$ represents a hydrogen atom or a methyl group.

(I) B represents an ethylene group or a trimethylene group, or $-A^2$-B- represents a single bond, in particular, we prefer that B should represent an ethylene group.

Still more preferred are those compounds in which $R^1$ is as defined in (A) above, $R^2$ is as defined in (B) above, $R^3$ is as defined in (C) above, $A^1$ is as defined in (D) above, B is as defined in (E) above, the group of formula (X) is as defined in (F) above, and $R^4$ is as defined in (G) above, or those compounds in which $R^1$ is as defined in (A) above, $R^2$ is as defined in (B) above, $R^3$ is as defined in (C) above, $A^2$ is as defined in (H) above, and B is as defined in (I) above.

Specific examples of compounds of the invention are those compounds having the following formulae (I-1) to (I-12), in which the substituents are as defined in the respective one of Tables 1 to 12, i.e. Table 1 relates to formula (I-1), Table 2 relates to formula (I-2), Table 3 relates to formula (I-3), and so on. In the Tables the following abbreviations are used are used for certain groups; otherwise, standard internationally recognised symbols are used to designate atoms:

| | |
|---|---|
| Dme | 5,6-dimethyl |
| Et | ethyl |
| cHx | cyclohexyl |
| Me | methyl |
| Ph | phenyl |
| Py | pyridyl |

Tfm trifluoromethyl
Tm trimethylene
Thi 2-thienyl

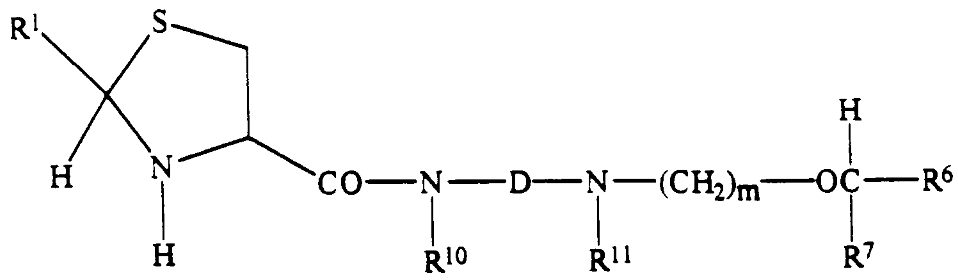
(I-1)

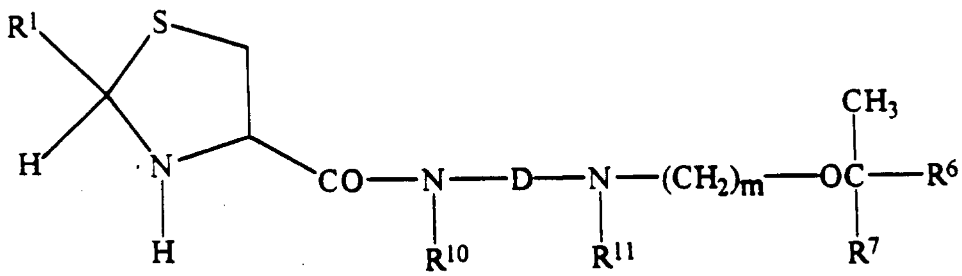
(I-2)

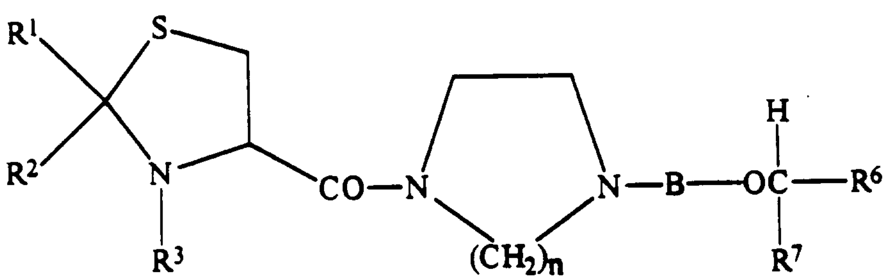
(I-3)

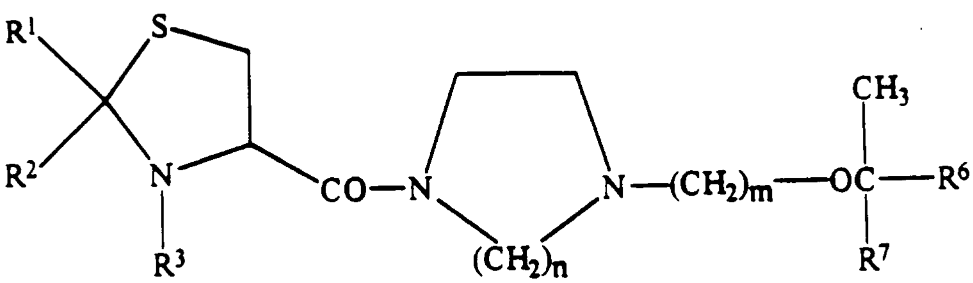
(I-4)

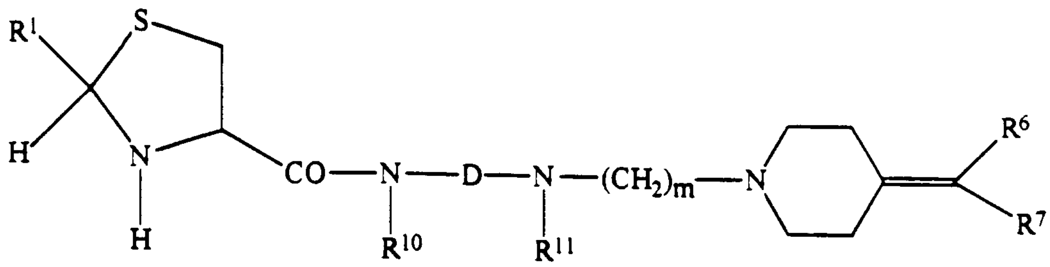

(I-5)

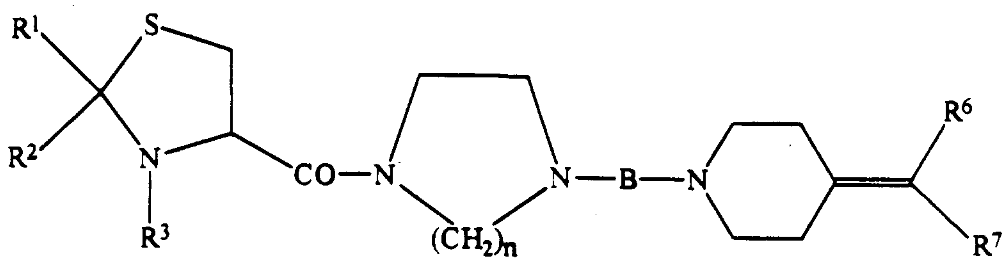

(I-6)

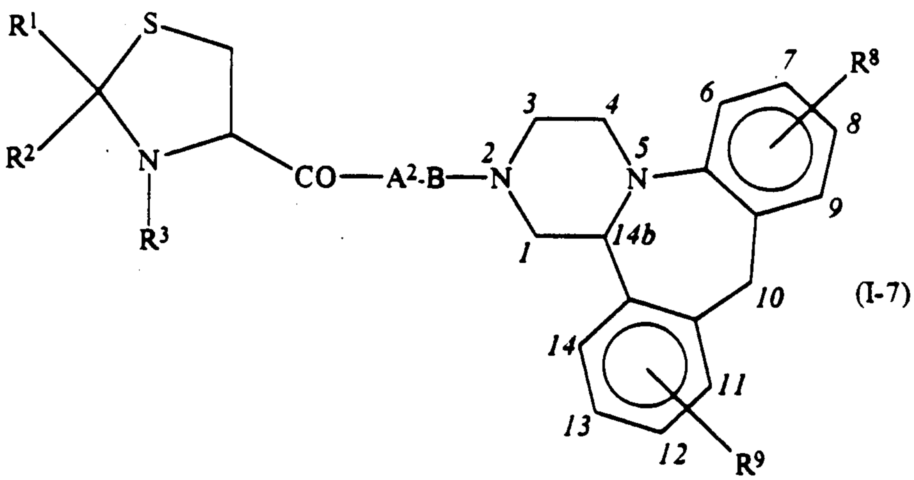

(I-7)

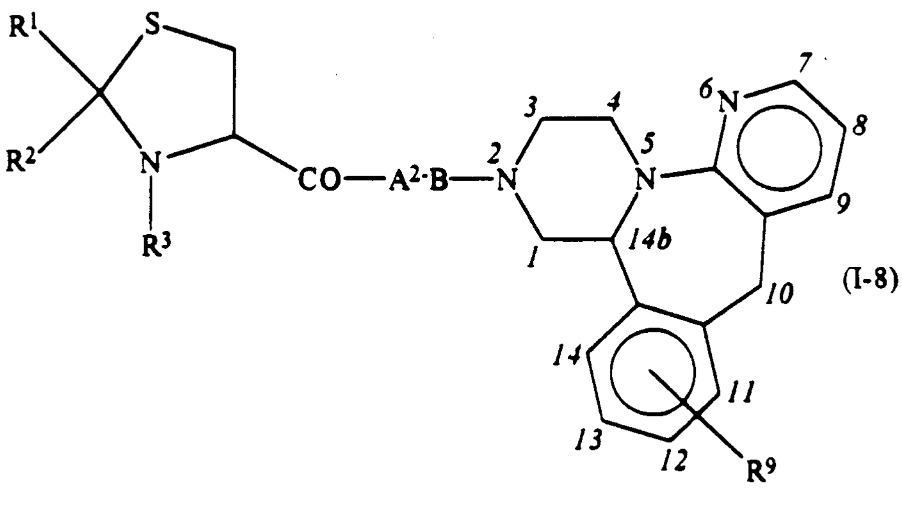

(I-8)

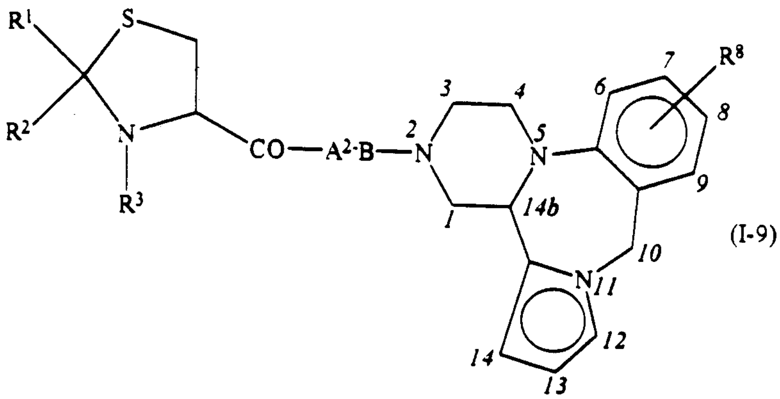
(I-9)

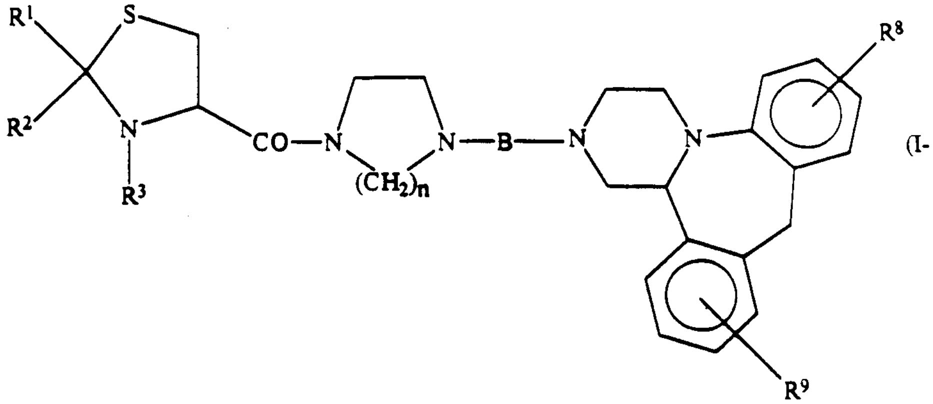
(I-10)

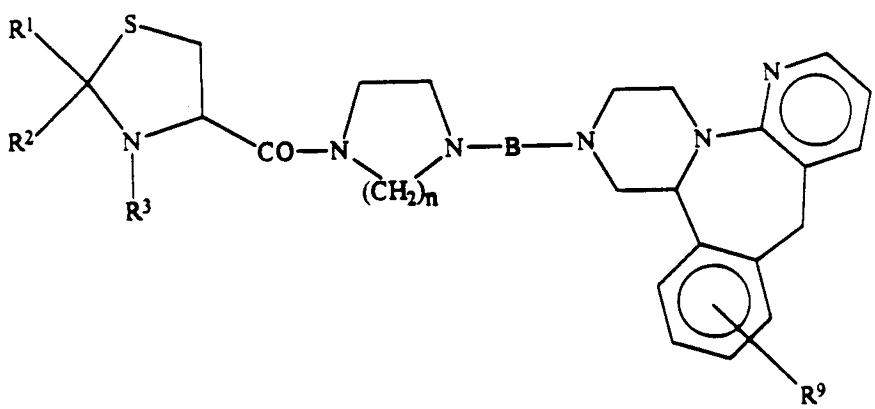
(I-11)

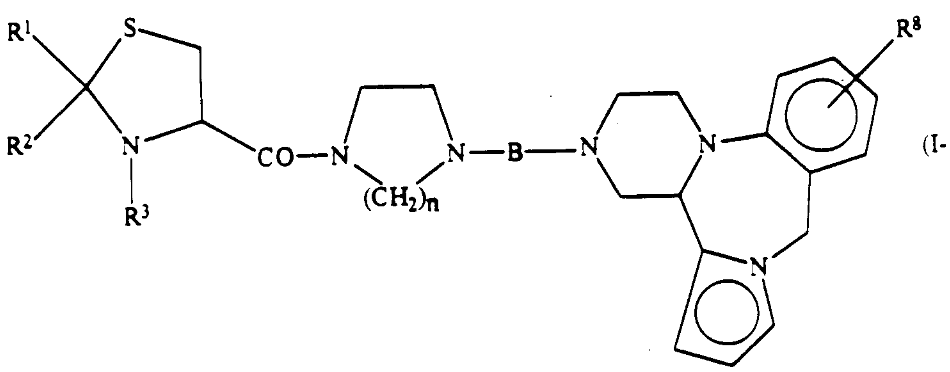
(I-12)

Table 1

| Cpd. No. | $R^1$ | $R^{10}$ | $R^{11}$ | D | m | $R^6$ | $R^7$ |
|---|---|---|---|---|---|---|---|
| 1-1 | 3-Py | Me | Me | $(CH_2)_3$ | 2 | 4-FPh | 4-FPh |
| 1-2 | 3-Py | Me | Me | $(CH_2)_4$ | 2 | 4-FPh | 4-FPh |
| 1-3 | 3-Py | Me | Me | $(CH_2)_5$ | 2 | 4-FPh | 4-FPh |
| 1-4 | 3-Py | Me | Me | $(CH_2)_3$ | 2 | 4-FPh | 3-FPh |
| 1-5 | 2-Py | Me | Me | $(CH_2)_3$ | 2 | Ph | 4-FPh |
| 1-6 | 3-Py | Me | Me | $(CH_2)_2$ | 2 | 4-FPh | 4-ClPh |
| 1-7 | 3-Py | Me | Me | $(CH_2)_3$ | 2 | 4-FPh | 4-MePh |
| 1-8 | 3-Py | Me | Me | $(CH_2)_4$ | 2 | 4-FPh | 3-MeOPh |
| 1-9 | 4-Py | Me | Me | $(CH_2)_3$ | 2 | 4-ClPh | 3-ClPh |
| 1-10 | 3-Py | Me | Me | $(CH_2)_5$ | 2 | 3-ClPh | 3-ClPh |
| 1-11 | 3-Py | Me | Me | $(CH_2)_3$ | 2 | 4-ClPh | 4-MeOPh |
| 1-12 | 3-Py | Me | Me | $(CH_2)_2$ | 2 | Ph | 4-MeOPh |
| 1-13 | 2-Py | Me | Me | $(CH_2)_3$ | 2 | 4-MeOPh | 4-MeOPh |
| 1-14 | 4-Py | Me | Me | $(CH_2)_2$ | 2 | Ph | 4-MePh |
| 1-15 | 3-Py | Me | Me | $(CH_2)_4$ | 2 | 4-MePh | 4-MePh |
| 1-16 | Dme-3-Py | Me | Me | $(CH_2)_3$ | 2 | 4-FPh | 4-FPh |
| 1-17 | 3-Py | Et | Et | $(CH_2)_3$ | 2 | Ph | 2-FPh |
| 1-18 | 2-Me-3-Py | Me | Me | $(CH_2)_2$ | 2 | Ph | Ph |
| 1-19 | 2-Py | Me | Me | $(CH_2)_3$ | 2 | 4-FPh | 4-FPh |
| 1-20 | 2-Py | Me | Me | $(CH_2)_3$ | 2 | Ph | Ph |
| 1-21 | 3-Py | Me | Me | $(CH_2)_3$ | 2 | Ph | 3-ClPh |
| 1-22 | 2-Me-3-Py | Et | Et | $(CH_2)_3$ | 2 | 4-FPh | 4-FPh |
| 1-23 | 3-Py | Et | Et | $(CH_2)_3$ | 2 | 4-FPh | 4-FPh |
| 1-24 | 3-Py | Me | Me | $(CH_2)_3$ | 2 | Ph | Ph |
| 1-25 | 3-Py | Me | Me | $(CH_2)_2$ | 2 | Ph | Ph |
| 1-26 | 3-Py | Me | Me | $(CH_2)_3$ | 3 | Ph | Ph |
| 1-27 | 4-Py | Me | Me | $(CH_2)_3$ | 3 | Ph | 2-FPh |
| 1-28 | 3-Py | Me | Me | $(CH_2)_3$ | 3 | 4-FPh | 4-FPh |

Table 1 (cont.)

| Cpd. No. | $R^1$ | $R^{10}$ | $R^{11}$ | D | m | $R^6$ | $R^7$ |
|---|---|---|---|---|---|---|---|
| 1-29 | 2-Py | Me | Me | $(CH_2)_4$ | 3 | 4-FPh | 4-FPh |
| 1-30 | 3-Py | Me | Me | $(CH_2)_5$ | 3 | Ph | 4-ClPh |
| 1-31 | 4-Py | Me | Me | $(CH_2)_2$ | 3 | Ph | 3-ClPh |
| 1-32 | 3-Py | Et | Et | $(CH_2)_2$ | 3 | 4-FPh | 4-FPh |
| 1-33 | 3-Py | Et | Et | $(CH_2)_3$ | 3 | 4-FPh | 4-FPh |
| 1-34 | 3-Py | Me | Me | $(CH_2)_2$ | 3 | Ph | Ph |
| 1-35 | 3-Py | Me | Me | $(CH_2)_4$ | 3 | Ph | Ph |
| 1-36 | 3-Py | Me | Me | $(CH_2)_3$ | 2 | Ph | Ph |
| 1-37 | 3-Py | Me | Me | $(CH_2)_3$ | 2 | Ph | 2-FPh |
| 1-38 | 3-Py | Me | Me | $(CH_2)_3$ | 2 | Ph | 3-ClPh |
| 1-39 | 3-Py | Me | Me | $(CH_2)_3$ | 2 | 4-FPh | 3-MePh |
| 1-40 | 3-Py | Me | Me | $(CH_2)_3$ | 2 | 4-FPh | 2-MeOPh |
| 1-41 | 3-Py | Me | Me | 2-MeTm | 2 | Ph | Ph |
| 1-42 | 3-Py | Me | Me | 2-MeTm | 2 | Ph | 4-ClPh |
| 1-43 | 3-Py | Et | Et | 2-MeTm | 2 | Ph | 4-FPh |
| 1-44 | 3-Py | Me | Me | 2-MeTm | 2 | 4-FPh | 4-FPh |
| 1-45 | 3-Py | Me | Me | 2-MeTm | 3 | Ph | Ph |
| 1-46 | 3-Py | Me | Me | $(CH_2)_3$ | 3 | Ph | Ph |
| 1-47 | 3-Py | Me | Me | $(CH_2)_3$ | 3 | 4-FPh | 4-FPh |
| 1-48 | 3-Py | Et | Et | $(CH_2)_3$ | 3 | Ph | Ph |
| 1-49 | 3-Py | Me | Me | $(CH_2)_2$ | 3 | Ph | 2-Py |
| 1-50 | 3-Py | Me | Me | $(CH_2)_3$ | 3 | Ph | 2-Py |
| 1-51 | 3-Py | Me | Me | $(CH_2)_4$ | 3 | Ph | 2-Py |
| 1-52 | 3-Py | Me | Me | $(CH_2)_5$ | 3 | Ph | 2-Py |
| 1-53 | 3-Py | Me | Me | $(CH_2)_2$ | 3 | Ph | 2-Py |
| 1-54 | 3-Py | Me | Me | $(CH_2)_3$ | 3 | Ph | 2-Py |
| 1-55 | 3-Py | Me | Me | $(CH_2)_2$ | 3 | 4-FPh | 2-Py |
| 1-56 | 3-Py | Et | Et | $(CH_2)_3$ | 2 | 4-FPh | 2-Py |

Table 1 (cont.)

| Cpd. No. | $R^1$ | $R^{10}$ | $R^{11}$ | D | m | $R^6$ | $R^7$ |
|---|---|---|---|---|---|---|---|
| 1-57 | 3-Py | Me | Me | $(CH_2)_2$ | 2 | 4-ClPh | 2-Py |
| 1-58 | 3-Py | Me | Me | $(CH_2)_4$ | 2 | 4-ClPh | 2-Py |
| 1-59 | 3-Py | Me | Me | $(CH_2)_3$ | 2 | 4-MePh | 2-Py |
| 1-60 | 3-Py | Me | Me | $(CH_2)_2$ | 2 | 4-TfmPh | 2-Py |
| 1-61 | 3-Py | Me | Me | $(CH_2)_2$ | 3 | Ph | 2-Py |
| 1-62 | 3-Py | Me | Me | $(CH_2)_4$ | 3 | Ph | 2-Py |
| 1-63 | 3-Py | Me | Me | $(CH_2)_2$ | 2 | Ph | 2-Py |
| 1-64 | 3-Py | Me | Me | $(CH_2)_2$ | 2 | Ph | 3-Py |
| 1-65 | 3-Py | Me | Me | $(CH_2)_3$ | 2 | 4-ClPh | 3-Py |
| 1-66 | 3-Py | Me | Me | $(CH_2)_4$ | 2 | 4-FPh | 3-Py |
| 1-67 | 4-Py | Me | Me | $(CH_2)_2$ | 2 | Ph | 3-Py |
| 1-68 | 3-Py | Me | Me | $(CH_2)_2$ | 3 | Ph | 3-Py |
| 1-69 | 3-Py | Me | Me | $(CH_2)_2$ | 2 | Ph | 4-Py |
| 1-70 | 3-Py | Me | Me | $(CH_2)_4$ | 2 | Ph | 4-Py |
| 1-71 | 4-Py | Me | Me | $(CH_2)_2$ | 2 | Ph | Ph |
| 1-72 | 2-Me-3-Py | Me | Me | $(CH_2)_2$ | 2 | Ph | Ph |
| 1-73 | 3-Py | Me | Me | $(CH_2)_3$ | 2 | Ph | 4-ClPh |
| 1-74 | 3-Py | Me | Me | $(CH_2)_2$ | 2 | Ph | 4-ClPh |
| 1-75 | 3-Py | Me | Me | $(CH_2)_4$ | 2 | Ph | Ph |
| 1-76 | 3-Py | Me | Me | $(CH_2)_2$ | 2 | 4-FPh | 4-FPh |

Table 2

| Cpd. No. | $R^1$ | $R^{10}$ | $R^{11}$ | D | m | $R^6$ | $R^7$ |
|---|---|---|---|---|---|---|---|
| 2-1 | 3-Py | Me | Me | $(CH_2)_3$ | 2 | 4-FPh | 4-FPh |
| 2-2 | 3-Py | Me | Me | $(CH_2)_4$ | 2 | 4-FPh | 4-FPh |
| 2-3 | 3-Py | Me | Me | $(CH_2)_3$ | 2 | 4-FPh | 3-FPh |
| 2-4 | 2-Py | Me | Me | $(CH_2)_3$ | 2 | Ph | 4-FPh |
| 2-5 | 3-Py | Me | Me | $(CH_2)_2$ | 2 | 4-FPh | 4-ClPh |
| 2-6 | 3-Py | Me | Me | $(CH_2)_3$ | 2 | 4-FPh | 4-MePh |
| 2-7 | 3-Py | Me | Me | $(CH_2)_4$ | 2 | 4-FPh | 3-MeOPh |
| 2-8 | 4-Py | Me | Me | $(CH_2)_3$ | 2 | 4-ClPh | 3-ClPh |
| 2-9 | 3-Py | Me | Me | $(CH_2)_3$ | 2 | 4-ClPh | 4-MeOPh |
| 2-10 | 3-Py | Me | Me | $(CH_2)_2$ | 2 | Ph | 4-MeOPh |
| 2-11 | 2-Py | Me | Me | $(CH_2)_3$ | 2 | 4-MeOPh | 4-MeOPh |
| 2-12 | 4-Py | Me | Me | $(CH_2)_2$ | 2 | Ph | 4-MePh |
| 2-13 | 3-Py | Me | Me | $(CH_2)_4$ | 2 | 4-MePh | 4-MePh |
| 2-14 | Dme-3-Py | Me | Me | $(CH_2)_3$ | 2 | 4-FPh | 4-FPh |
| 2-15 | 2-Me-3-Py | Me | Me | $(CH_2)_2$ | 2 | Ph | Ph |
| 2-16 | 2-Py | Me | Me | $(CH_2)_3$ | 2 | 4-FPh | 4-FPh |
| 2-17 | 2-Py | Me | Me | $(CH_2)_3$ | 2 | Ph | Ph |
| 2-18 | 3-Py | Me | Me | $(CH_2)_3$ | 2 | Ph | 3-ClPh |
| 2-19 | 3-Py | Me | Me | $(CH_2)_3$ | 2 | Ph | Ph |
| 2-20 | 3-Py | Me | Me | $(CH_2)_2$ | 2 | Ph | Ph |
| 2-21 | 3-Py | Me | Me | $(CH_2)_3$ | 3 | Ph | Ph |
| 2-22 | 4-Py | Me | Me | $(CH_2)_3$ | 3 | Ph | 2-FPh |
| 2-23 | 3-Py | Me | Me | $(CH_2)_3$ | 3 | 4-FPh | 4-FPh |
| 2-24 | 2-Py | Me | Me | $(CH_2)_4$ | 3 | 4-FPh | 4-FPh |
| 2-25 | 4-Py | Me | Me | $(CH_2)_2$ | 3 | Ph | 3-ClPh |
| 2-26 | 3-Py | Me | Me | $(CH_2)_2$ | 3 | Ph | Ph |
| 2-27 | 3-Py | Me | Me | $(CH_2)_4$ | 3 | Ph | Ph |
| 2-28 | 3-Py | Me | Me | $(CH_2)_3$ | 2 | Ph | Ph |

Table 2 (cont.)

| Cpd. No. | $R^1$ | $R^{10}$ | $R^{11}$ | D | m | $R^6$ | $R^7$ |
|---|---|---|---|---|---|---|---|
| 2-29 | 3-Py | Me | Me | $(CH_2)_3$ | 2 | Ph | 2-FPh |
| 2-30 | 3-Py | Me | Me | $(CH_2)_3$ | 2 | Ph | 3-ClPh |
| 2-31 | 3-Py | Me | Me | $(CH_2)_3$ | 2 | 4-FPh | 3-MePh |
| 2-32 | 3-Py | Me | Me | $(CH_2)_3$ | 2 | 4-FPh | 2-MeOPh |
| 2-33 | 3-Py | Me | Me | $(CH_2)_3$ | 3 | Ph | Ph |
| 2-34 | 3-Py | Me | Me | $(CH_2)_2$ | 3 | 4-FPh | 4-FPh |
| 2-35 | 3-Py | Et | Et | $(CH_2)_3$ | 3 | Ph | Ph |
| 2-36 | 3-Py | Me | Me | $(CH_2)_2$ | 2 | Ph | 2-Py |
| 2-37 | 3-Py | Me | Me | $(CH_2)_3$ | 3 | Ph | 2-Py |
| 2-38 | 3-Py | Me | Me | $(CH_2)_4$ | 3 | Ph | 2-Py |
| 2-39 | 3-Py | Me | Me | $(CH_2)_2$ | 3 | Ph | 2-Py |
| 2-40 | 3-Py | Me | Me | $(CH_2)_3$ | 3 | Ph | 2-Py |
| 2-41 | 3-Py | Me | Me | $(CH_2)_2$ | 3 | 4-FPh | 2-Py |
| 2-42 | 3-Py | Me | Me | $(CH_2)_2$ | 2 | 4-ClPh | 2-Py |
| 2-43 | 3-Py | Me | Me | $(CH_2)_4$ | 2 | 4-ClPh | 2-Py |
| 2-44 | 3-Py | Me | Me | $(CH_2)_3$ | 2 | 4-MePh | 2-Py |
| 2-45 | 3-Py | Me | Me | $(CH_2)_2$ | 2 | 4-TfmPh | 2-Py |
| 2-46 | 3-Py | Me | Me | $(CH_2)_2$ | 3 | Ph | 2-Py |
| 2-47 | 3-Py | Me | Me | $(CH_2)_4$ | 3 | Ph | 2-Py |
| 2-48 | 3-Py | Me | Me | $(CH_2)_2$ | 2 | Ph | 2-Py |
| 2-49 | 3-Py | Me | Me | $(CH_2)_2$ | 2 | Ph | 3-Py |
| 2-50 | 3-Py | Me | Me | $(CH_2)_3$ | 2 | 4-ClPh | 3-Py |
| 2-51 | 3-Py | Me | Me | $(CH_2)_4$ | 2 | 4-FPh | 3-Py |
| 2-52 | 4-Py | Me | Me | $(CH_2)_2$ | 2 | Ph | 3-Py |
| 2-53 | 3-Py | Me | Me | $(CH_2)_2$ | 3 | Ph | 3-Py |
| 2-54 | 3-Py | Me | Me | $(CH_2)_2$ | 2 | Ph | 4-Py |
| 2-55 | 3-Py | Me | Me | $(CH_2)_4$ | 2 | Ph | 4-Py |
| 2-56 | 3-Py | Me | Me | $(CH_2)_2$ | 2 | Ph | Ph |

Table 2 (cont.)

| Cpd. No. | $R^1$ | $R^{10}$ | $R^{11}$ | D | m | $R^6$ | $R^7$ |
|---|---|---|---|---|---|---|---|
| 2-57 | 3-Py | Me | Et | $(CH_2)_2$ | 2 | Ph | Ph |
| 2-58 | 3-Py | Me | Me | $(CH_2)_3$ | 2 | Ph | 4-ClPh |
| 2-59 | 3-Py | Me | Me | $(CH_2)_2$ | 2 | Ph | 4-ClPh |
| 2-60 | 3-Py | Me | Me | $(CH_2)_4$ | 2 | Ph | Ph |
| 2-61 | 3-Py | Me | Me | $(CH_2)_2$ | 2 | 4-FPh | 4-FPh |

Table 3

| Cpd. No. | $R^1$ | $R^2$ | $R^3$ | n | B | $R^6$ | $R^7$ |
|---|---|---|---|---|---|---|---|
| 3-1 | 3-Py | H | H | 2 | $(CH_2)_2$ | 4-FPh | 4-FPh |
| 3-2 | 3-Py | H | H | 2 | $(CH_2)_3$ | 4-FPh | 4-FPh |
| 3-3 | 3-Py | H | H | 2 | $(CH_2)_4$ | 4-FPh | 4-FPh |
| 3-4 | 3-Py | H | H | 2 | $(CH_2)_3$ | Ph | 4-FPh |
| 3-5 | 2-Py | H | H | 2 | $(CH_2)_2$ | 4-FPh | 4-ClPh |
| 3-6 | 3-Py | H | H | 2 | $(CH_2)_3$ | 4-FPh | 4-MePh |
| 3-7 | 3-Py | H | H | 2 | $(CH_2)_4$ | 4-FPh | 4-MeOPh |
| 3-8 | 4-Py | H | H | 2 | $(CH_2)_3$ | 4-ClPh | 3-ClPh |
| 3-9 | 3-Py | H | H | 2 | $(CH_2)_3$ | 4-ClPh | 4-MeOPh |
| 3-10 | 3-Py | H | H | 2 | $(CH_2)_2$ | Ph | 4-MeOPh |
| 3-11 | 3-Py | H | H | 2 | $(CH_2)_3$ | 4-MeOPh | 4-MeOPh |
| 3-12 | 4-Py | H | H | 2 | $(CH_2)_2$ | Ph | 4-MePh |
| 3-13 | 3-Py | H | H | 2 | $(CH_2)_4$ | 4-MePh | 4-MePh |
| 3-14 | Dme-3-Py | H | H | 2 | $(CH_2)_3$ | 4-FPh | 4-FPh |
| 3-15 | 3-Py | H | H | 2 | $(CH_2)_3$ | 4-FPh | 2-FPh |
| 3-16 | 2-Me-3-Py | H | H | 2 | $(CH_2)_2$ | Ph | Ph |
| 3-17 | 2-Py | H | H | 2 | $(CH_2)_3$ | 4-FPh | 4-FPh |
| 3-18 | 2-Py | H | H | 2 | $(CH_2)_3$ | Ph | Ph |
| 3-19 | 3-Py | H | H | 2 | $(CH_2)_3$ | Ph | 3-ClPh |
| 3-20 | 2-Me-6-Py | H | H | 2 | $(CH_2)_3$ | 4-FPh | 4-FPh |
| 3-21 | 3-Py | H | Me | 2 | $(CH_2)_3$ | 4-FPh | 4-FPh |
| 3-22 | 3-Py | H | H | 2 | $(CH_2)_3$ | Ph | Ph |
| 3-23 | 3-Py | H | H | 3 | $(CH_2)_2$ | Ph | Ph |
| 3-24 | 4-Py | H | H | 3 | $(CH_2)_3$ | Ph | Ph |
| 3-25 | 3-Py | H | H | 3 | $(CH_2)_2$ | 4-FPh | 4-FPh |
| 3-26 | 2-Py | H | H | 3 | $(CH_2)_4$ | 4-FPh | 4-FPh |
| 3-27 | 4-Py | H | H | 3 | $(CH_2)_2$ | 4-ClPh | 4-ClPh |
| 3-28 | 3-Py | H | H | 3 | $(CH_2)_3$ | 4-FPh | 4-ClPh |

Table 3 (cont.)

| Cpd. No. | $R^1$ | $R^2$ | $R^3$ | n | B | $R^6$ | $R^7$ |
|---|---|---|---|---|---|---|---|
| 3-29 | 3-Py | H | H | 2 | $(CH_2)_2$ | Ph | 4-MePh |
| 3-30 | 3-Py | H | H | 3 | $(CH_2)_3$ | Ph | 4-MePh |
| 3-31 | 3-Py | H | H | 3 | $(CH_2)_2$ | 4-MePh | 4-MePh |
| 3-32 | 3-Py | H | H | 3 | $(CH_2)_4$ | 4-MeOPh | 4-MeOPh |
| 3-33 | 3-Py | H | H | 2 | $(CH_2)_3$ | 4-FPh | 3-FPh |
| 3-34 | 3-Py | H | H | 2 | $(CH_2)_3$ | Ph | 2-FPh |
| 3-35 | 3-Py | H | H | 2 | $(CH_2)_3$ | Ph | 3-ClPh |
| 3-36 | 3-Py | H | H | 2 | $(CH_2)_3$ | 4-FPh | 3-MePh |
| 3-37 | 3-Py | H | H | 2 | $(CH_2)_3$ | 4-FPh | 2-MeOPh |
| 3-38 | 3-Py | H | H | 2 | 2-MeTm | Ph | Ph |
| 3-39 | 3-Py | H | H | 2 | 2-MeTm | Ph | 4-ClPh |
| 3-40 | 3-Py | H | H | 2 | 2-MeTm | Ph | 4-FPh |
| 3-41 | 3-Py | H | H | 2 | 2-MeTm | 4-FPh | 4-FPh |
| 3-42 | 3-Py | H | H | 2 | $(CH_2)_2$ | Ph | cHx |
| 3-43 | 3-Py | H | H | 3 | $(CH_2)_2$ | Ph | cHx |
| 3-44 | 3-Py | H | H | 2 | $(CH_2)_4$ | Ph | cHx |
| 3-45 | 3-Py | H | H | 3 | 2-MeTm | Ph | Ph |
| 3-46 | 3-Py | H | Me | 2 | $(CH_2)_3$ | Ph | Ph |
| 3-47 | 3-Py | H | Me | 2 | $(CH_2)_3$ | 4-FPh | 4-FPh |
| 3-48 | 3-Py | 3-Py | H | 2 | $(CH_2)_3$ | 4-FPh | 4-FPh |
| 3-49 | 3-Py | H | H | 2 | $(CH_2)_2$ | Ph | 2-Py |
| 3-50 | 3-Py | H | H | 2 | $(CH_2)_3$ | Ph | 2-Py |
| 3-51 | 3-Py | H | H | 2 | $(CH_2)_4$ | Ph | 2-Py |
| 3-52 | 3-Py | H | Me | 2 | $(CH_2)_2$ | Ph | 2-Py |
| 3-53 | 3-Py | H | Me | 2 | $(CH_2)_3$ | Ph | 2-Py |
| 3-54 | 3-Py | H | H | 2 | $(CH_2)_2$ | 4-FPh | 2-Py |
| 3-55 | 3-Py | H | H | 2 | $(CH_2)_3$ | 4-FPh | 2-Py |
| 3-56 | 3-Py | H | H | 2 | $(CH_2)_2$ | 4-ClPh | 2-Py |

Table 3 (cont.)

| Cpd. No. | $R^1$ | $R^2$ | $R^3$ | n | B | $R^6$ | $R^7$ |
|---|---|---|---|---|---|---|---|
| 3-57 | 3-Py | H | H | 2 | $(CH_2)_4$ | 4-ClPh | 2-Py |
| 3-58 | 3-Py | H | H | 2 | $(CH_2)_3$ | 4-MePh | 2-Py |
| 3-59 | 3-Py | H | H | 2 | $(CH_2)_2$ | 4-TfmPh | 2-Py |
| 3-60 | 3-Py | H | H | 3 | $(CH_2)_2$ | Ph | 2-Py |
| 3-61 | 3-Py | H | H | 3 | $(CH_2)_4$ | Ph | 2-Py |
| 3-62 | 4-Py | H | H | 2 | $(CH_2)_2$ | Ph | 2-Py |
| 3-63 | 3-Py | H | H | 2 | $(CH_2)_2$ | Ph | 3-Py |
| 3-64 | 3-Py | H | H | 2 | $(CH_2)_3$ | 4-ClPh | 3-Py |
| 3-65 | 3-Py | H | H | 2 | $(CH_2)_4$ | 4-FPh | 3-Py |
| 3-66 | 4-Py | H | H | 2 | $(CH_2)_2$ | Ph | 3-Py |
| 3-67 | 3-Py | H | H | 3 | $(CH_2)_2$ | Ph | 3-Py |
| 3-68 | 3-Py | H | H | 2 | $(CH_2)_2$ | Ph | 4-Py |
| 3-69 | 3-Py | H | H | 2 | $(CH_2)_4$ | Ph | 4-Py |
| 3-70 | 3-Py | H | H | 2 | $(CH_2)_2$ | 4-MePh | Ph |
| 3-71 | 3-Py | H | H | 2 | $(CH_2)_2$ | Ph | Ph |
| 3-72 | 3-Py | H | H | 2 | $(CH_2)_3$ | Ph | 4-ClPh |
| 3-73 | 3-Py | H | H | 2 | $(CH_2)_2$ | Ph | 4-ClPh |
| 3-74 | 3-Py | H | H | 2 | $(CH_2)_4$ | Ph | Ph |

Table 4

| Cpd. No. | $R^1$ | $R^2$ | $R^3$ | n | m | $R^6$ | $R^7$ |
|---|---|---|---|---|---|---|---|
| 4-1 | 3-Py | H | H | 2 | 3 | 4-FPh | 4-FPh |
| 4-2 | 3-Py | H | H | 2 | 4 | 4-FPh | 4-FPh |
| 4-3 | 3-Py | H | H | 3 | 3 | 4-FPh | 4-FPh |
| 4-4 | 3-Py | H | H | 2 | 3 | Ph | 4-FPh |
| 4-5 | 2-Py | H | H | 2 | 2 | 4-FPh | 4-ClPh |
| 4-6 | 3-Py | H | H | 2 | 3 | 4-FPh | 4-MePh |
| 4-7 | 4-Py | H | H | 2 | 3 | 4-ClPh | 3-ClPh |
| 4-8 | 3-Py | H | H | 2 | 3 | 4-ClPh | 4-MeOPh |
| 4-9 | 3-Py | H | H | 2 | 2 | Ph | 4-MeOPh |
| 4-10 | 3-Py | H | H | 2 | 3 | 4-MeOPh | 4-MeOPh |
| 4-11 | 4-Py | H | H | 2 | 2 | Ph | 4-MePh |
| 4-12 | Dme-3-Py | H | H | 2 | 3 | 4-FPh | 4-FPh |
| 4-13 | 3-Py | H | H | 2 | 3 | 4-FPh | 2-FPh |
| 4-14 | 2-Me-3-Py | H | H | 2 | 2 | Ph | Ph |
| 4-15 | 2-Py | H | H | 2 | 3 | 4-FPh | 4-FPh |
| 4-16 | 2-Py | H | H | 2 | 3 | Ph | Ph |
| 4-17 | 4-Py | H | H | 2 | 3 | Ph | 3-ClPh |
| 4-18 | 2-Me-6-Py | H | H | 2 | 3 | 4-FPh | 4-FPh |
| 4-19 | 3-Py | H | Me | 2 | 3 | 4-FPh | 4-FPh |
| 4-20 | 3-Py | H | H | 2 | 3 | Ph | Ph |
| 4-21 | 3-Py | H | H | 3 | 2 | Ph | Ph |
| 4-22 | 4-Py | H | H | 3 | 3 | Ph | Ph |
| 4-23 | 3-Py | H | H | 3 | 2 | 4-FPh | 4-FPh |
| 4-24 | 4-Py | H | H | 3 | 2 | 4-ClPh | 4-ClPh |
| 4-25 | 3-Py | H | H | 3 | 3 | 4-FPh | 4-ClPh |
| 4-26 | 3-Py | H | H | 2 | 2 | Ph | 4-MePh |
| 4-27 | 3-Py | H | H | 3 | 3 | Ph | 4-MePh |
| 4-28 | 3-Py | H | H | 3 | 2 | 4-MePh | 4-MePh |

Table 4 (cont.)

| Cpd. No. | $R^1$ | $R^2$ | $R^3$ | n | m | $R^6$ | $R^7$ |
|---|---|---|---|---|---|---|---|
| 4-29 | 3-Py | H | H | 3 | 4 | 4-MeOPh | 4-MeOPh |
| 4-30 | 3-Py | H | H | 2 | 3 | 4-FPh | 3-FPh |
| 4-31 | 3-Py | H | H | 2 | 3 | Ph | 2-FPh |
| 4-32 | 3-Py | H | H | 2 | 3 | Ph | 3-ClPh |
| 4-33 | 3-Py | H | H | 2 | 3 | 4-FPh | 3-MePh |
| 4-34 | 3-Py | H | H | 2 | 3 | 4-FPh | 2-MeOPh |
| 4-35 | 3-Py | H | H | 2 | 2 | Ph | cHx |
| 4-36 | 3-Py | H | H | 3 | 2 | Ph | cHx |
| 4-37 | 3-Py | H | Me | 2 | 3 | Ph | Ph |
| 4-38 | 3-Py | H | Me | 2 | 3 | 4-FPh | 4-FPh |
| 4-39 | 3-Py | 3-Py | H | 2 | 3 | 4-FPh | 4-FPh |
| 4-40 | 3-Py | H | H | 2 | 2 | Ph | 2-Py |
| 4-41 | 3-Py | H | H | 2 | 3 | Ph | 2-Py |
| 4-42 | 3-Py | H | Me | 2 | 2 | Ph | 2-Py |
| 4-43 | 3-Py | H | Me | 2 | 3 | Ph | 2-Py |
| 4-44 | 3-Py | H | H | 2 | 2 | 4-FPh | 2-Py |
| 4-45 | 3-Py | H | H | 2 | 3 | 4-FPh | 2-Py |
| 4-46 | 3-Py | H | H | 2 | 2 | 4-ClPh | 2-Py |
| 4-47 | 3-Py | H | H | 2 | 3 | 4-MePh | 2-Py |
| 4-48 | 3-Py | H | H | 2 | 2 | 4-TfmPh | 2-Py |
| 4-49 | 3-Py | H | H | 3 | 2 | Ph | 2-Py |
| 4-50 | 4-Py | H | H | 2 | 2 | Ph | 2-Py |
| 4-51 | 3-Py | H | H | 2 | 2 | Ph | 3-Py |
| 4-52 | 3-Py | H | H | 2 | 3 | 4-ClPh | 3-Py |
| 4-53 | 4-Py | H | H | 2 | 2 | Ph | 3-Py |
| 4-54 | 3-Py | H | H | 3 | 2 | Ph | 3-Py |
| 4-55 | 3-Py | H | H | 2 | 2 | Ph | 4-Py |
| 4-56 | 3-Py | H | H | 2 | 2 | Ph | Ph |

Table 4 (cont.)

| Cpd. No. | $R^1$ | $R^2$ | $R^3$ | n | m | $R^6$ | $R^7$ |
|---|---|---|---|---|---|---|---|
| 4-57 | 3-Py | H | H | 2 | 3 | Ph | Ph |
| 4-58 | 3-Py | H | H | 2 | 3 | Ph | 4-ClPh |
| 4-59 | 3-Py | H | H | 2 | 2 | Ph | 4-ClPh |
| 4-60 | 3-Py | H | H | 2 | 2 | 4-FPh | 4-FPh |

Table 5

| Cpd. No. | $R^1$ | $R^{10}$ | $R^{11}$ | D | m | $R^6$ | $R^7$ |
|---|---|---|---|---|---|---|---|
| 5-1 | 3-Py | Me | Me | $(CH_2)_3$ | 2 | 4-FPh | 4-FPh |
| 5-2 | 3-Py | Me | Me | $(CH_2)_4$ | 2 | 4-FPh | 4-FPh |
| 5-3 | 3-Py | Me | Me | $(CH_2)_3$ | 2 | 4-FPh | 3-FPh |
| 5-4 | 2-Py | Me | Me | $(CH_2)_3$ | 2 | Ph | 4-FPh |
| 5-5 | 3-Py | Me | Me | $(CH_2)_2$ | 2 | 4-FPh | 4-ClPh |
| 5-6 | 3-Py | Me | Me | $(CH_2)_3$ | 2 | 4-FPh | 4-MePh |
| 5-7 | 3-Py | Me | Me | $(CH_2)_4$ | 2 | 4-FPh | 3-MeOPh |
| 5-8 | 4-Py | Me | Me | $(CH_2)_3$ | 2 | 4-ClPh | 3-ClPh |
| 5-9 | 3-Py | Me | Me | $(CH_2)_3$ | 2 | 4-ClPh | 4-MeOPh |
| 5-10 | 3-Py | Me | Me | $(CH_2)_2$ | 2 | Ph | 4-MeOPh |
| 5-11 | 2-Py | Me | Me | $(CH_2)_3$ | 2 | 4-MeOPh | 4-MeOPh |
| 5-12 | 4-Py | Me | Me | $(CH_2)_2$ | 2 | Ph | 4-MePh |
| 5-13 | 3-Py | Me | Me | $(CH_2)_4$ | 2 | 4-MePh | 4-MePh |
| 5-14 | Dme-3-Py | Me | Me | $(CH_2)_3$ | 2 | 4-FPh | 4-FPh |
| 5-15 | 2-Me-3-Py | Me | Me | $(CH_2)_2$ | 2 | Ph | Ph |
| 5-16 | 2-Py | Me | Me | $(CH_2)_3$ | 2 | 4-FPh | 4-FPh |
| 5-17 | 2-Py | Me | Me | $(CH_2)_3$ | 2 | Ph | Ph |
| 5-18 | 3-Py | Me | Me | $(CH_2)_3$ | 2 | Ph | 3-ClPh |
| 5-19 | 3-Py | Me | Me | $(CH_2)_3$ | 2 | Ph | Ph |
| 5-20 | 3-Py | Me | Me | $(CH_2)_2$ | 2 | Ph | Ph |
| 5-21 | 3-Py | Me | Me | $(CH_2)_3$ | 3 | Ph | Ph |
| 5-22 | 4-Py | Me | Me | $(CH_2)_3$ | 3 | Ph | 2-FPh |
| 5-23 | 3-Py | Me | Me | $(CH_2)_3$ | 3 | 4-FPh | 4-FPh |
| 5-24 | 2-Py | Me | Me | $(CH_2)_4$ | 3 | 4-FPh | 4-FPh |
| 5-25 | 4-Py | Me | Me | $(CH_2)_2$ | 3 | Ph | 3-ClPh |
| 5-26 | 3-Py | Me | Me | $(CH_2)_2$ | 3 | Ph | Ph |
| 5-27 | 3-Py | Me | Me | $(CH_2)_4$ | 3 | Ph | Ph |
| 5-28 | 3-Py | Me | Me | $(CH_2)_3$ | 2 | Ph | Ph |

Table 5 (cont.)

| Cpd. No. | $R^1$ | $R^{10}$ | $R^{11}$ | D | m | $R^6$ | $R^7$ |
|---|---|---|---|---|---|---|---|
| 5-29 | 3-Py | Me | Me | $(CH_2)_3$ | 2 | Ph | 2-FPh |
| 5-30 | 3-Py | Me | Me | $(CH_2)_3$ | 2 | Ph | 3-ClPh |
| 5-31 | 3-Py | Me | Me | $(CH_2)_3$ | 2 | 4-FPh | 3-MePh |
| 5-32 | 3-Py | Me | Me | $(CH_2)_3$ | 2 | 4-FPh | 2-MeOPh |
| 5-33 | 3-Py | Me | Me | $(CH_2)_3$ | 3 | Ph | Ph |
| 5-34 | 3-Py | Me | Me | $(CH_2)_2$ | 3 | 4-FPh | 4-FPh |
| 5-35 | 3-Py | Et | Et | $(CH_2)_3$ | 3 | Ph | Ph |
| 5-36 | 3-Py | Me | Me | $(CH_2)_2$ | 2 | Ph | 2-Py |
| 5-37 | 3-Py | Me | Me | $(CH_2)_3$ | 3 | Ph | 2-Py |
| 5-38 | 3-Py | Me | Me | $(CH_2)_4$ | 3 | Ph | 2-Py |
| 5-39 | 3-Py | Me | Me | $(CH_2)_2$ | 3 | Ph | 2-Py |
| 5-40 | 3-Py | Me | Me | $(CH_2)_3$ | 3 | Ph | 2-Py |
| 5-41 | 3-Py | Me | Me | $(CH_2)_2$ | 3 | 4-FPh | 2-Py |
| 5-42 | 3-Py | Me | Me | $(CH_2)_2$ | 2 | 4-ClPh | 2-Py |
| 5-43 | 3-Py | Me | Me | $(CH_2)_4$ | 2 | 4-ClPh | 2-Py |
| 5-44 | 3-Py | Me | Me | $(CH_2)_3$ | 2 | 4-MePh | 2-Py |
| 5-45 | 3-Py | Me | Me | $(CH_2)_2$ | 2 | 4-TfmPh | 2-Py |
| 5-46 | 3-Py | Me | Me | $(CH_2)_2$ | 3 | Ph | 2-Py |
| 5-47 | 3-Py | Me | Me | $(CH_2)_4$ | 3 | Ph | 2-Py |
| 5-48 | 3-Py | Me | Me | $(CH_2)_2$ | 2 | Ph | 2-Py |
| 5-49 | 3-Py | Me | Me | $(CH_2)_2$ | 2 | Ph | 3-Py |
| 5-50 | 3-Py | Me | Me | $(CH_2)_3$ | 2 | 4-ClPh | 3-Py |
| 5-51 | 3-Py | Me | Me | $(CH_2)_4$ | 2 | 4-FPh | 3-Py |
| 5-52 | 4-Py | Me | Me | $(CH_2)_2$ | 2 | Ph | 3-Py |
| 5-53 | 3-Py | Me | Me | $(CH_2)_2$ | 3 | Ph | 3-Py |
| 5-54 | 3-Py | Me | Me | $(CH_2)_2$ | 2 | Ph | 4-Py |
| 5-55 | 3-Py | Me | Me | $(CH_2)_4$ | 2 | Ph | 4-Py |
| 5-56 | 3-Py | Me | Me | $(CH_2)_2$ | 2 | Ph | Ph |

Table 5 (cont.)

| Cpd. No. | $R^1$ | $R^{10}$ | $R^{11}$ | D | m | $R^6$ | $R^7$ |
|---|---|---|---|---|---|---|---|
| 5-57 | 3-Py | Me | Et | $(CH_2)_2$ | 2 | Ph | Ph |
| 5-58 | 3-Py | Me | Me | $(CH_2)_3$ | 2 | Ph | 4-ClPh |
| 5-59 | 3-Py | Me | Me | $(CH_2)_2$ | 2 | Ph | 4-ClPh |
| 5-60 | 3-Py | Me | Me | $(CH_2)_4$ | 2 | Ph | Ph |
| 5-61 | 3-Py | Me | Me | $(CH_2)_2$ | 2 | 4-FPh | 4-FPh |

Table 6

| Cpd. No. | $R^1$ | $R^2$ | $R^3$ | n | B | $R^6$ | $R^7$ |
|---|---|---|---|---|---|---|---|
| 6-1 | 3-Py | H | H | 2 | $(CH_2)_2$ | Ph | Ph |
| 6-2 | 3-Py | H | H | 2 | $(CH_2)_3$ | Ph | Ph |
| 6-3 | 3-Py | H | H | 2 | $(CH_2)_4$ | Ph | Ph |
| 6-4 | 3-Py | H | H | 2 | $(CH_2)_3$ | Ph | 4-FPh |
| 6-5 | 3-Py | H | H | 3 | $(CH_2)_4$ | 4-FPh | 4-FPh |
| 6-6 | 3-Py | H | H | 2 | $(CH_2)_3$ | Ph | 4-ClPh |
| 6-7 | 3-Py | H | H | 2 | $(CH_2)_3$ | 4-ClPh | 4-ClPh |
| 6-8 | 4-Py | H | H | 3 | $(CH_2)_2$ | Ph | 4-MePh |
| 6-9 | 3-Py | H | H | 2 | $(CH_2)_3$ | 4-MeOPh | 4-MeOPh |
| 6-10 | 4-Py | H | H | 3 | $(CH_2)_4$ | 4-MeOPh | 4-MeOPh |
| 6-11 | 2-Py | H | H | 2 | $(CH_2)_2$ | Ph | 4-MeOPh |
| 6-12 | 4-Py | H | Me | 3 | $(CH_2)_3$ | Ph | Ph |
| 6-13 | 3-Py | H | Me | 3 | $(CH_2)_3$ | Ph | Ph |
| 6-14 | 2-Me-6-Py | H | H | 2 | $(CH_2)_3$ | Ph | Ph |
| 6-15 | 2-Py | H | H | 2 | $(CH_2)_3$ | Ph | Ph |
| 6-16 | 3-Py | H | H | 2 | $(CH_2)_3$ | 4-FPh | 3-FPh |
| 6-17 | 3-Py | H | H | 3 | $(CH_2)_3$ | Ph | 3-FPh |
| 6-18 | 3-Py | H | H | 2 | $(CH_2)_3$ | Ph | 3-ClPh |
| 6-19 | 3-Py | H | H | 2 | $(CH_2)_3$ | 4-ClPh | 2-ClPh |
| 6-20 | 3-Py | H | H | 2 | $(CH_2)_3$ | Ph | 3-MePh |
| 6-21 | 3-Py | H | H | 2 | $(CH_2)_3$ | 4-MePh | 3-MeOPh |
| 6-22 | 3-Py | H | H | 2 | 2-MeTm | Ph | Ph |
| 6-23 | 3-Py | H | H | 2 | 2-MeTm | Ph | 4-FPh |
| 6-24 | 3-Py | H | H | 2 | 2-MeTm | 4-FPh | 4-FPh |
| 6-25 | 3-Py | 3-Py | H | 2 | $(CH_2)_3$ | Ph | Ph |
| 6-26 | 3-Py | H | Me | 2 | $(CH_2)_3$ | Ph | Ph |
| 6-27 | 3-Py | H | Me | 2 | $(CH_2)_3$ | 4-FPh | 4-FPh |
| 6-28 | 3-Py | H | H | 2 | $(CH_2)_2$ | 4-FPh | 4-FPh |

Table 6 (cont.)

| Cpd. No. | $R^1$ | $R^2$ | $R^3$ | n | B | $R^6$ | $R^7$ |
|---|---|---|---|---|---|---|---|
| 6-29 | 3-Py | H | H | 2 | $(CH_2)_3$ | 4-FPh | 4-FPh |
| 6-30 | 3-Py | H | Me | 2 | $(CH_2)_2$ | 4-FPh | 4-FPh |
| 6-31 | 3-Py | H | Me | 2 | $(CH_2)_3$ | 4-FPh | 4-FPh |
| 6-32 | 3-Py | H | H | 2 | $(CH_2)_2$ | Ph | 3-Thi |
| 6-33 | 3-Py | H | H | 2 | $(CH_2)_3$ | Ph | 3-Thi |
| 6-34 | 2-Me-5-Py | H | H | 2 | $(CH_2)_2$ | 4-FPh | 4-FPh |
| 6-35 | 2-Me-5-Py | H | H | 2 | $(CH_2)_3$ | 4-FPh | 4-FPh |
| 6-36 | 2-Me-5-Py | H | H | 2 | $(CH_2)_2$ | Ph | Ph |
| 6-37 | 2-Me-5-Py | H | H | 2 | $(CH_2)_3$ | Ph | Ph |
| 6-38 | 4-Py | H | H | 2 | $(CH_2)_3$ | Ph | Ph |
| 6-39 | 2-Py | H | H | 2 | $(CH_2)_2$ | Ph | Ph |
| 6-40 | 2-Py | H | H | 2 | $(CH_2)_4$ | 4-FPh | 4-FPh |
| 6-41 | 2-Py | H | H | 3 | $(CH_2)_2$ | Ph | Ph |
| 6-42 | 3-Py | H | H | 3 | $(CH_2)_2$ | Ph | Ph |
| 6-43 | 2-Py | H | H | 2 | $(CH_2)_3$ | Ph | Ph |
| 6-44 | 3-Py | H | H | 2 | $(CH_2)_3$ | Ph | 4-MePh |

Table 7

| Cpd. No. | $R^1$ | $R^2$ | $R^3$ | $A^2$-B | $R^8$ | $R^9$ |
|---|---|---|---|---|---|---|
| 7-1 | 3-Py | H | H | NH-$(CH_2)_2$ | H | H |
| 7-2 | 2-Py | H | H | NH-$(CH_2)_2$ | H | H |
| 7-3 | 4-Py | H | H | NH-$(CH_2)_2$ | H | H |
| 7-4 | 2-Me-3-Py | H | H | NH-$(CH_2)_2$ | H | H |
| 7-5 | Dme-3-Py | H | H | NH-$(CH_2)_2$ | H | H |
| 7-6 | 3-Py | H | H | N(Me)-$(CH_2)_2$ | H | H |
| 7-7 | 2-Py | H | H | N(Me)-$(CH_2)_2$ | H | H |
| 7-8 | 4-Py | H | H | N(Me)-$(CH_2)_2$ | H | H |
| 7-9 | 3-Py | H | H | N(Et)-$(CH_2)_2$ | H | H |
| 7-10 | 3-Py | Me | H | NH-$(CH_2)_2$ | H | H |
| 7-11 | 3-Py | Et | H | NH-$(CH_2)_2$ | H | H |
| 7-12 | 3-Py | 3-Py | H | NH-$(CH_2)_2$ | H | H |
| 7-13 | 3-Py | Me | H | N(Me)-$(CH_2)_2$ | H | H |
| 7-14 | 3-Py | H | Me | N(Me)-$(CH_2)_2$ | H | H |
| 7-15 | 2-Py | H | Me | N(Me)-$(CH_2)_2$ | H | H |
| 7-16 | 4-Py | H | Me | N(Me)-$(CH_2)_2$ | H | H |
| 7-17 | 2-Me-3-Py | H | Me | N(Me)-$(CH_2)_2$ | H | H |
| 7-18 | Dme-3-Py | H | Me | N(Me)-$(CH_2)_2$ | H | H |
| 7-19 | 3-Py | H | Et | N(Me)-$(CH_2)_2$ | H | H |
| 7-20 | 3-Py | H | H | NH-$(CH_2)_2$ | 8-F | H |
| 7-21 | 2-Py | H | H | NH-$(CH_2)_2$ | 8-F | H |
| 7-22 | 4-Py | H | H | NH-$(CH_2)_2$ | 8-F | H |
| 7-23 | 2-Me-3-Py | H | H | NH-$(CH_2)_2$ | 8-F | H |
| 7-24 | Dme-3-Py | H | H | NH-$(CH_2)_2$ | 8-F | H |
| 7-25 | 3-Py | H | H | N(Me)-$(CH_2)_2$ | 8-F | H |
| 7-26 | 2-Py | H | H | N(Me)-$(CH_2)_2$ | 8-F | H |
| 7-27 | 4-Py | H | H | N(Me)-$(CH_2)_2$ | 8-F | H |

Table 7 (cont.)

| Cpd. No. | $R^1$ | $R^2$ | $R^3$ | $A^2$-B | $R^8$ | $R^9$ |
|---|---|---|---|---|---|---|
| 7-28 | 3-Py | H | H | N(Et)-$(CH_2)_2$ | 8-F | H |
| 7-29 | 3-Py | Me | H | NH-$(CH_2)_2$ | 8-F | H |
| 7-30 | 3-Py | 3-Py | H | NH-$(CH_2)_2$ | 8-F | H |
| 7-31 | 3-Py | Me | H | N(Me)-$(CH_2)_2$ | 8-F | H |
| 7-32 | 3-Py | H | Me | N(Me)-$(CH_2)_2$ | 8-F | H |
| 7-33 | 2-Py | H | Me | N(Me)-$(CH_2)_2$ | 8-F | H |
| 7-34 | 4-Py | H | Me | N(Me)-$(CH_2)_2$ | 8-F | H |
| 7-35 | 2-Me-3-Py | H | Me | N(Me)-$(CH_2)_2$ | 8-F | H |
| 7-36 | Dme-3-Py | H | Me | N(Me)-$(CH_2)_2$ | 8-F | H |
| 7-37 | 3-Py | H | H | NH-$(CH_2)_2$ | 8-Cl | H |
| 7-38 | 2-Py | H | H | NH-$(CH_2)_2$ | 8-Cl | H |
| 7-39 | 4-Py | H | H | NH-$(CH_2)_2$ | 8-Cl | H |
| 7-40 | 2-Me-3-Py | H | H | NH-$(CH_2)_2$ | 8-Cl | H |
| 7-41 | Dme-3-Py | H | H | NH-$(CH_2)_2$ | 8-Cl | H |
| 7-42 | 3-Py | H | H | N(Me)-$(CH_2)_2$ | 8-Cl | H |
| 7-43 | 2-Py | H | H | N(Me)-$(CH_2)_2$ | 8-Cl | H |
| 7-44 | 4-Py | H | H | N(Me)-$(CH_2)_2$ | 8-Cl | H |
| 7-45 | 3-Py | H | H | N(Et)-$(CH_2)_2$ | 8-Cl | H |
| 7-46 | 3-Py | Me | H | NH-$(CH_2)_2$ | 8-Cl | H |
| 7-47 | 3-Py | 3-Py | H | NH-$(CH_2)_2$ | 8-Cl | H |
| 7-48 | 3-Py | Me | H | N(Me)-$(CH_2)_2$ | 8-Cl | H |
| 7-49 | 3-Py | H | Me | N(Me)-$(CH_2)_2$ | 8-Cl | H |
| 7-50 | 2-Py | H | Me | N(Me)-$(CH_2)_2$ | 8-Cl | H |
| 7-51 | 4-Py | H | Me | N(Me)-$(CH_2)_2$ | 8-Cl | H |
| 7-52 | 2-Me-3-Py | H | Me | N(Me)-$(CH_2)_2$ | 8-Cl | H |
| 7-53 | Dme-3-Py | H | Me | N(Me)-$(CH_2)_2$ | 8-Cl | H |
| 7-54 | 3-Py | H | H | NH-$(CH_2)_2$ | 8-Br | H |
| 7-55 | 3-Py | H | H | N(Me)-$(CH_2)_2$ | 8-Br | H |

Table 7 (cont.)

| Cpd. No. | $R^1$ | $R^2$ | $R^3$ | $A^2$-B | $R^8$ | $R^9$ |
|---|---|---|---|---|---|---|
| 7-56 | 3-Py | H | H | NH-$(CH_2)_2$ | 8-Me | H |
| 7-57 | 3-Py | H | H | N(Me)-$(CH_2)_2$ | 8-Me | H |
| 7-58 | 3-Py | H | H | NH-$(CH_2)_2$ | 8-OMe | H |
| 7-59 | 3-Py | H | H | N(Me)-$(CH_2)_2$ | 8-OMe | H |
| 7-60 | 3-Py | H | H | NH-$(CH_2)_2$ | 8-Tfm | H |
| 7-61 | 2-Py | H | H | NH-$(CH_2)_2$ | 8-Tfm | H |
| 7-62 | 4-Py | H | H | NH-$(CH_2)_2$ | 8-Tfm | H |
| 7-63 | 3-Py | H | Me | NH-$(CH_2)_2$ | 8-Tfm | H |
| 7-64 | 3-Py | H | H | N(Me)-$(CH_2)_2$ | 8-Tfm | H |
| 7-65 | 3-Py | H | H | NH-$(CH_2)_2$ | 8-OH | H |
| 7-66 | 3-Py | H | H | N(Me)-$(CH_2)_2$ | 8-OH | H |
| 7-67 | 3-Py | H | H | NH-$(CH_2)_2$ | H | 13-F |
| 7-68 | 2-Py | H | H | NH-$(CH_2)_2$ | H | 13-F |
| 7-69 | 3-Py | H | H | N(Me)-$(CH_2)_2$ | H | 13-F |
| 7-70 | 3-Py | H | Me | NH-$(CH_2)_2$ | H | 13-F |
| 7-71 | 3-Py | H | H | NH-$(CH_2)_2$ | 8-F | 13-F |
| 7-72 | 3-Py | H | H | NH-$(CH_2)_2$ | H | 13-Cl |
| 7-73 | 3-Py | H | Me | NH-$(CH_2)_2$ | H | 13-Cl |
| 7-74 | 3-Py | H | H | N(Me)-$(CH_2)_2$ | H | 13-Cl |
| 7-75 | 3-Py | H | H | NH-$(CH_2)_2$ | 8-Cl | 13-Cl |
| 7-76 | 3-Py | H | H | NH-$(CH_2)_2$ | H | 13-Me |
| 7-77 | 3-Py | H | H | NH-$(CH_2)_2$ | H | 13-OMe |
| 7-78 | 3-Py | H | H | NH-$(CH_2)_3$ | H | H |
| 7-79 | 2-Me-3-Py | H | H | NH-$(CH_2)_3$ | H | H |
| 7-80 | Dme-3-Py | H | H | NH-$(CH_2)_3$ | H | H |
| 7-81 | 2-Py | H | H | NH-$(CH_2)_3$ | H | H |
| 7-82 | 4-Py | H | H | NH-$(CH_2)_3$ | H | H |
| 7-83 | 3-Py | Me | H | NH-$(CH_2)_3$ | H | H |

Table 7 (cont.)

| Cpd. No. | $R^1$ | $R^2$ | $R^3$ | $A^2$-B | $R^8$ | $R^9$ |
|---|---|---|---|---|---|---|
| 7-84 | 3-Py | H | Me | NH-$(CH_2)_3$ | H | H |
| 7-85 | 3-Py | H | H | N(Me)-$(CH_2)_3$ | H | H |
| 7-86 | 2-Me-3-Py | H | H | N(Me)-$(CH_2)_3$ | H | H |
| 7-87 | Dme-3-Py | H | H | N(Me)-$(CH_2)_3$ | H | H |
| 7-88 | 2-Py | H | H | N(Me)-$(CH_2)_3$ | H | H |
| 7-89 | 4-Py | H | H | N(Me)-$(CH_2)_3$ | H | H |
| 7-90 | 3-Py | H | Me | N(Me)-$(CH_2)_3$ | H | H |
| 7-91 | 3-Py | H | H | NH-$(CH_2)_3$ | 8-F | H |
| 7-92 | 2-Py | H | H | NH-$(CH_2)_3$ | 8-F | H |
| 7-93 | 4-Py | H | H | NH-$(CH_2)_3$ | 8-F | H |
| 7-94 | 3-Py | H | Me | NH-$(CH_2)_3$ | 8-F | H |
| 7-95 | 3-Py | H | H | N(Me)-$(CH_2)_3$ | 8-F | H |
| 7-96 | 3-Py | H | H | NH-$(CH_2)_3$ | 8-Cl | H |
| 7-97 | 2-Py | H | H | NH-$(CH_2)_3$ | 8-Cl | H |
| 7-98 | 4-Py | H | H | NH-$(CH_2)_3$ | 8-Cl | H |
| 7-99 | 3-Py | H | Me | NH-$(CH_2)_3$ | 8-Cl | H |
| 7-100 | 3-Py | H | H | N(Me)-$(CH_2)_3$ | 8-Cl | H |
| 7-101 | 3-Py | H | H | NH-$(CH_2)_3$ | 8-Me | H |
| 7-102 | 3-Py | H | Me | NH-$(CH_2)_3$ | 8-Me | H |
| 7-103 | 3-Py | H | H | N(Me)-$(CH_2)_3$ | 8-Me | H |
| 7-104 | 3-Py | H | H | NH-$(CH_2)_3$ | 8-OMe | H |
| 7-105 | 3-Py | H | Me | NH-$(CH_2)_3$ | 8-OMe | H |
| 7-106 | 3-Py | H | H | N(Me)-$(CH_2)_3$ | 8-OMe | H |
| 7-107 | 3-Py | H | H | NH-$(CH_2)_3$ | 8-Tfm | H |
| 7-108 | 3-Py | H | Me | NH-$(CH_2)_3$ | 8-Tfm | H |
| 7-109 | 3-Py | H | H | N(Me)-$(CH_2)_3$ | 8-Tfm | H |
| 7-110 | 3-Py | H | H | NH-$(CH_2)_3$ | 8-OH | H |
| 7-111 | 3-Py | H | Me | NH-$(CH_2)_3$ | 8-OH | H |

Table 7 (cont.)

| Cpd. No. | $R^1$ | $R^2$ | $R^3$ | $A^2$-B | $R^8$ | $R^9$ |
|---|---|---|---|---|---|---|
| 7-112 | 3-Py | H | H | N(Me)-$(CH_2)_3$ | 8-OH | H |
| 7-113 | 3-Py | H | H | NH-$(CH_2)_3$ | H | 13-F |
| 7-114 | 3-Py | H | Me | NH-$(CH_2)_3$ | H | 13-F |
| 7-115 | 3-Py | H | H | N(Me)-$(CH_2)_3$ | H | 13-F |
| 7-116 | 3-Py | H | H | NH-$(CH_2)_3$ | H | 13-Cl |
| 7-117 | 3-Py | H | Me | NH-$(CH_2)_3$ | H | 13-Cl |
| 7-118 | 3-Py | H | H | N(Me)-$(CH_2)_3$ | H | 13-Cl |
| 7-119 | 3-Py | H | H | NH-$(CH_2)_3$ | H | 13-Me |
| 7-120 | 3-Py | H | Me | NH-$(CH_2)_3$ | H | 13-Me |
| 7-121 | 3-Py | H | H | N(Me)-$(CH_2)_3$ | H | 13-Me |
| 7-122 | 3-Py | H | H | NH-$(CH_2)_3$ | H | 13-OMe |
| 7-123 | 3-Py | H | Me | NH-$(CH_2)_3$ | H | 13-OMe |
| 7-124 | 3-Py | H | H | N(Me)-$(CH_2)_3$ | H | 13-OMe |
| 7-125 | 3-Py | H | H | NH-CH(Me)$CH_2$ | H | H |
| 7-126 | 3-Py | H | Me | NH-CH(Me)$CH_2$ | H | H |
| 7-127 | 3-Py | H | H | N(Me)-CH(Me)$CH_2$ | H | H |
| 7-128 | 3-Py | H | H | NH-CH(Me)$CH_2$ | 8-F | H |
| 7-129 | 3-Py | H | Me | NH-CH(Me)$CH_2$ | 8-F | H |
| 7-130 | 3-Py | H | H | N(Me)-CH(Me)$CH_2$ | 8-F | H |
| 7-131 | 3-Py | H | H | NH-CH(Me)$CH_2$ | 8-Cl | H |
| 7-132 | 3-Py | H | Me | NH-CH(Me)$CH_2$ | 8-Cl | H |
| 7-133 | 3-Py | H | H | N(Me)-CH(Me)$CH_2$ | 8-Cl | H |
| 7-134 | 3-Py | H | H | NH-CH(Me)$CH_2$ | 8-Tfm | H |
| 7-135 | 3-Py | H | Me | NH-CH(Me)$CH_2$ | 8-Tfm | H |
| 7-136 | 3-Py | H | H | N(Me)-CH(Me)$CH_2$ | 8-Tfm | H |
| 7-137 | 3-Py | H | H | NH-CH(Me)$CH_2$ | H | 13-F |
| 7-138 | 3-Py | H | Me | NH-CH(Me)$CH_2$ | H | 13-F |
| 7-139 | 3-Py | H | H | N(Me)-CH(Me)$CH_2$ | H | 13-F |

Table 7 (cont.)

| Cpd. No. | $R^1$ | $R^2$ | $R^3$ | $A^2$-B | $R^8$ | $R^9$ |
|---|---|---|---|---|---|---|
| 7-140 | 3-Py | H | H | NH-CH(Me)$CH_2$ | H | 13-Cl |
| 7-141 | 3-Py | H | Me | NH-CH(Me)$CH_2$ | H | 13-Cl |
| 7-142 | 3-Py | H | H | N(Me)-CH(Me)$CH_2$ | H | 13-Cl |
| 7-143 | 3-Py | H | H | NH-$(CH_2)_4$ | H | H |
| 7-144 | 3-Py | Me | H | NH-$(CH_2)_4$ | H | H |
| 7-145 | 3-Py | H | Me | NH-$(CH_2)_4$ | H | H |
| 7-146 | 3-Py | H | H | N(Me)-$(CH_2)_4$ | H | H |
| 7-147 | 3-Py | H | H | NH-$(CH_2)_4$ | 8-F | H |
| 7-148 | 3-Py | H | H | N(Me)-$(CH_2)_4$ | 8-F | H |
| 7-149 | 3-Py | H | H | NH-$(CH_2)_4$ | 8-Cl | H |
| 7-150 | 3-Py | H | H | N(Me)-$(CH_2)_4$ | 8-Cl | H |
| 7-151 | 3-Py | H | H | NH-$(CH_2)_4$ | 8-Me | H |
| 7-152 | 3-Py | H | H | NH-$(CH_2)_4$ | 8-OMe | H |
| 7-153 | 3-Py | H | H | NH-$(CH_2)_4$ | H | 13-F |
| 7-154 | 3-Py | H | H | NH-$(CH_2)_4$ | H | 13-Cl |
| 7-155 | 3-Py | H | H | NH-$CH_2$CH(Me)$CH_2$ | H | H |
| 7-156 | 3-Py | H | Me | NH-$CH_2$CH(Me)$CH_2$ | H | H |
| 7-157 | 3-Py | H | H | N(Me)-$CH_2$CH(Me)$CH_2$ | H | H |
| 7-158 | 3-Py | H | H | NH-$CH_2$CH(Me)$CH_2$ | 8-F | H |
| 7-159 | 3-Py | H | H | NH-$CH_2$CH(Me)$CH_2$ | 8-Cl | H |
| 7-160 | 3-Py | H | H | NH-$CH_2$CH(Me)$CH_2$ | H | 13-F |
| 7-161 | 3-Py | H | H | NH-$CH_2$CH(Me)$CH_2$ | H | 13-Cl |
| 7-162 | 3-Py | H | H | - | H | H |
| 7-163 | 2-Py | H | H | - | H | H |
| 7-164 | 4-Py | H | H | - | H | H |
| 7-165 | 2-Me-3-Py | H | H | - | H | H |
| 7-166 | Dme-3-Py | H | H | - | H | H |
| 7-167 | 3-Py | Me | H | - | H | H |

Table 7 (cont.)

| Cpd. No. | $R^1$ | $R^2$ | $R^3$ | $A^2$-B | $R^8$ | $R^9$ |
|---|---|---|---|---|---|---|
| 7-168 | 3-Py | H | Me | - | H | H |
| 7-169 | 3-Py | H | H | - | 8-F | H |
| 7-170 | 2-Py | H | H | - | 8-F | H |
| 7-171 | 4-Py | H | H | - | 8-F | H |
| 7-172 | 3-Py | Me | H | - | 8-F | H |
| 7-173 | 3-Py | H | Me | - | 8-F | H |
| 7-174 | 3-Py | H | H | - | 8-Cl | H |
| 7-175 | 2-Py | H | H | - | 8-Cl | H |
| 7-176 | 4-Py | H | H | - | 8-Cl | H |
| 7-177 | 3-Py | Me | H | - | 8-Cl | H |
| 7-178 | 3-Py | H | Me | - | 8-Cl | H |
| 7-179 | 3-Py | H | H | - | 8-Br | H |
| 7-180 | 3-Py | H | H | - | 8-Me | H |
| 7-181 | 3-Py | H | H | - | 8-OMe | H |
| 7-182 | 3-Py | H | H | - | 8-Tfm | H |
| 7-183 | 3-Py | H | H | - | 8-OH | H |
| 7-184 | 3-Py | H | H | - | H | 13-F |
| 7-185 | 3-Py | H | H | - | H | 13-Cl |

Table 8

| Cpd. No. | $R^1$ | $R^2$ | $R^3$ | $A^2$-B | $R^9$ |
|---|---|---|---|---|---|
| 8-1 | 3-Py | H | H | NH-$(CH_2)_2$ | H |
| 8-2 | 2-Py | H | H | NH-$(CH_2)_2$ | H |
| 8-3 | 4-Py | H | H | NH-$(CH_2)_2$ | H |
| 8-4 | 2-Me-3-Py | H | H | NH-$(CH_2)_2$ | H |
| 8-5 | Dme-3-Py | H | H | NH-$(CH_2)_2$ | H |
| 8-6 | 3-Py | H | H | N(Me)-$(CH_2)_2$ | H |
| 8-7 | 2-Py | H | H | N(Me)-$(CH_2)_2$ | H |
| 8-8 | 4-Py | H | H | N(Me)-$(CH_2)_2$ | H |
| 8-9 | 3-Py | Me | H | NH-$(CH_2)_2$ | H |
| 8-10 | 3-Py | Me | H | N(Me)-$(CH_2)_2$ | H |
| 8-11 | 3-Py | H | H | NH-$(CH_2)_2$ | 13-F |
| 8-12 | 3-Py | H | H | N(Me)-$(CH_2)_2$ | 13-F |
| 8-13 | 3-Py | H | Me | NH-$(CH_2)_2$ | 13-F |
| 8-14 | 3-Py | H | H | NH-$(CH_2)_2$ | 13-Cl |
| 8-15 | 3-Py | H | Me | NH-$(CH_2)_2$ | 13-Cl |
| 8-16 | 3-Py | H | H | NH-$(CH_2)_3$ | H |
| 8-17 | 2-Py | H | H | NH-$(CH_2)_3$ | H |
| 8-18 | 4-Py | H | H | NH-$(CH_2)_3$ | H |
| 8-19 | 3-Py | Me | H | NH-$(CH_2)_3$ | H |
| 8-20 | 3-Py | H | Me | NH-$(CH_2)_3$ | H |
| 8-21 | 3-Py | H | H | N(Me)-$(CH_2)_3$ | H |
| 8-22 | 3-Py | H | H | NH-$(CH_2)_3$ | 13-F |
| 8-23 | 3-Py | H | Me | NH-$(CH_2)_3$ | 13-F |
| 8-24 | 3-Py | H | H | N(Me)-$(CH_2)_3$ | 13-F |
| 8-25 | 3-Py | H | H | NH-$(CH_2)_3$ | 13-Cl |
| 8-26 | 3-Py | H | Me | NH-$(CH_2)_3$ | 13-Cl |
| 8-27 | 3-Py | H | H | N(Me)-$(CH_2)_3$ | 13-Cl |
| 8-28 | 3-Py | H | H | NH-CH(Me)$CH_2$ | H |

Table 8 (cont.)

| Cpd. No. | $R^1$ | $R^2$ | $R^3$ | $A^2$-B | $R^9$ |
|---|---|---|---|---|---|
| 8-29 | 3-Py | H | Me | NH-CH(Me)$CH_2$ | H |
| 8-30 | 3-Py | H | H | N(Me)-CH(Me)$CH_2$ | H |
| 8-31 | 3-Py | H | H | NH-CH(Me)$CH_2$ | 13-F |
| 8-32 | 3-Py | H | H | NH-CH(Me)$CH_2$ | 13-Cl |
| 8-33 | 3-Py | H | H | NH-$(CH_2)_4$ | H |
| 8-34 | 3-Py | Me | H | NH-$(CH_2)_4$ | H |
| 8-35 | 3-Py | H | Me | NH-$(CH_2)_4$ | H |
| 8-36 | 3-Py | H | H | N(Me)-$(CH_2)_4$ | H |
| 8-37 | 3-Py | H | H | NH-$(CH_2)_4$ | 13-F |
| 8-38 | 3-Py | H | H | NH-$(CH_2)_4$ | 13-Cl |
| 8-39 | 3-Py | H | H | NH-$CH_2$CH(Me)$CH_2$ | H |
| 8-40 | 3-Py | H | H | NH-$CH_2$CH(Me)$CH_2$ | 13-F |
| 8-41 | 3-Py | H | H | NH-$CH_2$CH(Me)$CH_2$ | 13-Cl |
| 8-42 | 3-Py | H | H | NH-$(CH_2)_2$ | H |
| 8-43 | 2-Py | H | H | NH-$(CH_2)_2$ | H |
| 8-44 | 4-Py | H | H | NH-$(CH_2)_2$ | H |
| 8-45 | 2-Me-3-Py | H | H | NH-$(CH_2)_2$ | H |
| 8-46 | Dme-3-Py | H | H | NH-$(CH_2)_2$ | H |
| 8-47 | 3-Py | Me | H | NH-$(CH_2)_2$ | H |
| 8-48 | 3-Py | H | H | NH-$(CH_2)_2$ | 13-F |
| 8-49 | 3-Py | H | Me | NH-$(CH_2)_2$ | 13-F |
| 8-50 | 3-Py | H | H | NH-$(CH_2)_2$ | 13-Cl |
| 8-51 | 3-Py | H | Me | NH-$(CH_2)_2$ | 13-Cl |

Table 9

| Cpd. No. | $R^1$ | $R^2$ | $R^3$ | $A^2$-B | $R^8$ |
|---|---|---|---|---|---|
| 9-1 | 3-Py | H | H | NH-$(CH_2)_2$ | H |
| 9-2 | 2-Py | H | H | NH-$(CH_2)_2$ | H |
| 9-3 | 4-Py | H | H | NH-$(CH_2)_2$ | H |
| 9-4 | 2-Me-3-Py | H | H | NH-$(CH_2)_2$ | H |
| 9-5 | Dme-3-Py | H | H | NH-$(CH_2)_2$ | H |
| 9-6 | 3-Py | H | H | N(Me)-$(CH_2)_2$ | H |
| 9-7 | 2-Py | H | H | N(Me)-$(CH_2)_2$ | H |
| 9-8 | 4-Py | H | H | N(Me)-$(CH_2)_2$ | H |
| 9-9 | 3-Py | H | H | N(Et)-$(CH_2)_2$ | H |
| 9-10 | 3-Py | Me | H | NH-$(CH_2)_2$ | H |
| 9-11 | 3-Py | Et | H | NH-$(CH_2)_2$ | H |
| 9-12 | 3-Py | 3-Py | H | NH-$(CH_2)_2$ | H |
| 9-13 | 3-Py | Me | H | N(Me)-$(CH_2)_2$ | H |
| 9-14 | 3-Py | H | Me | N(Me)-$(CH_2)_2$ | H |
| 9-15 | 2-Py | H | Me | N(Me)-$(CH_2)_2$ | H |
| 9-16 | 4-Py | H | Me | N(Me)-$(CH_2)_2$ | H |
| 9-17 | 2-Me-3-Py | H | Me | N(Me)-$(CH_2)_2$ | H |
| 9-18 | Dme-3-Py | H | Me | N(Me)-$(CH_2)_2$ | H |
| 9-19 | 3-Py | H | Et | N(Me)-$(CH_2)_2$ | H |
| 9-20 | 3-Py | H | H | NH-$(CH_2)_2$ | 8-F |
| 9-21 | 2-Py | H | H | NH-$(CH_2)_2$ | 8-F |
| 9-22 | 4-Py | H | H | NH-$(CH_2)_2$ | 8-F |
| 9-23 | 2-Me-3-Py | H | H | NH-$(CH_2)_2$ | 8-F |
| 9-24 | Dme-3-Py | H | H | NH-$(CH_2)_2$ | 8-F |
| 9-25 | 3-Py | H | H | N(Me)-$(CH_2)_2$ | 8-F |
| 9-26 | 2-Py | H | H | N(Me)-$(CH_2)_2$ | 8-F |
| 9-27 | 4-Py | H | H | N(Me)-$(CH_2)_2$ | 8-F |
| 9-28 | 3-Py | H | H | N(Et)-$(CH_2)_2$ | 8-F |

Table 9 (cont.)

| Cpd. No. | $R^1$ | $R^2$ | $R^3$ | $A^2$-B | $R^8$ |
|---|---|---|---|---|---|
| 9-29 | 3-Py | Me | H | NH-$(CH_2)_2$ | 8-F |
| 9-30 | 3-Py | 3-Py | H | NH-$(CH_2)_2$ | 8-F |
| 9-31 | 3-Py | Me | H | N(Me)-$(CH_2)_2$ | 8-F |
| 9-32 | 3-Py | H | Me | N(Me)-$(CH_2)_2$ | 8-F |
| 9-33 | 2-Py | H | Me | N(Me)-$(CH_2)_2$ | 8-F |
| 9-34 | 4-Py | H | Me | N(Me)-$(CH_2)_2$ | 8-F |
| 9-35 | 2-Me-3-Py | H | Me | N(Me)-$(CH_2)_2$ | 8-F |
| 9-36 | Dme-3-Py | H | Me | N(Me)-$(CH_2)_2$ | 8-F |
| 9-37 | 3-Py | H | H | NH-$(CH_2)_2$ | 8-Cl |
| 9-38 | 2-Py | H | H | NH-$(CH_2)_2$ | 8-Cl |
| 9-39 | 4-Py | H | H | NH-$(CH_2)_2$ | 8-Cl |
| 9-40 | 2-Me-3-Py | H | H | NH-$(CH_2)_2$ | 8-Cl |
| 9-41 | Dme-3-Py | H | H | NH-$(CH_2)_2$ | 8-Cl |
| 9-42 | 3-Py | H | H | N(Me)-$(CH_2)_2$ | 8-Cl |
| 9-43 | 2-Py | H | H | N(Me)-$(CH_2)_2$ | 8-Cl |
| 9-44 | 4-Py | H | H | N(Me)-$(CH_2)_2$ | 8-Cl |
| 9-45 | 3-Py | H | H | N(Et)-$(CH_2)_2$ | 8-Cl |
| 9-46 | 3-Py | Me | H | NH-$(CH_2)_2$ | 8-Cl |
| 9-47 | 3-Py | 3-Py | H | NH-$(CH_2)_2$ | 8-Cl |
| 9-48 | 3-Py | Me | H | N(Me)-$(CH_2)_2$ | 8-Cl |
| 9-49 | 3-Py | H | Me | N(Me)-$(CH_2)_2$ | 8-Cl |
| 9-50 | 2-Py | H | Me | N(Me)-$(CH_2)_2$ | 8-Cl |
| 9-51 | 4-Py | H | Me | N(Me)-$(CH_2)_2$ | 8-Cl |
| 9-52 | 2-Me-3-Py | H | Me | N(Me)-$(CH_2)_2$ | 8-Cl |
| 9-53 | Dme-3-Py | H | Me | N(Me)-$(CH_2)_2$ | 8-Cl |
| 9-54 | 3-Py | H | H | NH-$(CH_2)_2$ | 8-Br |
| 9-55 | 3-Py | H | H | N(Me)-$(CH_2)_2$ | 8-Br |
| 9-56 | 3-Py | H | H | NH-$(CH_2)_2$ | 8-Me |

Table 9 (cont.)

| Cpd. No. | $R^1$ | $R^2$ | $R^3$ | $A^2$-B | $R^8$ |
|---|---|---|---|---|---|
| 9-57 | 3-Py | H | H | $N(Me)-(CH_2)_2$ | 8-Me |
| 9-58 | 3-Py | H | H | $NH-(CH_2)_2$ | 8-OMe |
| 9-59 | 3-Py | H | H | $N(Me)-(CH_2)_2$ | 8-OMe |
| 9-60 | 3-Py | H | H | $NH-(CH_2)_2$ | 8-Tfm |
| 9-61 | 2-Py | H | H | $NH-(CH_2)_2$ | 8-Tfm |
| 9-62 | 4-Py | H | H | $NH-(CH_2)_2$ | 8-Tfm |
| 9-63 | 3-Py | H | Me | $NH-(CH_2)_2$ | 8-Tfm |
| 9-64 | 3-Py | H | H | $N(Me)-(CH_2)_2$ | 8-Tfm |
| 9-65 | 3-Py | H | H | $NH-(CH_2)_2$ | 8-OH |
| 9-66 | 3-Py | H | H | $N(Me)-(CH_2)_2$ | 8-OH |
| 9-67 | 3-Py | H | H | $NH-(CH_2)_3$ | H |
| 9-68 | 2-Me-3-Py | H | H | $NH-(CH_2)_3$ | H |
| 9-69 | Dme-3-Py | H | H | $NH-(CH_2)_3$ | H |
| 9-70 | 2-Py | H | H | $NH-(CH_2)_3$ | H |
| 9-71 | 4-Py | H | H | $NH-(CH_2)_3$ | H |
| 9-72 | 3-Py | Me | H | $NH-(CH_2)_3$ | H |
| 9-73 | 3-Py | H | Me | $NH-(CH_2)_3$ | H |
| 9-74 | 3-Py | H | H | $N(Me)-(CH_2)_3$ | H |
| 9-75 | 2-Me-3-Py | H | H | $N(Me)-(CH_2)_3$ | H |
| 9-76 | Dme-3-Py | H | H | $N(Me)-(CH_2)_3$ | H |
| 9-77 | 2-Py | H | H | $N(Me)-(CH_2)_3$ | H |
| 9-78 | 4-Py | H | H | $N(Me)-(CH_2)_3$ | H |
| 9-79 | 3-Py | H | Me | $N(Me)-(CH_2)_3$ | H |
| 9-80 | 3-Py | H | H | $NH-(CH_2)_3$ | 8-F |
| 9-81 | 2-Py | H | H | $NH-(CH_2)_3$ | 8-F |
| 9-82 | 4-Py | H | H | $NH-(CH_2)_3$ | 8-F |
| 9-83 | 3-Py | H | Me | $NH-(CH_2)_3$ | 8-F |
| 9-84 | 3-Py | H | H | $N(Me)-(CH_2)_3$ | 8-F |

Table 9 (cont.)

| Cpd. No. | $R^1$ | $R^2$ | $R^3$ | $A^2$-B | $R^8$ |
|---|---|---|---|---|---|
| 9-85 | 3-Py | H | H | NH-$(CH_2)_3$ | 8-Cl |
| 9-86 | 2-Py | H | H | NH-$(CH_2)_3$ | 8-Cl |
| 9-87 | 4-Py | H | H | NH-$(CH_2)_3$ | 8-Cl |
| 9-88 | 3-Py | H | Me | NH-$(CH_2)_3$ | 8-Cl |
| 9-89 | 3-Py | H | H | N(Me)-$(CH_2)_3$ | 8-Cl |
| 9-90 | 3-Py | H | H | NH-$(CH_2)_3$ | 8-Me |
| 9-91 | 3-Py | H | Me | NH-$(CH_2)_3$ | 8-Me |
| 9-92 | 3-Py | H | H | N(Me)-$(CH_2)_3$ | 8-Me |
| 9-93 | 3-Py | H | H | NH-$(CH_2)_3$ | 8-OMe |
| 9-94 | 3-Py | H | Me | NH-$(CH_2)_3$ | 8-OMe |
| 9-95 | 3-Py | H | H | N(Me)-$(CH_2)_3$ | 8-OMe |
| 9-96 | 3-Py | H | H | NH-$(CH_2)_3$ | 8-Tfm |
| 9-97 | 3-Py | H | Me | NH-$(CH_2)_3$ | 8-Tfm |
| 9-98 | 3-Py | H | H | N(Me)-$(CH_2)_3$ | 8-Tfm |
| 9-99 | 3-Py | H | H | NH-$(CH_2)_3$ | 8-OH |
| 9-100 | 3-Py | H | Me | NH-$(CH_2)_3$ | 8-OH |
| 9-101 | 3-Py | H | H | N(Me)-$(CH_2)_3$ | 8-OH |
| 9-102 | 3-Py | H | H | NH-CH(Me)$CH_2$ | H |
| 9-103 | 3-Py | H | H | N(Me)-CH(Me)$CH_2$ | H |
| 9-104 | 3-Py | H | H | NH-CH(Me)$CH_2$ | 8-F |
| 9-105 | 3-Py | H | H | N(Me)-CH(Me)$CH_2$ | 8-F |
| 9-106 | 3-Py | H | Me | N(Me)-CH(Me)$CH_2$ | 8-F |
| 9-107 | 3-Py | H | H | NH-CH(Me)$CH_2$ | 8-Cl |
| 9-108 | 3-Py | H | H | N(Me)-CH(Me)$CH_2$ | 8-Cl |
| 9-109 | 3-Py | H | Me | N(Me)-CH(Me)$CH_2$ | 8-Cl |
| 9-110 | 3-Py | H | H | NH-CH(Me)$CH_2$ | 8-Tfm |
| 9-111 | 3-Py | H | Me | NH-CH(Me)$CH_2$ | 8-Tfm |
| 9-112 | 3-Py | H | Me | N(Me)-CH(Me)$CH_2$ | 8-Tfm |

Table 9 (cont.)

| Cpd. No. | $R^1$ | $R^2$ | $R^3$ | $A^2$-B | $R^8$ |
|---|---|---|---|---|---|
| 9-113 | 3-Py | H | H | NH-$(CH_2)_4$ | H |
| 9-114 | 3-Py | Me | H | NH-$(CH_2)_4$ | H |
| 9-115 | 3-Py | H | Me | NH-$(CH_2)_4$ | H |
| 9-116 | 3-Py | H | H | N(Me)-$(CH_2)_4$ | H |
| 9-117 | 3-Py | H | H | NH-$(CH_2)_4$ | 8-F |
| 9-118 | 3-Py | H | H | N(Me)-$(CH_2)_4$ | 8-F |
| 9-119 | 3-Py | H | H | NH-$(CH_2)_4$ | 8-Cl |
| 9-120 | 3-Py | H | H | N(Me)-$(CH_2)_4$ | 8-Cl |
| 9-121 | 3-Py | H | H | NH-$(CH_2)_4$ | 8-Me |
| 9-122 | 3-Py | H | H | NH-$(CH_2)_4$ | 8-OMe |
| 9-123 | 3-Py | H | H | NH-$CH_2CH(Me)CH_2$ | H |
| 9-124 | 3-Py | H | Me | NH-$CH_2CH(Me)CH_2$ | H |
| 9-125 | 3-Py | H | H | N(Me)-$CH_2CH(Me)CH_2$ | H |
| 9-126 | 3-Py | H | H | NH-$CH_2CH(Me)CH_2$ | 8-F |
| 9-127 | 3-Py | H | H | NH-$CH_2CH(Me)CH_2$ | 8-Cl |
| 9-128 | 3-Py | H | H | - | H |
| 9-129 | 2-Py | H | H | - | H |
| 9-130 | 4-Py | H | H | - | H |
| 9-131 | 2-Me-3-Py | H | H | - | H |
| 9-132 | Dme-3-Py | H | H | - | H |
| 9-133 | 3-Py | Me | H | - | H |
| 9-134 | 3-Py | Et | H | - | H |
| 9-135 | 3-Py | 3-Py | H | - | H |
| 9-136 | 3-Py | H | Me | - | H |
| 9-137 | 3-Py | H | Et | - | H |
| 9-138 | 3-Py | H | H | - | 8-F |
| 9-139 | 2-Py | H | H | - | 8-F |
| 9-140 | 4-Py | H | H | - | 8-F |

Table 9 (cont.)

| Cpd. No. | $R^1$ | $R^2$ | $R^3$ | $A^2$-B | $R^8$ |
|---|---|---|---|---|---|
| 9-141 | 3-Py | Me | H | - | 8-F |
| 9-142 | 3-Py | H | Me | - | 8-F |
| 9-143 | 3-Py | H | H | - | 8-Cl |
| 9-144 | 2-Py | H | H | - | 8-Cl |
| 9-145 | 4-Py | H | H | - | 8-Cl |
| 9-146 | 3-Py | Me | H | - | 8-Cl |
| 9-147 | 3-Py | H | Me | - | 8-Cl |
| 9-148 | 3-Py | H | H | - | 8-Me |
| 9-149 | 3-Py | H | H | - | 8-OMe |
| 9-150 | 3-Py | H | H | - | 8-Tfm |
| 9-151 | 3-Py | H | Me | - | 8-Tfm |
| 9-152 | 3-Py | H | H | - | 8-OH |

Table 10

| Cpd. No. | $R^1$ | $R^2$ | $R^3$ | n | B | $R^8$ | $R^9$ |
|---|---|---|---|---|---|---|---|
| 10-1 | 3-Py | H | H | 2 | $(CH_2)_2$ | H | H |
| 10-2 | 2-Py | H | H | 2 | $(CH_2)_2$ | H | H |
| 10-3 | 4-Py | H | H | 2 | $(CH_2)_2$ | H | H |
| 10-4 | 2-Me-3-Py | H | H | 2 | $(CH_2)_2$ | H | H |
| 10-5 | Dme-3-Py | H | H | 2 | $(CH_2)_2$ | H | H |
| 10-6 | 3-Py | Me | H | 2 | $(CH_2)_2$ | H | H |
| 10-7 | 3-Py | H | Me | 2 | $(CH_2)_2$ | H | H |
| 10-8 | 3-Py | H | H | 2 | $(CH_2)_2$ | 8-F | H |
| 10-9 | 2-Py | H | H | 2 | $(CH_2)_2$ | 8-F | H |
| 10-10 | 4-Py | H | H | 2 | $(CH_2)_2$ | 8-F | H |
| 10-11 | 3-Py | Me | H | 2 | $(CH_2)_2$ | 8-F | H |
| 10-12 | 3-Py | H | Me | 2 | $(CH_2)_2$ | 8-F | H |
| 10-13 | 3-Py | H | H | 2 | $(CH_2)_2$ | 7-F | H |
| 10-14 | 3-Py | H | H | 2 | $(CH_2)_2$ | 8-Cl | H |
| 10-15 | 3-Py | Me | H | 2 | $(CH_2)_2$ | 8-Cl | H |
| 10-16 | 3-Py | H | Me | 2 | $(CH_2)_2$ | 8-Cl | H |
| 10-17 | 3-Py | H | H | 2 | $(CH_2)_2$ | 8-Me | H |
| 10-18 | 3-Py | H | H | 2 | $(CH_2)_2$ | 8-OMe | H |
| 10-19 | 3-Py | H | H | 2 | $(CH_2)_2$ | 8-Tfm | H |
| 10-20 | 3-Py | H | H | 2 | $(CH_2)_2$ | 8-OH | H |
| 10-21 | 3-Py | H | H | 2 | $(CH_2)_2$ | H | 13-F |
| 10-22 | 3-Py | H | Me | 2 | $(CH_2)_2$ | H | 13-F |
| 10-23 | 3-Py | H | H | 2 | $(CH_2)_2$ | H | 12-F |
| 10-24 | 3-Py | H | H | 2 | $(CH_2)_2$ | H | 13-Cl |
| 10-25 | 3-Py | H | Me | 2 | $(CH_2)_2$ | H | 13-Cl |
| 10-26 | 3-Py | H | H | 2 | $(CH_2)_3$ | H | H |
| 10-27 | 2-Py | H | H | 2 | $(CH_2)_3$ | H | H |
| 10-28 | 4-Py | H | H | 2 | $(CH_2)_3$ | H | H |

Table 10 (cont.)

| Cpd. No. | $R^1$ | $R^2$ | $R^3$ | n | B | $R^8$ | $R^9$ |
|---|---|---|---|---|---|---|---|
| 10-29 | 3-Py | H | Me | 2 | $(CH_2)_3$ | H | H |
| 10-30 | 3-Py | H | H | 2 | $(CH_2)_3$ | 8-F | H |
| 10-31 | 3-Py | H | Me | 2 | $(CH_2)_3$ | 8-F | H |
| 10-32 | 3-Py | H | H | 2 | $(CH_2)_3$ | 8-Cl | H |
| 10-33 | 3-Py | H | Me | 2 | $(CH_2)_3$ | 8-Cl | H |
| 10-34 | 3-Py | H | H | 2 | $(CH_2)_3$ | 8-Tfm | H |
| 10-35 | 3-Py | H | H | 2 | $(CH_2)_3$ | H | 13-F |
| 10-36 | 3-Py | H | H | 2 | $(CH_2)_3$ | H | 13-Cl |
| 10-37 | 3-Py | H | H | 2 | $CH(Me)CH_2$ | H | H |
| 10-38 | 3-Py | H | H | 2 | $CH(Me)CH_2$ | 8-F | H |
| 10-39 | 3-Py | H | H | 2 | $CH(Me)CH_2$ | 8-Cl | H |
| 10-40 | 3-Py | H | H | 2 | $CH(Me)CH_2$ | 8-Tfm | H |
| 10-41 | 3-Py | H | H | 2 | $CH(Me)CH_2$ | H | 13-F |
| 10-42 | 3-Py | H | H | 2 | $CH(Me)CH_2$ | H | 13-Cl |
| 10-43 | 3-Py | H | H | 2 | $(CH_2)_4$ | H | H |
| 10-44 | 3-Py | H | H | 2 | $(CH_2)_4$ | 8-F | H |
| 10-45 | 3-Py | H | H | 2 | $(CH_2)_4$ | 8-Cl | H |
| 10-46 | 3-Py | H | H | 2 | $(CH_2)_4$ | H | 13-F |
| 10-47 | 3-Py | H | H | 2 | $(CH_2)_4$ | H | 13-Cl |
| 10-48 | 3-Py | H | H | 2 | $CH_2CH(Me)CH_2$ | H | H |
| 10-49 | 3-Py | H | H | 3 | $(CH_2)_2$ | H | H |
| 10-50 | 2-Py | H | H | 3 | $(CH_2)_2$ | H | H |
| 10-51 | 4-Py | H | H | 3 | $(CH_2)_2$ | H | H |
| 10-52 | 2-Me-3-Py | H | H | 3 | $(CH_2)_2$ | H | H |
| 10-53 | Dme-3-Py | H | H | 3 | $(CH_2)_2$ | H | H |
| 10-54 | 3-Py | Me | H | 3 | $(CH_2)_2$ | H | H |
| 10-55 | 3-Py | H | Me | 3 | $(CH_2)_2$ | H | H |
| 10-56 | 3-Py | H | H | 3 | $(CH_2)_2$ | 8-F | H |

Table 10 (cont.)

| Cpd. No. | $R^1$ | $R^2$ | $R^3$ | n | B | $R^8$ | $R^9$ |
|---|---|---|---|---|---|---|---|
| 10-57 | 3-Py | H | H | 3 | $(CH_2)_2$ | 8-Cl | H |
| 10-58 | 3-Py | H | H | 3 | $(CH_2)_2$ | 8-Me | H |
| 10-59 | 3-Py | H | H | 3 | $(CH_2)_2$ | 8-OMe | H |
| 10-60 | 3-Py | H | H | 3 | $(CH_2)_2$ | 8-Tfm | H |
| 10-61 | 3-Py | H | H | 3 | $(CH_2)_2$ | 8-OH | H |
| 10-62 | 3-Py | H | H | 3 | $(CH_2)_2$ | H | 13-F |
| 10-63 | 3-Py | H | H | 3 | $(CH_2)_2$ | H | 13-Cl |
| 10-64 | 3-Py | H | H | 3 | $(CH_2)_3$ | H | H |
| 10-65 | 2-Py | H | H | 3 | $(CH_2)_3$ | H | H |
| 10-66 | 4-Py | H | H | 3 | $(CH_2)_3$ | H | H |
| 10-67 | 3-Py | H | H | 3 | $(CH_2)_3$ | 8-F | H |
| 10-68 | 3-Py | H | H | 3 | $(CH_2)_3$ | 8-Cl | H |
| 10-69 | 3-Py | H | H | 3 | $(CH_2)_3$ | 8-Tfm | H |
| 10-70 | 3-Py | H | H | 3 | $(CH_2)_3$ | H | 13-F |
| 10-71 | 3-Py | H | H | 3 | $(CH_2)_3$ | H | 13-Cl |
| 10-72 | 3-Py | H | H | 3 | $CH(Me)CH_2$ | H | H |
| 10-73 | 3-Py | H | H | 3 | $(CH_2)_4$ | H | H |
| 10-74 | 3-Py | H | H | 3 | $(CH_2)_4$ | 8-F | H |
| 10-75 | 3-Py | H | H | 3 | $(CH_2)_4$ | 8-Cl | H |
| 10-76 | 3-Py | H | H | 3 | $(CH_2)_4$ | H | 13-F |
| 10-77 | 3-Py | H | H | 3 | $(CH_2)_4$ | H | 13-Cl |
| 10-78 | 3-Py | H | H | 3 | $CH_2CH(Me)CH_2$ | H | H |

Table 11

| Cpd. No. | $R^1$ | $R^2$ | $R^3$ | n | B | $R^9$ |
|---|---|---|---|---|---|---|
| 11-1 | 3-Py | H | H | 2 | $(CH_2)_2$ | H |
| 11-2 | 2-Py | H | H | 2 | $(CH_2)_2$ | H |
| 11-3 | 4-Py | H | H | 2 | $(CH_2)_2$ | H |
| 11-4 | 2-Me-3-Py | H | H | 2 | $(CH_2)_2$ | H |
| 11-5 | Dme-3-Py | H | H | 2 | $(CH_2)_2$ | H |
| 11-6 | 3-Py | Me | H | 2 | $(CH_2)_2$ | H |
| 11-7 | 3-Py | H | Me | 2 | $(CH_2)_2$ | H |
| 11-8 | 3-Py | H | H | 2 | $(CH_2)_2$ | 13-F |
| 11-9 | 3-Py | H | H | 2 | $(CH_2)_2$ | 12-F |
| 11-10 | 3-Py | H | H | 2 | $(CH_2)_2$ | 13-Cl |
| 11-11 | 3-Py | H | H | 2 | $(CH_2)_3$ | H |
| 11-12 | 2-Py | H | H | 2 | $(CH_2)_3$ | H |
| 11-13 | 4-Py | H | H | 2 | $(CH_2)_3$ | H |
| 11-14 | 3-Py | H | H | 2 | $(CH_2)_3$ | 13-F |
| 11-15 | 3-Py | H | H | 2 | $(CH_2)_3$ | 13-Cl |
| 11-16 | 3-Py | H | H | 2 | $CH(Me)CH_2$ | H |
| 11-17 | 3-Py | H | H | 2 | $(CH_2)_4$ | H |
| 11-18 | 3-Py | H | H | 2 | $(CH_2)_4$ | 13-F |
| 11-19 | 3-Py | H | H | 2 | $(CH_2)_4$ | 13-Cl |
| 11-20 | 3-Py | H | H | 2 | $CH_2CH(Me)CH_2$ | H |
| 11-21 | 3-Py | H | H | 3 | $(CH_2)_2$ | H |
| 11-22 | 2-Py | H | H | 3 | $(CH_2)_2$ | H |
| 11-23 | 4-Py | H | H | 3 | $(CH_2)_2$ | H |
| 11-24 | 2-Me-3-Py | H | H | 3 | $(CH_2)_2$ | H |
| 11-25 | Dme-3-Py | H | H | 3 | $(CH_2)_2$ | H |
| 11-26 | 3-Py | H | H | 3 | $(CH_2)_2$ | 13-Cl |
| 11-27 | 3-Py | H | H | 3 | $(CH_2)_3$ | H |
| 11-28 | 2-Py | H | H | 3 | $(CH_2)_3$ | H |

Table 11 (cont.)

| Cpd. No. | $R^1$ | $R^2$ | $R^3$ | n | B | $R^9$ |
|---|---|---|---|---|---|---|
| 11-29 | 4-Py | H | H | 3 | $(CH_2)_3$ | H |
| 11-30 | 3-Py | H | H | 3 | $(CH_2)_3$ | 13-F |
| 11-31 | 3-Py | H | H | 3 | $(CH_2)_3$ | 13-Cl |
| 11-32 | 3-Py | H | H | 3 | $CH(Me)CH_2$ | H |
| 11-33 | 3-Py | H | H | 3 | $(CH_2)_4$ | H |
| 11-34 | 3-Py | H | H | 3 | $(CH_2)_4$ | 13-F |
| 11-35 | 3-Py | H | H | 3 | $(CH_2)_4$ | 13-Cl |
| 11-36 | 3-Py | H | H | 3 | $CH_2CH(Me)CH_2$ | H |

Table 12

| Cpd. No. | $R^1$ | $R^2$ | $R^3$ | n | B | $R^8$ |
|---|---|---|---|---|---|---|
| 12-1 | 3-Py | H | H | 2 | $(CH_2)_2$ | H |
| 12-2 | 2-Py | H | H | 2 | $(CH_2)_2$ | H |
| 12-3 | 4-Py | H | H | 2 | $(CH_2)_2$ | H |
| 12-4 | 2-Me-3-Py | H | H | 2 | $(CH_2)_2$ | H |
| 12-5 | Dme-3-Py | H | H | 2 | $(CH_2)_2$ | H |
| 12-6 | 3-Py | Me | H | 2 | $(CH_2)_2$ | H |
| 12-7 | 3-Py | H | Me | 2 | $(CH_2)_2$ | H |
| 12-8 | 3-Py | H | H | 2 | $(CH_2)_2$ | 8-F |
| 12-9 | 2-Py | H | H | 2 | $(CH_2)_2$ | 8-F |
| 12-10 | 4-Py | H | H | 2 | $(CH_2)_2$ | 8-F |
| 12-11 | 3-Py | Me | H | 2 | $(CH_2)_2$ | 8-F |
| 12-12 | 3-Py | H | Me | 2 | $(CH_2)_2$ | 8-F |
| 12-13 | 3-Py | H | H | 2 | $(CH_2)_2$ | 7-F |
| 12-14 | 3-Py | H | H | 2 | $(CH_2)_2$ | 8-Cl |
| 12-15 | 3-Py | Me | H | 2 | $(CH_2)_2$ | 8-Cl |
| 12-16 | 3-Py | H | Me | 2 | $(CH_2)_2$ | 8-Cl |
| 12-17 | 3-Py | H | H | 2 | $(CH_2)_2$ | 8-Me |
| 12-18 | 3-Py | H | H | 2 | $(CH_2)_2$ | 8-OMe |
| 12-19 | 3-Py | H | H | 2 | $(CH_2)_2$ | 8-Tfm |
| 12-20 | 3-Py | H | H | 2 | $(CH_2)_2$ | 8-OH |
| 12-21 | 3-Py | H | H | 2 | $(CH_2)_3$ | H |
| 12-22 | 2-Py | H | H | 2 | $(CH_2)_3$ | H |
| 12-23 | 4-Py | H | H | 2 | $(CH_2)_3$ | H |
| 12-24 | 3-Py | H | Me | 2 | $(CH_2)_3$ | H |
| 12-25 | 3-Py | H | H | 2 | $(CH_2)_3$ | 8-F |
| 12-26 | 3-Py | H | H | 2 | $(CH_2)_3$ | 8-Cl |
| 12-27 | 3-Py | H | H | 2 | $(CH_2)_3$ | 8-Tfm |
| 12-28 | 3-Py | H | H | 2 | $CH(Me)CH_2$ | H |

Table 12 (cont.)

| Cpd. No. | $R^1$ | $R^2$ | $R^3$ | n | B | $R^8$ |
|---|---|---|---|---|---|---|
| 12-29 | 3-Py | H | H | 2 | $(CH_2)_4$ | H |
| 12-30 | 3-Py | H | H | 2 | $(CH_2)_4$ | 8-F |
| 12-31 | 3-Py | H | H | 2 | $(CH_2)_4$ | 8-Cl |
| 12-32 | 3-Py | H | H | 2 | $CH_2CH(Me)CH_2$ | H |
| 12-33 | 3-Py | H | H | 3 | $(CH_2)_2$ | H |
| 12-34 | 2-Py | H | H | 3 | $(CH_2)_2$ | H |
| 12-35 | 4-Py | H | H | 3 | $(CH_2)_2$ | H |
| 12-36 | 2-Me-3-Py | H | H | 3 | $(CH_2)_2$ | H |
| 12-37 | Dme-3-Py | H | H | 3 | $(CH_2)_2$ | H |
| 12-38 | 3-Py | H | H | 3 | $(CH_2)_2$ | 8-F |
| 12-39 | 3-Py | H | H | 3 | $(CH_2)_2$ | 8-Cl |
| 12-40 | 3-Py | H | H | 3 | $(CH_2)_2$ | 8-Me |
| 12-41 | 3-Py | H | H | 3 | $(CH_2)_2$ | 8-OMe |
| 12-42 | 3-Py | H | H | 3 | $(CH_2)_2$ | 8-Tfm |
| 12-43 | 3-Py | H | H | 3 | $(CH_2)_2$ | 8-OH |
| 12-44 | 3-Py | H | H | 3 | $(CH_2)_3$ | H |
| 12-45 | 2-Py | H | H | 3 | $(CH_2)_3$ | H |
| 12-46 | 4-Py | H | H | 3 | $(CH_2)_3$ | H |
| 12-47 | 3-Py | H | H | 3 | $(CH_2)_3$ | 8-F |
| 12-48 | 3-Py | H | H | 3 | $(CH_2)_3$ | 8-Cl |
| 12-49 | 3-Py | H | H | 3 | $(CH_2)_3$ | 8-Tfm |
| 12-50 | 3-Py | H | H | 3 | $CH(Me)CH_2$ | H |
| 12-51 | 3-Py | H | H | 3 | $(CH_2)_4$ | H |
| 12-52 | 3-Py | H | H | 3 | $(CH_2)_4$ | 8-F |
| 12-53 | 3-Py | H | H | 3 | $(CH_2)_4$ | 8-Cl |
| 12-54 | 3-Py | H | H | 3 | $CH_2CH(Me)CH_2$ | H |

Of the compounds listed above, preferred compounds are Compounds No.: 1-1, 1-2, 1-3, 1-6, 1-12, 1-17, 1-23, 1-24, 1-25, 1-26, 1-27, 1-28, 1-32, 1-33, 1-34, 1-36, 1-37, 1-46, 1-47, 1-48, 1-73, 1-74, 1-76, 2-1, 2-2, 2-4, 2-15, 2-17, 2-19, 2-20, 2-21, 2-23, 2-26, 2-27, 2-28, 2-33, 2-34, 2-35, 2-56, 2-57, 2-61, 3-1, 3-10, 3-23, 3-25, 3-27, 3-29, 3-31, 3-42, 3-43, 3-49, 3-52, 3-54, 3-56, 3-60, 3-70, 3-71, 3-73, 4-9, 4-14, 4-21, 4-23, 4-26, 4-28, 4-35, 4-36, 4-51, 4-56, 4-59, 4-60, 5-1, 5-2, 5-3, 5-15, 5-17, 5-19, 5-20, 5-21, 5-23, 5-24, 5-26, 5-27, 5-28, 5-33, 5-34, 5-56, 5-57, 5-60, 5-61, 6-1, 6-2, 6-3, 6-5, 6-13, 6-24, 6-26, 6-28, 6-29, 6-42, 7-1, 7-6, 7-9, 7-14, 7-20, 7-25, 7-28, 7-32, 7-37, 7-42, 7-45, 7-54, 7-56, 7-57, 7-58, 7-65, 7-67, 7-69, 7-71, 7-72, 7-75, 7-76, 7-77, 7-78, 7-84, 7-85, 7-91, 7-94, 7-95, 7-96, 7-100, 7-101, 7-104, 7-107, 7-110, 7-112, 7-113, 7-116, 7-118, 7-119, 7-122, 7-124, 7-125, 7-143, 7-146, 7-147, 7-149, 7-151, 7-152, 7-153, 7-154, 7-162, 7-169, 7-174, 7-180, 7-181, 7-184, 8-1, 8-6, 8-11, 8-12, 8-14, 8-16, 8-21, 8-22, 8-24, 8-25, 8-27, 8-28, 8-33, 8-42, 8-48, 9-1, 9-6, 9-9, 9-20, 9-25, 9-28, 9-37, 9-42, 9-45, 9-54, 9-56, 9-57, 9-58, 9-59, 9-60, 9-65, 9-67, 9-74, 9-80, 9-83, 9-84, 9-85, 9-89, 9-90, 9-92, 9-93, 9-95, 9-96, 9-99, 9-102, 9-113, 9-117, 9-119, 9-121, 9-122, 9-128, 9-138, 9-143, 9-148, 9-149, 10-1, 10-8, 10-13, 10-14, 10-17, 10-18, 10-19, 10-20, 10-21, 10-23, 10-24, 10-26, 10-30, 10-32, 10-35, 10-36, 10-43, 10-44, 10-45, 10-46, 10-47, 10-49, 10-56, 10-57, 10-58, 10-62, 10-64, 10-67, 10-68, 10-70, 10-73, 10-74, 10-76, 11-1, 11-8, 11-9, 11-10, 11-11, 11-14, 11-15, 11-17, 11-18, 11-21, 11-27, 11-29, 11-33, 12-1, 12-8, 12-13, 12-14, 12-17, 12-18, 12-19, 12-20, 12-21, 12-25, 12-28, 12-29, 12-30, 12-38, 12-44, 12-47, 12-50, 12-51 and 12-52.

More preferred specific compounds from this list are Compounds No.: 1-1, 1-2, 1-17, 1-23, 1-24, 1-25, 1-26, 1-28, 1-32, 1-33, 1-34, 1-36, 1-46, 1-47, 1-48, 1-76, 2-1, 2-2, 2-19, 2-20, 2-21, 2-23, 2-28, 2-56, 2-57, 2-61, 3-1, 3-23, 3-25, 3-29, 3-42, 3-43, 3-70, 3-71, 3-73, 4-21, 4-23, 4-26, 4-28, 4-35, 4-36, 4-56, 4-59, 4-60, 5-1, 5-17, 5-19, 5-20, 5-23, 5-26, 5-28, 5-33, 5-34, 5-56, 5-61, 6-1, 6-2, 6-3, 6-13, 6-26, 6-28, 6-29, 6-42, 7-1, 7-6, 7-9, 7-14, 7-20, 7-25, 7-28, 7-37, 7-42, 7-56, 7-57, 7-58, 7-69, 7-71, 7-76, 7-77, 7-78, 7-84, 7-85, 7-91, 7-95, 7-96, 7-101, 7-104, 7-107, 7-110, 7-113, 7-116, 7-118, 7-119, 7-122, 7-143, 7-146, 7-147, 7-153, 7-162, 7-169, 7-174, 8-1, 8-6, 8-16, 8-21, 8-33, 8-42, 9-1, 9-6, 9-67, 9-74, 9-102, 9-113, 9-128, 10-1, 10-8, 10-14, 10-17, 10-18, 10-20, 10-26, 10-30, 10-32, 10-43, 10-44, 10-45, 10-49, 10-56, 10-57, 10-67, 10-68-, 10-73, 10-74, 11-1, 11-11, 11-17, 11-21, 11-27, 11-29, 11-33, 12-1, 12-21, 12-29, 12-30, 12-44, 12-50 and 12-51.

Still more preferred specific compounds are Compounds No.: 1-1, 1-2, 1-17, 1-24, 1-25, 1-34, 1-36, 1-46, 1-47, 1-76, 2-1, 2-19, 2-20, 2-28, 2-56, 2-61, 3-1, 3-23, 3-25, 3-29, 3-42, 3-71, 3-73, 4-21, 4-23, 4-35, 4-56, 4-59, 4-60, 5-1, 5-17, 5-20, 5-28, 5-33, 5-56, 5-61, 6-1, 6-2, 6-29, 6-42, 7-1, 7-6, 7-14, 7-20, 7-25, 7-37, 7-42, 7-56, 7-78, 7-85, 7-91, 7-95, 7-104, 7-110, 7-142, 7-143, 7-162, 7-169, 7-174, 8-1, 8-16, 8-42, 9-1, 9-6, 9-67, 9-113, 9-128, 10-1, 10-8, 10-18, 10-26, 10-30, 10-43, 10-49, 10-56, 10-73, 11-1, 11-11, 11-17, 12-1, 12-21, 12-29, 12-44 and 12-51.

Of these, the most preferred are Compounds No.:

1-1. N-Methyl-N-{3-{N-[2-bis(4-fluorophenyl)methoxyethyl]-N-methylamino}propyl}-2-(3-pyridyl)-thiazolidine-4-carboxamide;

1-24. N-Methyl-N-{3-[N-(2-diphenylmethoxyethyl)-N-methylamino]propyl}-2-(3-pyridyl)thiazolidine-4-carboxamide;

1-25. N-Methyl-N-{2-[N-(2-diphenylmethoxyethyl)-N-methylamino]ethyl}-2-(3-pyridyl)thiazolidine-4-carboxamide;

3-1. 1-[2-Bis(4-fluorophenyl)methoxyethyl]-4-[2-(3-pyridyl)thiazolidin-4-ylcarbonyl]piperazine;

3-23. 1-(2-Diphenylmethoxyethyl)-4-[2-(3-pyridyl)thiazolidin-4-ylcarbonyl]homopiperazine;

3-25. 1-{2-[Bis(4-fluorophenyl)methoxy]ethyl}-4-[2-(3-pyridyl)thiazolidin-4-ylcarbonyl]homopiperazine;

3-29. 1-{2-[α-(4-Methylphenyl)benzyloxy]ethyl}-4-[2-(3-pyridyl)thiazolidin-4-ylcarbonyl]piperazine;

3-42. 1-[2-(α-Cyclohexylbenzyloxy)ethyl]-4-[2-(3-pyridyl)thiazolidin-4-ylcarbonyl]piperazine;

3-71. 1-(2-Diphenylmethoxyethyl)-4-[2-(3-pyridyl)thiazolidin-4-ylcarbonyl]piperazine;

3-73. 1-{2-[α-(4-Chlorophenyl)benzyloxy]ethyl}-4-[2-(3-pyridyl)thiazolidin-4-ylcarbonyl]piperazine;

4-21. 1-[2-(1,1-Diphenylethoxy)ethyl]-4-[2-(3-pyridyl)thiazolidin-4-ylcarbonyl]homopiperazine;

4-23. 1-{2-[1,1-bis(4-Fluorophenyl)ethoxy]ethyl}-4-[2-(3-pyridyl)thiazolidin-4-ylcarbonyl]homopiperazine;

4-56. 1-[2-(1,1-Diphenylethoxy)ethyl]-4-[2-(3-pyridyl)thiazolidin-4-ylcarbonyl]piperazine;

4-59. 1-{2-[1-(4-Chlorophenyl)-1-phenylethoxy]ethyl}-4-[2-(3-pyridyl)thiazolidin-4-ylcarbonyl]-piperazine;

4-60. 1-{2-[1,1-Bis(4-fluorophenyl)ethoxy]ethyl}-4-[2-(3-pyridyl)thiazolidin-4-ylcarbonyl]piperazine;

6-1. 1-{2-[4-(Diphenylmethylene)-1-piperidinyl]ethyl}-4-[2-(3-pyridyl)thiazolidin-4-ylcarbonyl]piperazine;

7-1. N-{2-(1,2,3,4,10,14b-Hexahydrodibenzo[c,f]pyrazino[1,2-a]azepin-2-yl)ethyl}-2-(3-pyridyl)thiazolidine-4-carboxamide;

7-6. N-{2-(1,2,3,4,10,14b-Hexahydrodibenzo[c,f]-pyrazino[1,2-a]azepin-2-yl)ethyl}-N-methyl-2-(3-pyridyl)-thiazolidine-4-carboxamide;

7-20. N-{2-(8-Fluoro-1,2,3,4,10,14b-hexahydrodibenzo[c,f]pyrazino[1,2-a]azepin-2-yl)ethyl}-2-(3-pyridyl)-thiazolidine-4-carboxamide;

7-37. N-{2-(8-Chloro-1,2,3,4,10,14b-hexahydrodibenzo[c,f]pyrazino[1,2-a]azepin-2-yl)ethyl}-2-(3-pyridyl)-thiazolidine-4-carboxamide;

7-78. N-{3-(1,2,3,4,10,14b-Hexahydrodibenzo[c,f]pyrazino[1,2-a]azepin-2-yl)propyl}-2-(3-pyridyl)-thiazolidine-4-carboxamide;

7-143. N-{4-(1,2,3,4,10,14b-Hexahydrodibenzo[c,f]pyrazino[1,2-a]azepin-2-yl)butyl}-2-(3-pyridyl)-thiazolidine-4-carboxamide;

7-162. 2-[2-(3-Pyridyl)thiazolidin-4-ylcarbonyl]-1,2,3,4,10,14b-hexahydrodibenzo[c,f]pyrazino[1,2-a]azepine;

8-1. N-{2-[1,2,3,4,10,14b-Hexahydropyrazino[2,1-a]pyrido[2,3-c][2]benzoazepin-2-yl]ethyl}-2-(3-pyridyl) thiazolidine-4-carboxamide;

9-1. N-{2-[1,2,3,4,10,14b-Hexahydropyrazino[1,2-a]pyrrolo[2,1-c][1,4]benzodiazepin-2-yl]ethyl}-2-(3-pyridyl)thiazolidine-4-carboxamide;

10-1. 1-{2-[1,2,3,4,10,14b-Hexahydrodibenzo[c,f]pyrazino[1,2-a]azepin-2-yl]ethyl}-4-[2-(3-pyridyl)-thiazolidin-4-ylcarbonyl]piperazine;

and salts thereof.

The compounds of the present invention may be prepared by a variety of processes well known in the art for the preparation of this type of compound. For example, in general terms, they may be prepared by reacting a carboxylic acid compound of formula (XII):

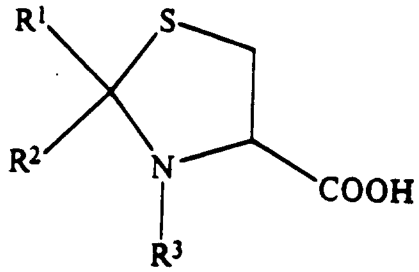

(XII)

(in which $R^1$, $R^2$ and $R^3$ are as defined above), or a reactive derivative thereof, with an amine compound of formula (XIII):

$H\text{-}R^4$ (XIII)

(in which $R^4$ is as defined above).

The reaction between the carboxylic acid of formula (XII) itself and the amine derivative of formula (XIII) may be carried out in the presence or absence of a base; it is also preferably carried out in the presence of an inert solvent and preferably in the presence of a condensing agent.

There is no particular restriction on the nature of the condensing agents employed, and any such agent commonly used in reactions of this type may equally be employed here, provided that it can form an amide bond from the carboxylic acid and the amine. Preferred examples include: dicyclohexylcarbodiimide (DCC), diethyl cyanophosphonate (DEPC), carbonyldiimidazole, diphenylphosphorylazide (DPPA), dicyclohexylcarbodiimide plus 1-hydroxybenzotriazole and diethylazodicarboxylate plus triphenylphosphine, more preferably dicyclohexylcarbodiimide plus 1-hydroxybenzotriazole or diethyl cyanophosphonate.

There is equally no particular restriction on the nature of the base, when employed, provided that it has no adverse effect on other parts of the molecule. Examples of suitable bases include organic amines, such as trimethylamine, triethylamine, pyridine, dimethylaniline, N-methylmorpholine and 4-dimethylaminopyridine, most preferably triethylamine or N-methylmorpholine.

The reaction is normally and preferably carried out in the presence of an inert solvent, the nature of which is not critical, provided that it has no adverse effect upon the reaction and that it can dissolve the reagents, at least to some extent. Examples of suitable solvents include: aromatic hydrocarbons, such as benzene, toluene and xylene; halogenated hydrocarbons, especially halogenated aliphatic hydrocarbons, such as methylene chloride, dichloroethane and chloroform; esters, such as ethyl acetate and propyl acetate; ethers, such as diethyl ether, tetrahydrofuran and dioxane; amides, such as dimethylformamide, dimethylacetamide and hexamethylphosphoric triamide; and nitriles, such as acetonitrile. Of these, we prefer the ethers (particularly tetrahydrofuran), halogenated hydrocarbons (particularly methylene chloride), amides (particularly dimethylformamide) and esters (particularly ethyl acetate).

The reaction will take place over a wide range of temperatures, and the precise reaction temperature chosen is not critical to the invention. In general, we find it convenient to carry out the reaction at a temperature in the range of from -10 °C to 50 °C (more preferably from 0 °C to 30 °C). The time required for the reaction may likewise vary widely, depending on many factors, notably the reaction temperature and the nature of the reagents. However, in most cases, a period of from 30 minutes to 24 hours will normally suffice.

Instead of using the carboxylic acid of formula (XII) itself, a reactive derivative can be employed in this reaction, as is well known in the art for reactions of this type. Examples of reactive derivatives of carboxylic acids include: acid halides, such as the acid chlorides and acid bromides; acid amides; active esters with 1-hydroxybenzotriazole, N-hydroxysuccinimide, etc.; acid anhydrides of the carboxylic acid of formula (XII); or mixed acid anhydrides with a monoalkyl carbonate in which the alkyl group has from 1 to 4 carbon atoms, such as monomethyl carbonate, monoethyl carbonate or monoisobutyl carbonate, or with a monoaryl carbonate, such as monophenyl carbonate or monotolyl (especially mono-p-tolyl) carbonate; of these, we prefer the mixed acid anhydrides with alkyl carbonates.

Reactive derivatives of carboxylic acids, such as the acid halides and the acid anhydrides, can be obtained by conventional methods, for example, by reacting a carboxylic acid of formula (XII) with a halogenating agent (for example thionyl chloride or thionyl bromide), with an acid chloride or acid bromide of a desired carboxylic acid, or with methyl chlorocarbonate, ethyl chlorocarbonate, isobutyl chlorocarbonate, phenyl chlorocarbonate or tolyl chlorocarbonate. This reaction normally and preferably takes place in an inert solvent (for example methylene chloride, benzene or tetrahydrofuran) at a suitable temperature, for example from 20°C to 100°C, for the required time, which is generally from 1 to 20 hours. If necessary, the reaction may be carried out in the presence of a base (for example pyridine, triethylamine and dimethylaniline).

Reactive derivatives, such as the acid azides and active esters, can be prepared by reacting a carboxylic acid of formula (XII) with an azidating or esterifying agent, for example hydrogen azide, 1-hydroxybenzotriazole or N-hydroxysuccinimide); the reaction conditions employed are preferably the same as those employed for the reaction between the carboxylic acid of formula (XII) and the amine of formula (XIII).

The reaction between a reactive derivative of the carboxylic acid of formula (XII) and the amine of formula (XIII) is normally and preferably carried out in an inert solvent, the nature of which is not critical, provided that it has no adverse effect upon the reaction and that it can dissolve the reagents, at least to some extent. Examples of suitable solvents include: halogenated hydrocarbons, especially halogenated aliphatic hydrocarbons, such as methylene chloride, dichloroethane and chloroform; ethers, such as diethyl ether, tetrahydrofuran and dioxane; esters, such as ethyl acetate; and aromatic hydrocarbons, such as benzene, toluene and xylene. Of these, we prefer the aromatic hydrocarbons and the ethers, such as tetrahydrofuran.

Alternatively, a large excess of the compound of formula (XIII) may be used and will also serve as the solvent.

The reaction will take place over a wide range of temperatures, and the precise reaction temperature chosen is not critical to the invention. In general, we find it convenient to carry out the reaction at a temperature in the range of from -10°C to 50°C (more preferably from 0°C to 25°C). The time required for the reaction may likewise vary widely, depending on many factors, notably the reaction temperature and the nature of the reagents. However, in most cases, a period of from 5 minutes to 20 hours (more preferably from 30 minutes to 10 hours) will normally suffice.

The desired product of this reaction can be recovered from the reaction mixture by conventional methods. For example, one suitable recovery procedure comprises: suitably neutralizing the reaction mixture; and then distilling the solvent from the reaction mixture. If necessary, after the solvent is distilled off from the reaction mixture, the residue may be poured into water and the resulting mixture extracted with a water-immiscible organic solvent. The solvent is then removed from the extract by evaporation, optionally under reduced pressure, to obtain the desired compound. If desired, the resulting compound can be further purified by conventional techniques, such as recrystallization, reprecipitation or the various chromatography techniques, notably column chromatography.

Those compounds of formula (I) in which $R^3$ represents an alkyl group can be prepared by acylating the corresponding compound of formula (I) in which $R^3$ represents a hydrogen atom to give a compound in which $R^3$ is replaced by an acyl group and then reducing this acyl group to the corresponding alkyl group. The first step of acylation may be carried out by formylating the starting material by a known method [for example, as described in J. Org. Chem., 27, 4058 (1962), the disclosure of which is incorporated herein by reference] or by reacting the starting material with a fatty acid halide having from 2 to 4 carbon atoms (for example acetyl chloride, propionyl chloride or butyryl chloride) in an inert solvent (for example, methylene chloride) at a suitable temperature, for example around room temperature, for a suitable period, for example from 30 minutes to 2 hours, in the presence of a base (for example triethylamine or pyridine) to provide an acyl compound. This acyl compound may then be reacted with a reducing agent (for example an aluminium hydride, such as lithium aluminium hydride) in an inert solvent (for example an ether, such as diethyl ether or tetrahydofuran) at a suitable temperature, for example from -10°C to 80°C, for a suitable period, for

example from 30 minutes to 5 hours.

The starting compound of formula (XII) is known or may easily be produced by known methods (for example as described in French Patent No. 2267089 or Japanese Unexamined Patent Publication No. 2-179) or by analogous methods. A compound of formula (XII) in which $R^3$ represents an alkyl group may easily be prepared from the corresponding compound in which $R^3$ represents a hydrogen atom by a reaction analogous to that described above for converting a compound of formula (I) in which $R^3$ represents a hydrogen atom to a corresponding compound in which $R^3$ represents an alkyl group.

The starting compound of formula (XIII) is known or may easily be produced by known methods [for example as described in Chem. Pharm. Bull., 37, 100 (1989) or J. Med. Chem., 32, 583 (1989)], for example as shown below in Reaction Schemes A and B, or by analogous methods.

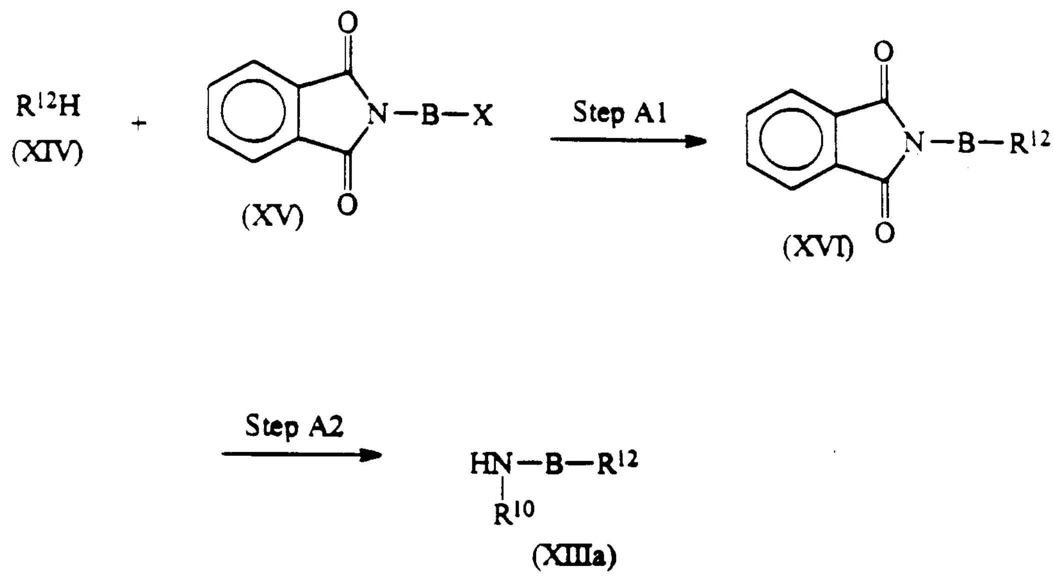


(XIV) (XV) (XVI) (XIIIa)

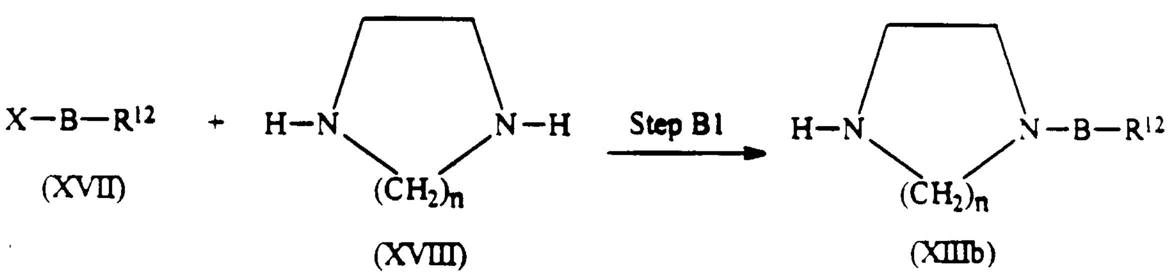


(XVII) (XVIII) (XIIIb)

In the above formulae, $R^{10}$, B and n are as defined above; X represents a halogen atom (preferably a chlorine, bromine or iodine atom) and $R^{12}$ represents a group of formula (XIX), (XX) or (XXI):

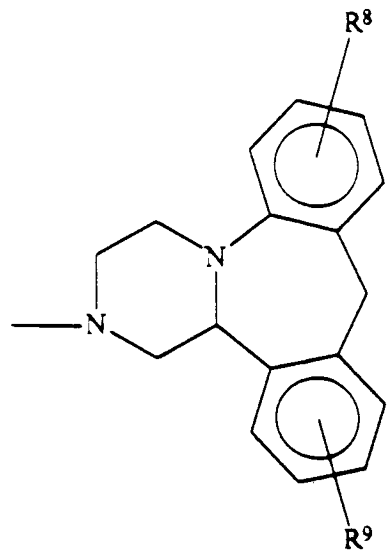

(XIX)

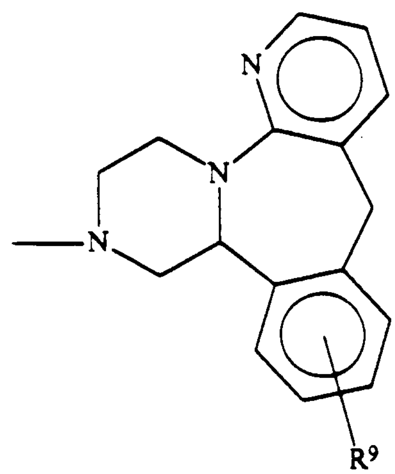

(XX)

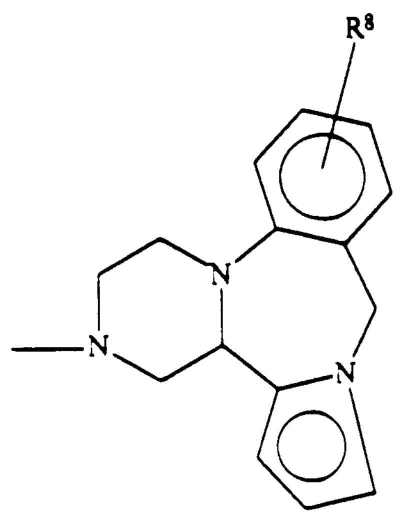

(XXI)

In the above formulae, $R^8$ and $R^9$ are as defined above.

In Reaction Scheme A, a compound of formula (XIIIa), which corresponds to a compound of formula (XIII) in which $A^2$ represents a group of the formula $N(R^{10})$-B (in which $R^{10}$ and B are as defined above) is prepared in two steps.

In Step A1, a compound of formula (XVI) is prepared by reacting a compound of formula (XIV) with a compound of formula (XV). This reaction is normally and preferably carried out in an inert solvent and in the presence of a base.

There is no particular limitation on the nature of the base which may be employed, and any base commonly used in reactions of this type may equally be used here. Preferred examples include: alkali metal carbonates, such as sodium carbonate and potassium carbonate; and alkali metal hydrogencarbonates, such as sodium hydrogencarbonate and potassium hydrogencarbonate. Of these, we particularly prefer sodium carbonate or potassium carbonate.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ketones, such as acetone, methyl ethyl ketone and methyl isobutyl ketone; and amides, such as dimethylformamide, dimethylacetamide and hexamethylphosphoric triamide. Of these, we prefer the ketones, particularly methyl isobutyl ketone.

The reaction can take place over a wide range of temperatures, and the precise reaction temperature is not critical to the invention. In general, we find it convenient to carry out the reaction at a temperature of from -10 °C to 120 °C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours will usually suffice.

This reaction may also be carried out, if necessary, in the presence of a catalytic amount of an alkali metal iodide, such as sodium iodide or potassium iodide.

In Step A2 of this Reaction Scheme, a compound of formula (XIIIa) is prepared by reacting a compound of formula (XVI), which may have been prepared as described in Step A1, with an amine, followed by alkylation of the amino group, if desired. Examples of amines which may be employed in this reaction include hydrazine and butylamine.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include alcohols, such as methanol, ethanol and propyl alcohol.

The reaction can take place over a wide range of temperatures, and the precise reaction temperature is not critical to the invention. In general, we find it convenient to carry out the reaction at a temperature of from -10 °C to 90 °C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours will usually suffice.

The optional alkylation reaction may be carried out by reacting a compound of formula (XIIIa) in which $R^{10}$ represents a hydrogen atom with an acylating agent and then a reducing agent, in the same manner as in the alkylation of $R^3$ in the preparation of the compounds of formula (I).

In Reaction Scheme B, a compound of formula (XIIIb), which corresponds to the compound of formula (XIII) in which A represents a piperazine or homopiperazine group, is prepared.

In this reaction, a compound of formula (XVII) is allowed to react with a compound of formula (XVIII), normally and preferably in an inert solvent and in the presence of a base, to give a compound of formula (XIIIb).

There is no particular limitation on the nature of the base which may be employed, and any base commonly used in reactions of this type may equally be used here. Preferred examples include organic amines such as triethylamine or pyridine; alternatively, an excess of the compound of formula (XVIII) can be used and will serve also as the base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include aromatic hydrocarbons, such as benzene, toluene and xylene.

The reaction can take place over a wide range of temperatures, and the precise reaction temperature is not critical to the invention. In general, we find it convenient to carry out the reaction at a temperature of from 20 °C to 130 °C. The time required for the reaction may also vary widely, depending on many factors, notably the reaction temperature and the nature of the reagent's and solvent employed. However, provided that the reaction is effected under the preferred conditions outlined above, a period of from 30 minutes to 24 hours will usually suffice.

The desired product of each step can be recovered from the reaction mixture by conventional methods. For example, one suitable recovery procedure comprises: suitably neutralizing the reaction mixture; and then distilling the solvent from the reaction mixture. If necessary, after the solvent has been distilled from the reaction mixture, the residue may be poured into water and the resulting mixture extracted with a water-immiscible organic solvent, followed by evaporation of the solvent from the extract to obtain the desired compound. If desired, the resulting compound can be further purified by conventional techniques, such as recrystallization, reprecipitation or the various chromatography techniques, notably column chromatography.

The pyridylthiazolinecarboxylic acid amide derivatives of the present invention have excellent anti-allergic and anti-asthmatic activities. In addition, since they are antagonists to the manifestation of inflammation caused by PAF and can thus inhibit the accumulation of eosinophiles, they are effective for the treatment not only of early allergic reactions but also of late allergic reactions. They are thus useful as therapeutic agents for the treatment or prophylaxis of allergic diseases and asthma.

The compounds of the present invention may therefore be used in the treatment and prophylaxis of disorders such as those referred to above, and, for this purpose, may be formulated as conventional pharmaceutical preparations, as is well known in the art. Thus, the compounds may be administered orally, e.g. in the form of tablets, capsules, granules, powders, syrups, or other such well known forms, parenterally, e.g. by injections, suppositories, or by other means, for example, as patches, inhalation or ophthalmic solutions.

These pharmaceutical preparations can be prepared by conventional means and may contain known adjuvants of a type commonly used in this field, for example vehicles, binders, disintegrators, lubricants, stabilisers, corrigents, etc. depending upon the intended use and form of the preparation. The dose will depend upon the condition, age, and body weight of the patient as well as upon the nature and severity of the disorder to be treated, but, in the case of oral administration to an adult human patient, we would normally suggest a total daily dose of from 10 mg to 1000 mg, more preferably from 10 mg to 500 mg, which may be administered in a single dose or in divided doses, e.g. from one to three times a day.

## BIOLOGICAL ACTIVITY

The biological activity of the compounds of the present invention is shown in the following Experiments. In these Experiments, the compounds of the invention are identified by reference to the number of one of the subsequent Examples which illustrates their preparation.

## EXPERIMENT 1

### Inhibitory effect on passive cutaneous anaphylaxis (PCA) in rats

According to Mota's method [I. Mota, Immunology, 7, 681 - 699 (1964)], antiserum (256 times the PCA titer) of rat against egg albumin was prepared and diluted four times with physiological saline. Male SD rats (5 weeks old) were used as the test animals in groups, each containing 4 animals. The rats were sensitized by intradermal injection of 0.05 ml of the diluted antiserum solution in the dorsal position. 48 hours after this injection, a suspension of the test compound in an aqueous 0.5% w/v tragacanth solution was orally administered to the rats, which had been fasted for one day, and 60 minutes later they were injected in the caudal vein with 5 ml/kg body weight of physiological saline containing 0.4% w/v egg albumin and 1.0% w/v Evans Blue. 30 minutes after this last injection, the rats were sacrificed with carbon dioxide and the Evans Blue exuded in the dorsal intradermal portion was determined according to Harada's method (Harada et al., J. Pharm. Pharmac., 23, 218 - 219 (1971)].

The results achieved from the test groups which were treated with a test compound were evaluated to determine the inhibitory rate by comparison with the average amount of exuded dye in a control group, which was not given the test compound.

The inhibitory rate was calculated by the following equation.

$$\text{Inhibitory rate (\%)} = (1-B/A) \times 100$$

A: amount of exuded dye in the control group

B: amount of exuded dye in the test group.

The results are shown in Table 13.

Table 13

| Compound of Example No. | Salt | Dose (p.o., mg/kg) | Inhibitory rate (%) |
|---|---|---|---|
| 1 | hydrochloride | 6.4 | 33 |
| 5 | hydrochloride | 6.4 | 42 |
| 6 | hydrochloride | 6.4 | 45 |
| 16 | hydrochloride | 6.4 | 62 |
| 17 | hydrochloride | 6.4 | 73 |
| 25 | hydrochloride | 6.4 | 55 |

EXPERIMENT 2

Inhibitory effect in vitro against PAF-induced blood platelet aggregation

Blood samples were obtained by cardiac puncture from a rabbit and one part by volume of each sample was immediately mixed with 0.1 part of a 3.8% w/v aqueous solution of sodium citrate. A platelet rich plasma (PRP) fraction was prepared by centrifuging the samples at 150 x G for 15 minutes at room temperature, and a platelet poor plasma (PPP) fraction was then prepared by further centrifugation at 1,000 x G for 15 minutes. The platelet count in the PRP was adjusted to 6 x $10^5$ per $\mu\ell$ by the addition of an appropriate amount of the PPP fraction. According to the method reported by Born et al. [G.V.R. Born et al.: J. Physiol. 62, 67 - 68 (1962)], blood platelet aggregation was determined turbidimetrically in a 6-channel aggregometer (Hemetracer, NKB, Tokyo, Japan). Aliquots of the PRP (272 $\mu\ell$) were preincubated with 3 $\mu\ell$ of a solution of the test compound in dimethyl sulphoxide for 1 minute, and then stimulated with $\ell$-$C_{16:0}$ PAF (at a final concentration of $10^{-8}$ ~ 3 x $10^{-8}$ M) at 37°C with stirring (100 rpm). Changes in light transmission were monitored for 5 minutes. Vehicle (dimethyl sulphoxide) controls were tested simultaneously, and the inhibitory effects of the test compounds were assessed on the maximal aggregation. The $IC_{50}$ values were calculated by the method of least squares.

Table 14 below shows the results.

Table 14

| Compound of Example No. | Salt | Platelet aggregation inhibition, $IC_{50}$ (g/ml) |
|---|---|---|
| 1 | hydrochloride | 6.3 x $10^{-8}$ |
| 4 | hydrochloride | 8.5 x $10^{-8}$ |
| 6 | hydrochloride | 5.5 x $10^{-8}$ |
| 7 | hydrochloride | 9.5 x $10^{-8}$ |
| 9 | hydrochloride | 2.8 x $10^{-7}$ |
| 11 | hydrochloride | 7.2 x $10^{-8}$ |
| 12 | hydrochloride | 1.9 x $10^{-7}$ |
| 13 | hydrochloride | 2.2 x $10^{-7}$ |
| 14 | oxalate | 1.4 x $10^{-7}$ |

EXPERIMENT 3

Inhibitory effect on PAF-receptor binding

Blood samples were drawn from the heart of a rabbit. 1 part by volume of each sample was mixed immediately with 1/9 part of a 0.077 M solution of disodium ethylenediaminetetraacetate. After a similar procedure to that described in Experiment 2, a precipitated blood platelet sample was obtained. This blood platelet sample was washed, and, after repeated freesing and thawing to rupture the cells, it was placed on top of two layers consisting of 0.25 M and 1.5 M sucrose solutions. By centrifugation at 63,500 x G, for 2

hours at 4°C, the fraction obtained from the interface between the 0.25 M and 1.5 M sucrose solutions was collected and is regarded as a PAF-receptor membrane fraction. A receptor binding experiment was then conducted according to a method very similar to that reported by Hwang et al. [San-Bao Hwang et al.: J. Biol. Chem. 260, 15639 - 15645 (1985)]. The specific binding of $^3$H-PAF was measured using a Wattman GF/C filter. A test compound was dissolved in dimethyl sulphoxide and diluted 100 fold with a buffer solution containing 0.5% bovine serum albumin. Nine parts by volume of the solution, for a receptor binding experiment, was mixed with one part of the test compound solution prepared above. The percent inhibition of the specific binding was plotted against the log of the concentration of the test compound, and the 50% inhibitory concentration ($IC_{50}$) was calculated from the linear line connecting all the plotted points.

The results are shown in Table 15.

Table 15

| Compound of Example No. | Salt | Receptor binding inhibition, $IC_{50}$ (g/ml) |
|---|---|---|
| 4 | hydrochloride | $2.7 \times 10^{-8}$ |
| 6 | hydrochloride | $2.1 \times 10^{-8}$ |
| 7 | free base | $2.5 \times 10^{-8}$ |
| 9 | hydrochloride | $2.1 \times 10^{-7}$ |
| 11 | hydrochloride | $2.2 \times 10^{-7}$ |
| 12 | hydrochloride | $2.1 \times 10^{-7}$ |
| 13 | hydrochloride | $1.4 \times 10^{-7}$ |
| 14 | oxalate | $0.1 \times 10^{-7}$ |

EXPERIMENT 4

Inhibitory effect on intracutaneous reaction in rats by PAF

Male SD rats (5 weeks old) were used as the test animals in groups, each containing 4 animals. Each rat was administered orally with a suspension of the test compound in a 0.5% w/v aqueous tragacanth solution. Sixty minutes after the administration, aqueous physiological saline containing 1.0% w/v Evans blue was injected into the tail vein in an amount of 5 ml/kg of the body weight. Immediately after the injection, 0.05 ml of a solution containing 2 µg/ml of PAF was intradermally injected into the back of the rat to induce a cutanous reaction. After 30 minutes, the rats were sacrificed using carbon dioxide, and the quantity of Evans blue which had leaked into the skin of the back was determined by the method of Harada et al. [J. Pharm. Pharmac., 23, 218 - 219 (1971)]. The inhibition rate of the compound was calculated by comparing the average amount of colourant which had leaked in the group treated with the test compound with that of control group to which no test compound was administered.

The inhibition rate was calculated according to the following equation:

$$\text{Inhibition rate (\%)} = (1 - B/A) \times 100$$

where:

A: Amount of colourant leaked in the control group

B: Amount of colourant leaked in the group to which the test compound was administered.

The results are shown in Table 16.

Table 16

| Compound of Example No. | Salt | Dose (p.o., mg/kg) | Inhibitory rate (%) |
|---|---|---|---|
| 1 | hydrochloride | 6.4 | 57 |
| 4 | hydrochloride | 6.4 | 64 |
| 7 | hydrochloride | 6.4 | 70 |
| 14 | hydrochloride | 6.4 | 47 |
| 16 | hydrochloride | 6.4 | 62 |

The invention is further illustrated by the following non-limiting Examples. Preparation of certain of the starting materials employed in these Examples is illustrated by the subsequent Preparations.

EXAMPLE 1

(4R)-N-Methyl-N-{2-[N-(2-diphenylmethoxyethyl)-N-methylamino]ethyl}-2-(3-pyridyl)thiazolidine-4-carboxamide and its hydrochloride

A mixture of 500 mg (2.4 mmole) of (4R)-2-(3-pyridyl)thiazolidine-4-carboxylic acid, 710 mg (2.4 mmole) of N-[2-(diphenylmethoxy)ethyl]-N-methyl-N'-methylethylenediamine (prepared as described in Preparation 2), 490 mg (2.4 mmole) of dicyclohexylcarbodiimide, 320 mg (2.4 mmole) of 1-hydroxybenzotriazole and 10 ml of dimethylformamide was stirred overnight at room temperature. At the end of this time, the reaction mixture was diluted with ethyl acetate, and the insolubles were filtered off. The filtrate was washed with water, and then the solvent was removed by distillation under reduced pressure. The residue was subjected to column chromatography through silica gel, using a 20 : 1 by volume mixture of chloroform and methanol as the eluent, to obtain 540 mg (yield 60%) of the title compound as an oil.
Infrared Absorption Spectrum ($CHCl_3$) $\nu_{max}$ $cm^{-1}$:
3280, 2980, 2830, 1640, 1400.
Mass spectrum, m/z (%):
490 ($M^+$, 5), 254 (34), 167 (100).

The oil thus obtained was dissolved in ethyl acetate, and a 4 N solution of hydrogen chloride in ethyl acetate was added thereto. The crystals which precipitated were collected by filtration, to obtain the hydrochloride of the title compound, melting at 64 - 67 °C, in a quantitative yield.

EXAMPLE 2

(4R)-N-Methyl-N-{3-[N-(2-diphenylmethoxyethyl)-N-methylamino]propyl}-2-(3-pyridyl)thiazolidine-4-carboxamide and its hydrochloride

Following a procedure similar to that described in Example 1, but using (4R)-2-(3-pyridyl)thiazolidine-4-carboxylic acid and N-[2-(diphenylmethoxy)ethyl]-N-methyl-N'-methylpropanediamine (prepared as described in Preparation 1), the title compound was obtained in a yield of 42%.
Infrared Absorption Spectrum ($CHCl_3$) $\nu_{max}$ $cm^{-1}$:
3280, 2930, 1640, 1400.
Mass spectrum, m/z (%):
503 ($M^+$, 4), 307 (63), 167 (100).

The hydrochloride of the title compound, melting at 80 - 83 °C, was obtained in a quantitative yield by following a procedure similar to that described in the second step of Example 1.

EXAMPLE 3

(4R)-N-Methyl-N-{3-{N-[2-bis(4-fluorophenyl)methoxyethyl]-N-methylamino}propyl}-2-(3-pyridyl)thiazolidine-4-carboxamide

Following a procedure similar to that described in Example 1, but using (4R)-2-(3-pyridyl)thiazolidine-4-carboxylic acid and N-{2-[bis(4-fluorophenyl)methoxy]-ethyl}-N-methyl-N'-methylpropanediarnine (prepared as described in Preparation 3), the title compound was obtained in a yield of 52%.

Infrared Absorption Spectrum ($CHCl_3$) $\nu_{max}$ $cm^{-1}$:
3180, 2930, 1640, 1505, 1400.
Mass spectrum, m/z (%):
540 ($M^+$, 6), 307 (50), 203 (100).

EXAMPLE 4

(4R)-1-{2-[α-(4-Chlorophenyl)benzyloxylethyl}-4-[2-(3-pyridyl)thiazolidin-4-ylcarbonyl]piperazine and its hydrochloride

A mixture of 500 mg (2.4 mmole) of (4R)-2-(3-pyridyl)thiazolidine-4-carboxylic acid, 800 mg (2.4 mmole) of 1-{2-[α-(4-chlorophenyl)benzyloxy]ethyl}piperazine (prepared as described in Preparation 4), 490 mg (2.4 mmole) of dicyclohexylcarbodiimide, 320 mg (2.4 mmole) of 1-hydroxybenzotriazole and 10 ml of dimethylformamide was stirred overnight at room temperature. At the end of this time, the reaction mixture was diluted with ethyl acetate, and the insolubles were filtered off. The filtrate was washed with water, and then the solvent was removed by distillation under reduced pressure. The resulting residue was subjected to column chromatography through silica gel, using a 10 : 1 by volume mixture of ethyl acetate and ethanol as the eluent, to obtain 1.1 g (yield 85%) of the title compound as an oil.
Infrared Absorption Spectrum ($CHCl_3$) $\nu_{max}$ $cm^{-1}$:
3280, 2980, 1640, 1415.
Mass spectrum, m/z (%):
522 ($M^+$, 4), 201 (69), 168 (100).

The oil thus obtained was dissolved in ethyl acetate, and then a 4 N solution of hydrogen chloride in ethyl acetate was added to the resulting solution. The crystals which precipitated were collected by filtration to obtain the hydrochloride of the title compound, melting at 125 - 127 °C (with decomposition), in a quantitative yield.

EXAMPLE 5

(4R)-1-{2-[4-(Diphenylmethylene)-1-piperidinyl]ethyl}-4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-[2-(4-diphenylmethylene-1-piperidinyl)ethyl]piperazine (prepared as described in Preparation 5), the title compound was obtained in a yield of 46%.
Infrared Absorption Spectrum ($CHCl_3$) $\nu_{max}$ $cm^{-1}$:
3300, 2940, 2820, 1645, 1435, 1415.
Mass spectrum, m/z (%):
553 ($M^+$, 2), 384 (22), 262 (100).

This compound was treated with a 4 N solution of hydrogen chloride in ethyl acetate in the same manner as described in the second step of Example 1, to obtain the hydrochloride of the title compound, melting at 183 - 186 °C (with decomposition), in a quantitative yield.

EXAMPLE 6

(4R)-1-{2-[α-(4-Methylphenyl)benzyloxylethyl}4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-{2-[α-(4-methylphenyl)benzyloxy]ethyl}piperazine(prepared as described in Preparation 6), the title compound was obtained in a yield of 92%.
Infrared Absorption Spectrum ($CHCl_3$) $\nu_{max}$ $cm^{-1}$:
3280, 2980, 2640, 1415.
Mass spectrum, m/z (%):
502 ($M^+$, 6), 168 (55), 181 (100).

This compound was treated with a 4 N solution of hydrogen chloride in ethyl acetate in the same manner as described in the second step of Example 1, to obtain the hydrochloride of the title compound, melting at 135 - 137 °C (with decomposition).

EXAMPLE 7

(4R)-1-(2-Diphenylmethoxyethyl)-4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-[2-(diphenylmethoxy)ethyl]piperazine (prepared as described in Preparation 7), the title compound was obtain in a yield of 98%.

Infrared Absorption Spectrum ($CHCl_3$) $\nu_{max}$ $cm^{-1}$:

3300, 2900, 1640, 1420.

Mass spectrum, m/z (%):

488 ($M^+$, 23), 291 (46), 167 (100).

This compound was treated with a 4 N solution of hydrogen chloride in ethyl acetate in the same manner as described in the second step of Example 1, to obtain the hydrochloride of the title compound, melting at 108 - 120 °C.

EXAMPLE 8

(4R)-1-[2-(1,1-Diphenylethoxy)ethyl]-4-[2-(3-pyridyl)thiazolidin-4-ylcarbonyl]piperazine and its hydrochloride

A mixture of 500 mg (2.4 mmole) of (4R)-2-(3-pyridyl)thiazolidine-4-carboxylic acid, 740 mg (2.4 mmole) of 1-[2-(1,1-diphenylethoxy)ethyl]piperazine (prepared as described in Preparation 8), 490 mg (2.4 mmole) of dicyclohexylcarbodiimide, 320 mg (2.4 mmole) of 1-hydroxybenzotriazole and 10 ml of dimethylformamide was stirred overnight at room temperature. At the end of this time, the reaction mixture was diluted with ethyl acetate, and the insolubles were filtered off. The filtrate was washed with water, and the solvent was removed by distillation under reduced pressure. The resulting residue was subjected to column chromatography through silica gel, using a 20 : 1 by volume mixture of chloroform and methanol as the eluent, to obtain 1.1 g (yield 92%) of the title compound as an oil.

Infrared Absorption Spectrum ($CHCl_3$) $\nu_{max}$ $cm^{-1}$:

3280, 2975, 2920, 1640, 1440, 1415.

Mass spectrum, m/z (%):

502 ($M^+$, 40), 29 (80), 181 (100).

The oil thus obtained was treated with a 4 N solution of hydrogen chloride in ethyl acetate in the same manner as described in the second step of Example 1, to obtain the hydrochloride of the title compound, melting at 121 - 123 °C.

EXAMPLE 9

(4R)-1-{2-[1-(4-Chlorophenyl)-1-phenylethoxy]ethyl}-4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-{2-[1-(4-chlorophenyl)-1-phenylethoxy]ethyl}piperazine (prepared as described in Preparation 9), the title compound was obtained in a yield of 98%.

Infrared Absorption Spectrum ($CHCl_3$) $\nu_{max}$ $cm^{-1}$:

3280, 2930, 1640, 1440.

Mass spectrum, m/z (%):

503 ($M^+$, 4), 307 (63), 167 (100).

This compound was treated with a 4 N solution of hydrogen chloride in ethyl acetate in the same manner as described in the second step of Example 1, to obtain the hydrochloride of the title compound, melting at 113 - 115 °C.

EXAMPLE 10

(4R)-1-{2-[1,1-Bis(4-fluorophenyl)ethoxy]ethyl}4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-{2-[1,1-bis(4-fluorophenyl)ethoxy]ethyl}piperazine (prepared as described in Preparation 10), the title compound was obtained in a yield of 91%.
Infrared Absorption Spectrum ($CHCl_3$) $\nu_{max}$ $cm^{-1}$:
3280, 2970, 2920, 1640, 1505, 1415.
Mass spectrum, m/z (%):
538 ($M^+$, 36), 257 (54), 217 (100).

This compound was treated with a 4 N solution of hydrogen chloride in ethyl acetate in the same manner as described in the second step of Example 1, to obtain the hydrochloride of the title compound, melting at 116 - 118 °C (with decomposition).

EXAMPLE 11

(4R)-1-[2-(α-Cyclohexylbenzyloxy)ethyl]-4-[2-(3-pyridyl)thiazolidin-4-ylcarbonyl]piperazine and its hydrochloride

Following a procedure similar to that described in Example 1, but using (4R)-2-(3-pyridyl)thiazolidine-4-carboxylic acid and 1-[2-(α-cyclohexylbenzyloxy)ethyl]piperazine (prepared as described in Preparation 11), the title compound was obtained in a yield of 82%.
Infrared Absorption Spectrum ($CHCl_3$) $\nu_{max}$ $cm^{-1}$:
3380, 2820, 2840, 1640, 1415.
Mass spectrum, m/z (%):
494 ($M^+$, 59), 300 (64), 291 (100).

This compound was treated with a 4 N solution of hydrogen chloride in ethyl acetate in the same manner as described in the second step of Example 1, to obtain the hydrochloride of the title compound, melting at 140 - 142 °C (with decomposition).

EXAMPLE 12

(4R)-1-[2-Bis(4-fluorophenyl)methoxyethyl]-4-[2-(3-pyridyl)thiazolidin-4-ylcarbonyl]homopiperazine and its hydrochloride

Following a procedure similar to that described in Example 1, but using (4R)-2-(3-pyridyl)thiazolidine-4-carboxylic acid and 1-{2-[bis(4-fluorophenyl)methoxy]ethyl}homopiperazine (prepared as described in Preparation 12), the title compound was obtained in a yield of 70%.
Infrared Absorption Spectrum ($CHCl_3$) $\nu_{max}$ $cm^{-1}$:
3310, 2960, 1645, 1510, 1415.
Mass spectrum, m/z (%):
538 ($M^+$, 5), 305 (61), 203 (100).

This compound was treated with a 4 N solution of hydrogen chloride in ethyl acetate in the same manner as described in the second step of Example 1, to obtain the hydrochloride of the title compound, melting at 124 - 126 °C (with decomposition).

EXAMPLE 13

(4R)-1-(2-Diphenylmethoxyethyl)-4-[2-(3-pyridyl)thiazolidin-4-ylcarbonyl]homopiperazine and its hydrochloride

Following a procedure similar to that described in Example 1, but using (4R)-2-(3-pyridyl)thiazolidine-4-carboxylic acid and 1-[2-(diphenylmethoxy)ethyl]homopiperazine (prepared as described in Preparation 13), the title compound was obtained in a yield of 63%.
Infrared Absorption Spectrum ($CHCl_3$) $\nu_{max}$ $cm^{-1}$:
3300, 3000, 2930, 1640, 1410.
Mass spectrum, m/z (%):
503 ($M^+$, 4), 305 (50), 167 (100).

This compound was treated with a 4 N solution of hydrogen chloride in ethyl acetate in the same manner as described in the second step of Example 1, to obtain the hydrochloride of the title compound, melting at 56 - 58 °C (with decomposition).

EXAMPLE 14

(4R)-1-[2-Bis(4-fluorophenyl)methoxyethyl]-4-[2-(3-pyridyl)thiazolidin-4-ylcarbonyl]piperazine and its oxalate

Following a procedure similar to that described in Example 1, but using (4R)-2-(3-pyridyl)thiazolidine-4-carboxylic acid and 1-[2-bis(4-fluorophenyl)methoxyethyl]piperazine (prepared in a similar manner to that described in Preparation 8), the title compound was obtained in a yield of 96%.

Infrared Absorption Spectrum ($CHCl_3$) $\nu_{max}$ $cm^{-1}$:

3200, 3000, 2930, 1670, 1645, 1605, 1510.

Mass spectrum, m/z (%):

524 ($M^+$, 21), 291 (34), 203 (100).

This compound was dissolved in ethanol, and a solution of oxalic acid in ethanol was added to the resulting solution. The crystals which precipitated were collected by filtration, to obtain the oxalate of the title compound, melting at 60 - 62 °C.

EXAMPLE 15

(4R)-N-{2-(1,2,3,4,10,14b-Hexahydrodibenzo[c,f]pyrazino[1,2-a]azepin-2-yl)ethyl}-2-(3-pyridyl)thiazolidine-4-carboxamide and its hydrochloride

A mixture of 0.72 g (3.42 mmole) of (4R)-2-(3-pyridyl)thiazolidine-4-carboxylic acid, 1.0 g (3.40 mmole) of 2-(2-aminoethyl)-1,2,3,4,10,14b-hexahydrodibenzo[c,f]-pyrazino[1,2-a]azepine (prepared as described in Preparation 14), 0.7 g (3.42 mmole) of dicyclohexylcarbodiimide, 0.46 g (3.4 mmole) of 1-hydroxybenzotriazole and 15 ml of dimethylformamide was stirred overnight at room temperature. At the end of this time, the mixture was diluted with ethyl acetate, and insolubles were filtered off. The filtrate was washed with water and concentrated by evaporation under reduced pressure. The resulting residue was subjected to column chromatography through silica gel, using a 20 : 1 by volume mixture of chloroform and methanol as the eluent, to obtain 1.30 g (yield 79%) of the title compound as an oil.

Infrared Absorption Spectrum ($CHCl_3$) $\nu_{max}$ $cm^{-1}$:

3380, 3000, 2940, 2810, 1670, 1515, 1490.

Mass spectrum, m/z (%):

485 ($M^+$, 11), 263 (90), 73 (100).

The oil thus obtained was dissolved in ethyl acetate, and a 4 N solution of hydrogen chloride in ethyl acetate was added to the resulting solution. The crystals which precipitated were collected by filtration, to obtain the hydrochloride of the title compound, melting at 164 - 167 °C (with decomposition).

EXAMPLE 16

(4R)-N-{2-[(14bR)-1,2,3,4,10,14b-Hexahydrodibenzo[c,f]pyrazino[1,2-a]azepin-2-yl]ethyl}-2-(3-pyridyl)-thiazolidine-4-carboxamide and its hydrochloride

Following a procedure similar to that described in Example 15, but using (4R)-2-(3-pyridyl)thiazolidine-4-carboxylic acid and (14bR)-2-(2-aminoethyl)-1,2,3,4,10,14b-hexahydrodibenzo[c,f]pyrazino[1,2-a]azepine (prepared as described in Preparation 15), the title compound was obtained in a yield of 78%.

Infrared Absorption Spectrum ($CHCl_3$) $\nu_{max}$ $cm^{-1}$:

3380, 3010, 2950, 2820, 1675, 1525, 1495.

Mass spectrum, m/z (%):

485 ($M^+$, 27), 263 (100), 208 (88).

This compound was treated with a 4 N solution of hydrogen chloride in ethyl acetate in the same manner as described in the second step of Example 1, to obtain the hydrochloride of the title compound, melting at 175 - 178 °C (with decomposition).

EXAMPLE 17

(4R)-N-{2-[(14bS)-1,2,3,4,10,14b-Hexahydrodibenzo[c,f]pyrazino[1,2-a]azepin-2-yl]ethyl}-2-(3-pyridyl)-thiazolidine-4-carboxamide and its hydrochloride

Following a procedure similar to that described in Example 15, but using (4R)-2-(3-pyridyl)thiazolidine-4-carboxylic acid and (14bS)-2-(2-aminoethyl)-1,2,3,4,10,14b-hexahydrodibenzo[c,f]pyrazino[1,2-a]azepine (prepared as described in Preparation 16), the title compound was obtained in a yield of 83%.
Infrared Absorption Spectrum ($CHCl_3$) $\nu_{max}$ $cm^{-1}$:
3380, 3010, 2950, 2820, 1675, 1515, 1495.
Mass spectrum, m/z (%):
485 ($M^+$, 18), 263 (100), 208 (73).

This compound was treated with a 4 N solution of hydrogen chloride in ethyl acetate in the same manner as described in the second step of Example 1, to obtain the hydrochloride of the title compound, melting at 188 - 190 °C (with decomposition).

EXAMPLE 18

(4R)-N-{2-[(14bR)-1,2,3,4,10,14b-Hexahydrodibenzo[c,f]pyrazino[1,2-a]azepin-2-yl]ethyl}-N-methyl-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bR)-2-[2-(N-methylamino)ethyl]-1,2,3,4,10,14b-hexahydrodibenzo[c,f]pyrazino[1,2-a]-azepine (prepared as described in Preparation 17), the title compound was obtained in a yield of 73%.
Infrared Absorption Spectrum ($CHCl_3$) $\nu_{max}$ $cm^{-1}$:
3320, 3000, 2950, 2820, 1645, 1495.
Mass spectrum, m/z (%):
499 ($M^+$, 9), 263 (100), 208 (85).

This compound was treated with a 4 N solution of hydrogen chloride in ethyl acetate in the same manner as described in the second step of Example 1, to obtain the hydrochloride of the title compound, melting at 192 - 194 °C (with decomposition).

EXAMPLE 19

(4R)-N-{2-[(14bS)-1,2,3,4,10,14b-Hexahydrodibenzo[c,f]pyrazino[1,2-a]azepin-2-yl)ethyl]-N-methyl-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bS)-2-[2-(N-methylamino)ethyl]-1,2,3,4,10,14b-hexahydrodibenzo[c,f]pyrazino[1,2-a]-azepine (prepared as described in Preparation 18), the title compound was obtained in a yield of 84%.
Infrared Absorption Spectrum ($CHCl_3$) $\nu_{max}$ $cm^{-1}$:
3320, 3020, 2950, 2820, 1675, 1650, 1500.
Mass spectrum, m/z (%):
499 ($M^+$, 16), 263 (100), 208 (90).

This compound was treated with a 4 N solution of hydrogen chloride in ethyl acetate in the same manner as described in the second step of Example 1, to obtain the hydrochloride of the title compound, melting at 191 - 194 °C (with decomposition).

EXAMPLE 20

(4R)-N-{3-[(14bR -1,2,3,4,10,14b-Hexahydrodibenzo[c,f]pyrazino[1,2-a]azepin-2-yl]propyl}-2-(3-pyridyl)-thiazolidine-4-carboxamide and its hydrochloride

Following a procedure similar to that described in Example 15, but using (4R)-2-(3-pyridyl)thiazolidine-4-carboxylic acid and (14bR)-2-(3-aminopropyl)-1,2,3,4,10,14b-hexahydrodibenzo[c,f]pyrazino[1,2-a]azepine (prepared as described in Preparation 19), the title compound was obtained in a yield of 59%.
Infrared Absorption Spectrum ($CHCl_3$) $\nu_{max}$ $cm^{-1}$:
3350, 3010, 2950, 2820, 1680, 1520, 1495.

Mass spectrum, m/z (%):
499 ($M^+$, 4), 208 (84), 193 (71), 92 (74).

This compound was treated with a 4 N solution of hydrogen chloride in ethyl acetate in the same manner as described in the second step of Example 1, to obtain the hydrochloride of the title compound, melting at 143 - 145 °C (with decomposition).

EXAMPLE 21

(4R)-N-{4-[(14bR)-1,2,3,4,10,14b-Hexahydrodibenzo[c,f]pyrazino[1,2-a]azepin-2-yl]butyl}-2-(3-pyridyl)-thiazolidine-4-carboxamide and its hydrochloride

Following a procedure similar to that described in Example 15, but using (4R)-2-(3-pyridyl)thiazolidine-4-carboxylic acid and (14bR)-2-(4-aminobutyl)-1,2,3,4,10,14b-hexahydrodibenzo[c,f]pyrazino[1,2-a]azepine (prepared as described in Preparation 20), the title compound was obtained in a yield of 66%.

Infrared Absorption Spectrum ($CHCl_3$) $\nu_{max}$ $cm^{-1}$:
3400, 3010, 2950, 2820, 1680, 1525, 1495.

Mass spectrum, m/z (%):
513 ($M^+$, 0.1), 208 (49), 193 (100).

This compound was treated with a 4 N solution of hydrogen chloride in ethyl acetate in the same manner as described in the second step of Example 1, to obtain the hydrochloride of the title compound, melting at 181 - 183 °C (with decomposition).

EXAMPLE 22

(4R)-N-{2-[(14bR)-8-Chloro-1,2,3,4,10,14b-hexahydrodibenzo[c,f]pyrazino[1,2-a]azepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bR)-2-(2-aminoethyl)-8-chloro-1,2,3,4,10,14b-hexahydrodibenzo[c,f]pyrazino[1,2-a]-azepine (prepared as described in Preparation 21), the title compound was obtained in a yield of 63%.

Infrared Absorption Spectrum ($CHCl_3$) $\nu_{max}$ $cm^{-1}$:
3350, 2950, 2850, 1670, 1550, 1480.

Mass spectrum, m/z (%):
519 ($M^+$, 15), 485 (15), 325 (14), 297 (100).

This compound was treated with a 4 N solution of hydrogen chloride in ethyl acetate in the same manner as described in the second step of Example 1, to obtain the hydrochloride of the title compound, melting at 80 - 85 °C.

EXAMPLE 23

(4R)-N-{2-[(14bS)-8-Chloro-1,2,3,4,10-14b-hexahydrodibenzo[c,f]pyrazino[1,2-a]azepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bS)-2-(2-aminoethyl)-8-chloro-1,2,3,4,10,14b-hexahydrodibenzo[c,f]pyrazino[1,2-a]-azepine (prepared as described in Preparation 22), the title compound was obtained in a yield of 57%.

Infrared Absorption Spectrum ($CHCl_3$) $\nu_{max}$ $cm^{-1}$:
3350, 2950, 2850, 1670, 1550, 1480.

Mass spectrum, m/z (%):
519 ($M^+$, 18), 485 (18), 325 (16), 297 (100).

This compound was treated with a 4 N solution of hydrogen chloride in ethyl acetate in the same manner as described in the second step of Example 1, to obtain the hydrochloride of the title compound, melting at 78 - 80 °C.

EXAMPLE 24

(4R)-N-{2-[(14bR)-8-Fluoro-1,2,3,4,10,14b-hexahydrodibenzo[c,f]pyrazino[1,2-a]azepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bR)-2-(2-aminoethyl)-8-fluoro-1,2,3,4,10,14b-hexahydrodibenzo[c,f]pyrazino[1,2-a]-azepine (prepared as described in Preparation 25), the title compound was obtained in a yield of 62%.
Infrared Absorption Spectrum ($CHCl_3$) $\nu_{max}$ $cm^{-1}$:
3380, 2990, 2940, 2810, 1670, 1495, 1445.
Mass spectrum, m/z (%):
503 ($M^+$, 15), 281 (100), 211 (50).
This compound was treated with a 4 N solution of hydrogen chloride in ethyl acetate in the same manner as described in the second step of Example 1, to obtain the hydrochloride of the title compound, melting at 60 - 70 °C.

EXAMPLE 25

(4R)-N-{2-[(14bS)-8-Fluoro-1,2,3,4,10,14b-hexahydrodibenzo[c,f]pyrazino[1,2-a]azepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bS)-2-(2-aminoethyl)-8-fluoro-1,2,3,4,10,14b-hexahydrodibenzo[c,f]pyrazino[1,2-a] azepine (prepared as described in Preparation 26), the title compound was obtained in a yield of 67%.
Infrared Absorption Spectrum ($CHCl_3$) $\nu_{max}$ $cm^{-1}$:
3380, 2990, 2940, 2810, 1670, 1495, 1445.
Mass spectrum, m/z (%):
503 ($M^+$, 16), 281 (100), 211 (43).
This compound was treated with a 4 N solution of hydrogen chloride in ethyl acetate in the same manner as described in the second step of Example 1, to obtain the hydrochloride of the title compound, melting at 75 - 80 °C.

EXAMPLE 26

2-[2-(3-Pyridyl)thiazolidin-4-ylcarbonyl]-1,2,3,4,10,14b-hexahydrodibenzo[c,f]pyrazino[1,2-a]azepine and its hydrochloride

Following a procedure similar to that described in Example 15, but using (4R)-2-(3-pyridyl)thiazolidine-4-carboxylic acid and 1,2,3,4,10,14b-hexahydrodibenzo[c,f]pyrazino[1,2-a]azepine, the title compound was obtained in a yield of 50%.
Infrared Absorption Spectrum ($CHCl_3$) $\nu_{max}$ $cm^{-1}$:
3300, 3000, 2940, 2820, 1645, 1490.
Mass spectrum, m/z (%):
442 ($M^+$, 56), 248 (98), 208 (100).
This compound was treated with a 4 N solution of hydrogen chloride in ethyl acetate in the same manner as described in the second step of Example 1, to obtain the hydrochloride of the title compound, melting at 165 - 167 °C (with decomposition).

EXAMPLE 27

(4R)-N-{2-[(14bR)-1,2,3,4,10,14b-Hexahydropyrazino[2,1-a]pyrido[2,3-c][2]benzoazepin-2-yl]ethyl}-2-(3-pyridyl thiazolidine-4-carboxamide and its hydrochloride

Following a procedure similar to that described in Example 15, but using (4R)-2-(3-pyridyl)thiazolidine-4-carboxylic acid and (14bR)-2-(2-aminoethyl)-1,2,3,4,10,14b-hexahydropyrazino[2,1-a]pyrido[2,3-c][2]-benzoazepine (prepared as described in Preparation 27), the title compound was obtained in a yield of 77%.
Infrared Absorption Spectrum ($CHCl_3$) $\nu_{max}$ $cm^{-1}$:

3350, 2950, 2850, 1670, 1590, 1510, 1440.
Mass spectrum, m/z (%):
486 ($M^+$, 9), 368 (15), 264 (38), 195 (100).

This compound was treated with a 4 N solution of hydrogen chloride in ethyl acetate in the same manner as described in the second step of Example 1, to obtain the hydrochloride of the title compound, melting at 164 - 178 °C.

EXAMPLE 28

(4R)-N-{2-[(14bS)-1,2,3,4,10,14b-Hexahydropyrazino[2,1-a]pyrido[2,3-c][2]benzoazepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bS)-2-(2-aminoethyl)-1,2,3,4,10,14b-hexahydropyrazino[2,1-a]pyrido[2,3-c][2]-benzoazepine (prepared as described in Preparation 28), the title compound was obtained in a yield of 82%.
Infrared Absorption Spectrum ($CHCl_3$) $\nu_{max}$ $cm^{-1}$:
3350, 2950, 2850, 1670, 1590, 1510, 1440.
Mass spectrum, m/z (%):
486 ($M^+$, 1), 264 (38), 195 (100).

This compound was treated with a 4 N solution of hydrogen chloride in ethyl acetate in the same manner as described in the second step of Example 1, to obtain the hydrochloride of the title compound, melting at 117 - 121 °C (with decomposition).

EXAMPLE 29

(4R)-N-{2-[(14bR)-1,2,3,4,10,14b-Hexahydropyrazino[1,2-a]pyrrolo[2,1-c][1,4]benzodiazepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bR)-2-(2-aminoethyl)-1,2,3,4,10,14b-hexahydropyrazino[1,2-a]pyrrolo[2,1-c][1,4]-benzodiazepine (prepared as described in Preparation 24), the title compound was obtained in a yield of 76%.
Infrared Absorption Spectrum ($CHCl_3$) $\nu_{max}$ $cm^{-1}$:
3380, 2990, 2820, 1665, 1515, 1495.
Mass spectrum, m/z (%):
474 ($M^+$, 10), 252 (100), 197 (65).

This compound was treated with a 4 N solution of hydrogen chloride in ethyl acetate in the same manner as described in the second step of Example 1, to obtain the hydrochloride of the title compound, melting at 143 - 145 °C (with decomposition).

EXAMPLE 30

(4R)-N-{2-[(14bS)-1,2,3,4,10,14b-Hexahydropyrazino[1,2-a]pyrrolo[2,1-c][1,4]benzodiazepin-2-yl]ethyl}-2-(3-pyridyl)thiazolidine-4-carboxamide and its hydrochloride

Following a procedure similar to that described in Example 15, but using (4R)-2-(3-pyridyl)thiazolidine-4-carboxylic acid and (14bS)-2-(2-aminoethyl)-1,2,3,4,10,14b-hexahydropyrazino[1,2-a]pyrrolo[2,1-c]-[1,4]-benzodiazepine (prepared as described in Preparation 23), the title compound was obtained in a yield of 42%.
Infrared Absorption Spectrum ($CHCl_3$) $\nu_{max}$ $cm^{-1}$:
3380, 2990, 2820, 1670, 1515, 1495.
Mass spectrum, m/z (%):
474 ($M^+$, 28), 252 (100), 197 (95).

This compound was treated with a 4 N solution of hydrogen chloride in ethyl acetate in the same manner as described in the second step of Example 1, to obtain the hydrochloride of the title compound, melting at 145 - 147 °C (with decomposition).

EXAMPLE 31

1-{2-[(14bR)-1,2,3,4,10,14b-Hexahydrodibenzo[c,f]pyrazino[1,2-a]azepin-2-yl]ethyl}-4-[(4R)-2-(3-pyridyl)-thiazolidin-4-ylcarbonyl]piperazine and its hydrochloride

Following a procedure similar to that described in Example 15, but using (4R)-2-(3-pyridyl)thiazolidine-4-carboxylic acid and 1-{2-[(14bR)-1,2,3,4,10,14b-hexahydrodibenzo[c,f]pyrazino[1,2-a]azepin-2-yl]-ethyl}piperazine (prepared as described in Preparation 29), the title compound was obtained in a yield of 85%.

Infrared Absorption Spectrum ($CHCl_3$) $\nu_{max}$ $cm^{-1}$:

3000, 2950, 2820, 1745, 1595, 1490.

Mass spectrum, m/z (%):

263 (100), 431 (3), 554 (1.6).

This compound was treated with a 4 N solution of hydrogen chloride in ethyl acetate in the same manner as described in the second step of Example 1, to obtain the hydrochloride of the title compound, melting at 208 - 210 °C (with decomposition).

PREPARATION 1

N-[2-(Diphenylmethoxy)ethyl]-N-methyl-N'-methylpropanediamine

A mixture of 3 g (12.2 mmole) of 2-(diphenylmethoxy)ethyl chloride, 5 g (49 mmole) of N,N'-dimethylpropanediamine and 40 ml of toluene was heated under reflux overnight. At the end of this time, the toluene layer was separated and washed with water; 25 ml of a 10% w/v aqueous solution of acetic acid was then added thereto. The aqueous layer was separated, and a 10% w/v aqueous solution of sodium hydroxide was added to make it alkaline. The mixture was then extracted with diethyl ether, and the solvent was removed by distillation under reduced pressure, to obtain 2.4 g (yield 63%) of the title compound as an oil.

Infrared Absorption Spectrum ($CHCl_3$) $\nu_{max}$ $cm^{-1}$:

2920, 2820, 2780, 1490, 1450.

PREPARATION 2

N-[2-(Diphenylmethoxy)ethyl]-N-methyl-N'-methylethylenediamine

Following a procedure similar to that described in Preparation 1, but using 2-(diphenylmethoxy)ethyl chloride and N,N'-dimethylethylenediamine, the title compound was obtained in a yield of 66%.

Infrared Absorption Spectrum ($CHCl_3$) $\nu_{max}$ $cm^{-1}$:

3300, 2930, 2840, 1490, 1450.

PREPARATION 3

N-{2-[bis(4-Fluorophenyl)methoxy]ethyl}-N-methyl-N'-methylpropanediamine

Following a procedure similar to that described in Preparation 1, but using 2-[bis(4-fluorophenyl)-methoxy]ethyl chloride and N,N'-dimethylpropanediamine, the title compound was obtained in a yield of 97%.

Infrared Absorption Spectrum ($CHCl_3$) $\nu_{max}$ $cm^{-1}$:

3270, 3130, 1600, 1505.

PREPARATION 4

1-{2-[α-(4-Chlorophenyl)benzyloxy]ethyl}piperazine

Following a procedure similar to that described in Preparation 1, but using 2-[α-(4-chlorophenyl)-benzyloxy]ethyl chloride and anhydrous piperazine, the title compound was obtained in a yield of 73%.

Infrared Absorption Spectrum ($CHCl_3$) $\nu_{max}$ $cm^{-1}$:

3340, 3150, 2930, 2810, 1490, 1450.

PREPARATION 5

1-[2-(4-Diphenylmethylene-1-piperidinyl)ethyl]piperazine

5(a) 4-(Diphenylmethylene)-1-(2-hydroxyethyl)piperidine

A mixture of 2.0 g (8 mmole) of 4-(diphenylmethylene)piperidine, 0.8 g (9.9 mmole) of 2-chloroethanol, 4.8 g (45.7 mmole) of sodium carbonate, 0.05 g of sodium iodide and 40 ml of methyl isobutyl ketone was heated under reflux overnight. At the end of this time, the reaction mixture was filtered. The filtrate was concentrated by evaporation under reduced pressure, and the resulting residue was subjected to column chromatography through silica gel, using a 20 : 1 by volume mixture of ethyl acetate and triethylamine as the eluent, to obtain the title compound as crystals, melting at 58 - 59 °C, in a yield of 61%.
Infrared Absorption Spectrum ($CHCl_3$) $\nu_{max}$ $cm^{-1}$:
3430, 2940, 1600, 1490, 1440.

5(b) 4-(Diphenylmethylene)-1-(2-chloroethyl)piperidine

A solution of 2.2 g (7.5 mmole) of 4-(diphenylmethylene)-1-(2-hydroxyethyl)piperidine [prepared as described in step (a) above] and 1.6 ml (22 mmole) of thionyl chloride in 20 ml of chloroform was heated under reflux for 1 hour. At the end of this time, the reaction mixture was poured into ice water, and the mixture was made alkaline by the addition of a 10% w/v aqueous solution of sodium hydroxide. The chloroform layer was distilled off, and the residue was subjected to column chromatography through silica gel, using a 5 : 1 by volume mixture of hexane and ethyl acetate as the eluent, to obtain the title compound as crystals, melting at 85 - 86 °C, in a yield of 86%.
Infrared Absorption Spectrum ($CHCl_3$) $\nu_{max}$ $cm^{-1}$:
2940, 2800, 1595, 1490, 1440.

5(c) 1-[2-(4-(Diphenylmethylene)-1-piperidinyl)ethyl]piperazine

A mixture of 2.2 g (7 mmole) of 4-(diphenylmethylene)-1-(2-chloroethyl)piperidine [prepared as described in step (b) above], 4.8 g (55.7 mmole) of anhydrous piperazine and 60 ml of toluene was heated under reflux overnight. At the end of this time, water was added to the reaction mixture, the toluene layer was separated, and a 10% w/v aqueous solution of acetic acid was added to the toluene layer to extract the desired compound. The aqueous layer was separated and made alkaline by the addition of a 10% w/v aqueous solution of sodium hydroxide, after which it was extracted with diethyl ether. The diethyl ether was removed by distillation under reduced pressure, to obtain the title compound as crystals, melting at 73 - 75 °C, in a yield of 86%.
Infrared Absorption Spectrum ($CHCl_3$) $\nu_{max}$ $cm^{-1}$:
3200, 2930, 2810, 1595, 1490, 1440.

PREPARATION 6

1-{2-[α-(4-Methylphenyl)benzyloxy]ethyl}piperazine

Following a procedure similar to that described in Preparation 5(c), but using 2-[α-(4-methylphenyl)-benzyloxy]ethyl chloride and anhydrous piperazine, the title compound was obtained in a yield of 65%.
Infrared Absorption Spectrum ($CHCl_3$) $\nu_{max}$ $cm^{-1}$:
3330, 3150, 2950, 2820, 1490, 1440.

PREPARATION 7

1-[2-(Diphenylmethoxy)ethyl]piperazine

Following a procedure similar to that described in Preparation 5(c), but using 2-(diphenylmethoxy)ethyl chloride and anhydrous piperazine, the title compound was obtained in a yield of 75%.
Infrared Absorption Spectrum ($CHCl_3$) $\nu_{max}$ $cm^{-1}$:
2875, 1630, 1490, 1450, 1320.

PREPARATION 8

1-[2-(1,1-Diphenylethoxy)ethyl]piperazine

8(a) 1-Ethoxycarbonyl-4-[2-(1,1-diphenylethoxy)ethyl]piperazine

A mixture of 1.8 g (9.1 mmole) of α-methylbenzhydrol, 2.0 g (9.1 mmole) of 1-(2-chloroethyl)-4-(ethoxycarbonyl)piperazine, 0.36 g (9.2 mmole) of sodium amide and 20 ml of benzene was heated under reflux overnight. At the end of this time, water was poured onto the reaction mixture, and the mixture was extracted with ethyl acetate. The solvent was then removed from the mixture by distillation under reduced pressure, and the residue was subjected to column chromatography through silica gel, using a 1 : 1 by volume mixture of ethyl acetate and hexane as the eluent, to obtain 2.5 g (yield 75%) of the title compound as an oil.
Infrared Absorption Spectrum ($CHCl_3$) $\nu_{max}$ $cm^{-1}$:
3450, 2970, 1680, 1435.

8(b) 1-[2-(1,1-Diphenylethoxy)ethyl]piperazine

A mixture of 2.6 g (6.8 mmole)of 4-ethoxycarbonyl-1-[2-(1,1-diphenylethoxy)ethyl]piperazine [prepared as described in step (a) above], 3.0 g (5.3 mmole) of potassium hydroxide, 9 ml of water and 30 ml of ethylene glycol was stirred at 160 °C for 2 hours while the water formed was removed by evaporation. The reaction mixture was then poured into ice water, and the mixture was extracted with chloroform, after which it was washed with water. The solvent was then removed by evaporation under reduced pressure, to obtain 1.5 g (yield 71%) of the title compound as an oil.
Infrared Absorption Spectrum ($CHCl_3$) $\nu_{max}$ $cm^{-1}$:
3430, 2930, 1600, 1445.

PREPARATION 9

1-{2-[1-(4-Chlorophenyl)-1-phenylethoxy]ethyl}piperazine

9(a) 4-{2-[1-(4-Chlorophenyl)-1-phenylethoxy]ethyl}-1-(ethoxycarbonyl)piperazine

Following a procedure similar to that described in Preparation 8(a), but using 1-(4-chlorophenyl)-1-phenylethanol and 1-(2-chloroethyl)-4-(ethoxycarbonyl)piperazine, the title compound was obtained in a yield of 89%.
Infrared Absorption Spectrum ($CHCl_3$) $\nu_{max}$ $cm^{-1}$:
2970, 2920, 1680, 1480, 1450, 1430.

9(b) 1-{2-[1-(4-Chlorophenyl)-1-phenylethoxy]ethyl}piperazine

Following a procedure similar to that described in Preparation 8(b), but using 4-{2-[1-(4-chlorophenyl)-1-phenylethoxy]ethyl}-1-(ethoxycarbonyl)piperazine [prepared as described in step (a) above], potassium hydroxide and ethylene glycol, the title compound was obtained in a yield of 77%.
Infrared Absorption spectrum ($CHCl_3$) $\nu_{max}$ $cm^{-1}$:
3330, 3150, 2920, 2820, 1490, 1445.

PREPARATION 10

1-{2-[1,1-Bis(4-fluorophenyl)ethoxy]ethyl}piperazine

Following a procedure similar to that described in Preparation 8(b), but using 4-{2-[1,1-bis(4-fluorophenyl)ethoxy]ethyl}-1-ethoxycarbonylpiperazine [prepared in a similar manner to that described in Preparation 8(a)], potassium hydroxide and ethylene glycol, the title compound was obtained in a yield of 83%.
Infrared Absorption Spectrum ($CHCl_3$) $\nu_{max}$ $cm^{-1}$:
3280, 2970, 2920, 1640, 1510.

PREPARATION 11

1-[2-(α-Cyclohexylbenzyloxy)ethyl]piperazine

Following a procedure similar to that described in Preparation 5(c), but using 2-(α-cyclohexylbenzyl)-ethyl chloride and anhydrous piperazine, the title compound was obtained in a yield of 72%.
Infrared Absorption Spectrum ($CHCl_3$) $\nu_{max}$ $cm^{-1}$:
3330, 3150, 2920, 2840, 1410.

PREPARATION 12

1-{2-[Bis(4-fluorophenyl)methoxy]ethyl}homopiperazine

Following a procedure similar to that described in Preparation 1, but using 2-[bis(4-fluorophenyl)-methoxy]ethyl chloride and homopiperazine, the title compound was obtained in a yield of 88%.
Infrared Absorption Spectrum ($CHCl_3$) $\nu_{max}$ $cm^{-1}$:
3280, 3200, 2970, 1610, 1515.

PREPARATION 13

1-[2-(Diphenylmethoxy)ethyl]homopiperazine

Following a procedure similar to that described in Preparation 1, but using 2-(diphenylmethoxy)ethyl chloride and homopiperazine, the title compound was obtained in a yield of 68%.
Infrared Absorption Spectrum ($CHCl_3$) $\nu_{max}$ $cm^{-1}$:
3360, 3170, 3080, 2930, 1495, 1450.

PREPARATION 14

2-(2-Aminoethyl)-1,2,3,4,10,14b-hexahydrodibenzo[c,f]pyrazino[1,2-a]azepine

14(a) 2-(2-Phthalimidoethyl)-1,2,3,4,10,14b-hexahydrodibenzo[c,f]pyrazino[1,2-a]azepine

A mixture of 1.5 g (5.99 mmole) of 1,2,3,4,10,14b-hexahydrodibenzo[c,f]pyrazino[1,2-a]azepine, 1.68 g (6.61 mmole) of N-(2-bromoethyl)phthalimide, 2.55 g (24 mmole) of sodium carbonate and 30 mg of sodium iodide in 50 ml of methyl isobutyl ketone was heated under reflux overnight. At the end of this time, the reaction mixture was filtered, and the solvent was removed by distillation under reduced pressure. The resulting residue was subjected to column chromatography through silica gel, using a 1 : 1 by volume mixture of ethyl acetate and hexane as the eluent, to obtain 2.18 g (yield 86%) of the title compound, melting at 130 - 132 °C.
Infrared Absorption Spectrum ($CHCl_3$) $\nu_{max}$ $cm^{-1}$:
2950, 2820, 1770, 1710, 1490, 1395.

14(b) 2-(2-Aminoethyl)-1,2,3,4,10,14b-hexahydrodibenzo[c,f]pyrazino[1,2-a]azepine

A mixture of 1.7 g (4.01 mmole) of 2-(2-phthalimidoethyl)-1,2,3,4,10,14b-hexahydrodibenzo[c,f]pyrazino-[1,2-a]azepine [prepared as described in step (a) above], 0.6 g of hydrazine hydrate and 100 ml of ethanol was heated under reflux for 2 hours. At the end of this time, the crystals which had precipitated were filtered off, and the solvent was removed by distillation under reduced pressure, to obtain 1.00 g (yield 89%) of the title compound as an oil.
Infrared Absorption Spectrum ($CHCl_3$) $\nu_{max}$ $cm^{-1}$:
2950, 2810, 1595, 1490, 1450.

PREPARATION 15

(14bR)-2-(2-Aminoethyl)-1,2,3,4,10,14b-hexahydrodibenzo[c,f]pyrazino[1,2-a]azepine

15(a) (14bR)-2-(2-Phthalimidoethyl)-1,2,3,4,10,14b-hexahydrodibenzo[c,f]pyrazino[1,2-a]azepine

Following a procedure similar to that described in Preparation 14(a), but using (14bR)-1,2,3,4,10,14b-hexahydrodibenzo[c,f]pyrazino[1,2-a]azepine, the title compound was obtained in a yield of 97%.
Infrared Absorption Spectrum ($CHCl_3$) $\nu_{max}$ $cm^{-1}$:
2820, 1775, 1710, 1495, 1445, 1400.

15(b) (14bR)-2-(2-Aminoethyl)-1,2,3,4,10,14b-hexahydrodibenzo[c,f]pyrazino[1,2-a]azepine

Following a procedure similar to that described in Preparation 14(b), but using (14bR)-2-(2-phthalimidoethyl)-1,2,3,4,10,14b-hexahydrodibenzo[c,f]pyrazino[1,2-a]azepine [prepared as described in step (a) above], the title compound was obtained in a quantitative yield.
Infrared Absorption Spectrum ($CHCl_3$) $\nu_{max}$ $cm^{-1}$:
2950, 2820, 1600, 1490, 1450.

PREPARATION 16

(14bS)-2-(2-Aminoethyl)-1,2,3,4,10,14b-hexahydrodibenzo[c,f]pyrazino[1,2-a]azepine

16(a) (14bS)-2-(2-Phthalimidoethyl)-1,2,3,4,10,14b-hexahydrodibenzo[c,f]pyrazino[1,2-a]azepine

Following a procedure similar to that described in Preparation 14(a), but using (14bS)-1,2,3,4,10,14b-hexahydrodibenzo[c,f]pyrazino[1,2-a]azepine, the title compound was obtained in a yield of 52%:
Infrared Absorption Spectrum ($CHCl_3$) $\nu_{max}$ $cm^{-1}$:
2950, 2800, 1770, 1710, 1440, 1395.

16(b) (14bS)-2-(2-Aminoethyl)-1,2,3,4,10,14b-hexahydrodibenzo[c,f]pyrazino[1,2-a]azepine

Following a procedure similar to that described in Preparation 14(b), but using (14bS)-2-(2-phthalimidoethyl)-1,2,3,4,10,14b-hexahydrodibenzo[c,f]pyrazino[1,2-a]azepine [prepared as described in step (a) above], the title compound was obtained in a yield of 94%.
Infrared Absorption Spectrum ($CHCl_3$) $\nu_{max}$ $cm^{-1}$:
2950, 1590, 1440, 1330.

PREPARATION 17

(14bR)-2-[2-(N-Methylamino)ethyl]-1,2,3,4,10,14b-hexahydrodibenzo[c,f]pyrazino[1,2-a]azepine

17(a) (14bR)-2-[2-(N-Formylamino)ethyl]-1,2,3,4,10,14b-hexahydrodibenzo[c,f]pyrazino[1,2-a]azepine

A mixture of 1.0 g (3.4 mmole) of (14bR)-2-(2-aminoethyl)-1,2,3,4,10,14b-hexahydrodibenzo[c,f]-pyrazino[1,2-a]azepine [prepared as described in Preparation 15(b)], and 10 ml of ethyl formate was heated under reflux for 8 hours. At the end of this time, the reaction mixture was concentrated by evaporation under reduced pressure, and the crystals which precipitated were subjected to column chromatography through silica gel, using a 1 : 4 by volume mixture of ethanol and ethyl acetate as the eluent, to obtain 1.10 g (a quantitative yield) of the title compound as crystals, melting at 137 - 138 °C.
Infrared Absorption Spectrum ($CHCl_3$) $\nu_{max}$ $cm^{-1}$:
3420, 3020, 2960, 2830, 1685, 1495.

17(b) (14bR)-2-[2-(N-Methylamino)ethyl]-1,2,3,4,10,14b-hexahydrodibenzo[c,f]pyrazino [1,2-a]azepine

A mixture of 0.6 g (1.87 mmole) of (14bR)-2-[2-(N-formylamino)ethyl]-1,2,3,4,10,14b-hexahydrodibenzo-[c,f]pyrazino[1,2-a]azepine [prepared as described in step (a) above], 0.12 g (3.17 mmole) of lithium aluminium hydride and 11 ml of tetrahydrofuran was stirred at room temperature for 1 hour, after which it

was heated under reflux for 3 hours. At the end of this time, 0.6 g of sodium sulphate decahydrate was added to the reaction mixture, after which water was added, and the crystals which precipitated were filtered off. The filtrate was concentrated by evaporation under reduced pressure, to obtain 0.52 g (yield 87%) of the title compound as an oil.
Infrared Absorption Spectrum ($CHCl_3$) $\nu_{max}$ $cm^{-1}$:
2950, 2820, 1600, 1495, 1450.

PREPARATION 18

(14bS)-2-[2-(N-Methylamino)ethyl]-1,2,3,4,10,14b-hexahydrodibenzo[c,f]pyrazino[1,2-a]azepine

18(a) (14bS)-2-[2-(N-Formylamino)ethyl]-1,2,3,4,10,14b-hexahydrodibenzo[c,f]pyrazino[1,2-a]azepine

Following a procedure similar to that described in Preparation 17(a), but using 2-aminoethyl-(14bS)-1,2,3,4,10,14b-hexahydrodibenzo[c,f]pyrazino[1,2-a]-azepine, the title compound was obtained in a yield of 87%.
Infrared Absorption Spectrum ($CHCl_3$) $\nu_{max}$ $cm^{-1}$:
3400, 3000, 2940, 2820, 1680, 1490, 1450.

18(b) (14bS)-2-[2-(N-Methylamino)ethyl]-1,2,3,4,10,14b-hexahydrodibenzo[c,f]pyrazino[1,2-a]azepine

Following a procedure similar to that described in Preparation 17(b), but using (14bS)-2-[2-(N-formylamino)ethyl]-1,2,3,4,10,14b-hexahydrodibenzo[c,f]pyrazino[1,2-a]azepine [prepared as described in step (a) above], the title compound was obtained in a yield of 83%.
Infrared Absorption Spectrum ($CHCl_3$) $\nu_{max}$ $cm^{-1}$:
2940, 2820, 1730, 1600, 1490, 1450.

PREPARATION 19

(14bR -2-(3-Aminopropyl)-1,2,3,4,10,14b-hexahydrodibenzo[c,f]pyrazino[1,2-a]azepine

19(a) (14bR)-2-(3-Phthalimidopropyl)-1,2,3,4,10,14b-hexahydrodibenzo[c,f]pyrazino[1,2-a]azepine

Following a procedure similar to that described in Preparation 14(a), but using (14bR)-1,2,3,4,10,14b-hexahydrodibenzo[c,f]pyrazino[1,2-a]azepine and N-(3-bromopropyl)phthalimide, the title compound was obtained in a quantitative yield.
Infrared Absorption Spectrum ($CHCl_3$) $\nu_{max}$ $cm^{-1}$:
2950, 2820, 1770, 1710, 1490, 1395.

19(b) (14bR)-2-(3-Aminopropyl)-1,2,3,4,10,14b-hexahydrodibenzo[c,f]pyrazino[1,2-a]azepine

Following a procedure similar to that described in Preparation 14(b), but using (14bR)-2-(3-phthalimidopropyl)-1,2,3,4,10,14b-hexahydrodibenzo[c,f]pyrazino[1,2-a]azepine [prepared as described in step (a) above], the title compound was obtained in a quantitative yield.
Infrared Absorption Spectrum ($CHCl_3$) $\nu_{max}$ $cm^{-1}$:
2950, 2810, 1595, 1495.

PREPARATION 20

(14bR)-2-(4-Aminobutyl)-1,2,3,4,10,14b-hexahydrodibenzo[c,f]pyrazino[1,2-a]azepine

20(a) (14bR)-2-(4-Phthalimidobutyl)-1,2,3,4,10,14b-hexahydrodibenzo[c,f]pyrazino[1,2-a]azepine

Following a procedure similar to that described in Preparation 14(a), but using (14bR)-1,2,3,4,10,14b-hexahydrodibenzo[c,f]pyrazino[1,2-a]azepine and N-(4-bromobutyl)phthalimide, the title compound was obtained in a quantitative yield.
Infrared Absorption Spectrum ($CHCl_3$) $\nu_{max}$ $cm^{-1}$:
2950, 2820, 1770, 1710, 1490, 1395.

20(b) (14bR)-2-(4-Aminobutyl)-1,2,3,4,10,14b-hexahydrodibenzo[c,f]pyrazino[1,2-a]azepine

Following a procedure similar to that described in Preparation 14(b), but using (14bR)-2-(4-phthalimidobutyl)-1,2,3,4,10,14b-hexahydrodibenzo[c,f]pyrazino[1,2-a]azepine [prepared as described in step (a), above], the title compound was obtained in a yield of 97%.
Infrared Absorption Spectrum ($CHCl_3$) $\nu_{max}$ $cm^{-1}$:
2930, 2820, 1595, 1490.

PREPARATION 21

(14bR)-2-(2-Aminoethyl)-8-chloro-1,2,3,4,10,14b-hexahydrodibenzo[c,f]pyrazino[1,2-a]azepine

21(a) (14bR)-8-Chloro-2-(2-phthalimidoethyl)-1,2,3,4,10,14b-hexahydrodibenzo[c,f]pyrazino[1,2-a]azepine

Following a procedure similar to that described in Preparation 14(a), but using (14bR)-8-chloro-1,2,3,4,10,14b-hexahydrodibenzo[c,f]pyrazino[1,2-a]azepine and N-(2-bromoethyl)phthalimide, the title compound was obtained in a yield of 47%.
Infrared Absorption Spectrum ($CHCl_3$) $\nu_{max}$ $cm^{-1}$:
2810, 1775, 1710, 1485, 1395.

21(b) (14bR)-2-(2-Aminoethyl)-8-chloro-1,2,3,4,10,14b-hexahydrodibenzo[c,f]pyrazino[1,2-a]azepine

Following a procedure similar to that described in Preparation 14(b), but using (14bR)-8-chloro-2-(2-phthalimidoethyl)-1,2,3,4,10,14b-hexahydrodibenzo[c,f]pyrazino[1,2-a]azepine [prepared as described in step (a) above], the title compound was obtained in a quantitative yield.
Infrared Absorption Spectrum ($CHCl_3$) $\nu_{max}$ $cm^{-1}$:
2950, 2820, 1485, 1450.

PREPARATION 22

(14bS)-2-(2-Aminoethyl)-8-chloro-1,2,3,4,10,14b-hexahydrodibenzo[c,f]pyrazino[1,2-a]azepine

22(a) (14b,S)-8-Chloro-2-(2-phthalimidoethyl)-1,2,3,4,10,14b-hexahydrodibenzo[c,f]pyrazino[1,2-a]azepine

Following a procedure similar to that described in Preparation 14(a), but using (14bS)-8-chloro-1,2,3,4,10,14b-hexahydrodibenzo[c,f]pyrazino[1,2-a]azepine and N-(2-bromoethyl)phthalimide, the title compound was obtained in a yield of 58%.
Infrared Absorption Spectrum ($CHCl_3$) $\nu_{max}$ $cm^{-1}$:
2810, 1775, 1710, 1485, 1395.

22(b) (14bS)-2-(2-Aminoethyl)-8-chloro-1,2,3,4,10,14b-hexahydrodibenzo[c,f]pyrazino[1,2-a]azepine

Following a procedure similar to that described in Preparation 14(b), but using (14bS)-8-chloro-2-(2-phthalimidoethyl)-1,2,3,4,10,14b-hexahydrodibenzo[c,f]pyrazino[1,2-a]azepine [prepared as described in step (a) above], the title compound was obtained in a yield of 41%.
Infrared Absorption Spectrum ($CHCl_3$) $\nu_{max}$ $cm^{-1}$:
2950, 2820, 1485, 1450.

PREPARATION 23

(14bS)-2-(2-Aminoethyl)-1,2,3,4,10,14b-hexahydropyrazino[1,2-a]pyrrolo[2,1-c][1,4]benzodiazepine

23(a) (14bS)-2-(2-Phthalimidoethyl)-1,2,3,4,10,14b-hexahydropyrazino[1,2-a]pyrrolo[2,1-c][1,4]-benzodiazepine

Following a procedure similar to that described in Preparation 14(a), but using (14bS)-1,2,3,4,10,14b-hexahydropyrazino[1,2-a]pyrrolo[2,1-c][1,4]benzodiazepine and N-(2-bromoethyl)phthalimide, the title compound was obtained in a quantitative yield.

Infrared Absorption Spectrum ($CHCl_3$) $\nu_{max}$ $cm^{-1}$:
3000, 2820, 1770, 1710, 1600, 1490, 1395.

23(b) (14bS)-2-(2-Aminoethyl)-1,2,3,4,10,14b-hexahydropyrazino[1,2-a]pyrrolo[2,1-c][1,4]benzodiazepine

Following a procedure similar to that described in Preparation 14(b), but using (14bS)-2-(2-phthalimidoethyl)-1,2,3,4,10,14b-hexahydropyrazino[1,2-a]pyrrolo[2,1-c][1,4]benzodiazepine [prepared as described in step (a) above], the title compound was obtained in a quantitative yield.
Infrared Absorption Spectrum ($CHCl_3$) $\nu_{max}$ $cm^{-1}$:
2980, 2930, 2800, 1600, 1490.

PREPARATION 24

(14bR)-2-(2-Aminoethyl)-1,2,3,4,10,14b-hexahydropyrazino[1,2-a]pyrrolo[2,1-c][1,4]benzodiazepine

24(a) (14bR)-2-(2-Phthalimidoethyl)-1,2,3,4,10,14b-hexahydropyrazino[1,2-a]pyrrolo[2,1-c][1,4]-benzodiazepine

Following a procedure similar to that described in Preparation 14(a), but using (14bR)-1,2,3,4,10,14b-hexahydropyrazino[1,2-a]pyrrolo[2,1-c][1,4]benzodiazepine and N-(2-bromoethyl)phthalimide, the title compound was obtained in a quantitative yield.
Infrared Absorption Spectrum ($CHCl_3$) $\nu_{max}$ $cm^{-1}$:
3000, 2820, 1770, 1690, 1490, 1395.

24(b) (14bR)-2-(2-Aminoethyl)-1,2,3,4,10,14b-hexahydropyrazino[1,2-a]pyrrolo[2,1-c][1,4]benzodiazepine

Following a procedure similar to that described in Preparation 14(b), but using (14bR)-2-(2-phthalimidoethyl)-1,2,3,4,10,14b-hexahydropyrazino[1,2-a]pyrrolo[2,1-c][1,4]benzodiazepine [prepared as described in step (a) above], the title compound was obtained in a yield of 94%.
Infrared Absorption Spectrum ($CHCl_3$) $\nu_{max}$ $cm^{-1}$:
2980, 2930, 2800, 1600, 1490.

PREPARATION 25

(14bR)-2-(2-Aminoethyl)-8-fluoro-1,2,3,4,10,14b-hexahydrodibenzo[c,f]pyrazino[1,2-a]azepine

25(a) (14bR)-2-(2-Phthalimidoethyl)-8-fluoro-1,2,3,4,10,14b-hexahydrodibenzo[c,f]pyrazino[1,2-a]azepine

Following a procedure similar to that described in Preparation 14(a), but using (14bR)-8-fluoro-1,2,3,4,10,14b-hexahydrodibenzo[c,f]pyrazino[1,2-a]azepine and N-(2-bromoethyl)phthalimide, the title compound was obtained in a yield of 93%.
Infrared Absorption Spectrum ($CHCl_3$) $\nu_{max}$ $cm^{-1}$:
2800, 1770, 1705, 1490, 1395.

25(b) (14bR)-2-(2-Aminoethyl)-8-fluoro-1,2,3,4,10,14b-hexahydrodibenzo[c,f]pyrazino[1,2-a]azepine

Following a procedure similar to that described in Preparation 14(b), but using (14bR)-2-(2-phthalimidoethyl)-8-fluoro-1,2,3,4,10,14b-hexahydrodibenzo[c,f]pyrazino[1,2-a]azepine [prepared as described in step (a) above], the title compound was obtained in a quantitative yield.
Infrared Absorption Spectrum ($CHCl_3$) $\nu_{max}$ $cm^{-1}$:
2940, 2800, 1595, 1495, 1445.

PREPARATION 26

(14bS)-2-(2-Aminoethyl)-8-fluoro-1,2,3,4,10,14b-hexahydrodibenzo[c,f]pyrazino[1,2-a]azepine

26(a) (14bS)-2-(2-Phthalimidoethyl)-8-fluoro-1,2,3,4,10,14b-hexahydrodibenzo[c,f]pyrazino[1,2-a]azepine

Following a procedure similar to that described in Preparation 14(a), but using (14bS)-8-fluoro-1,2,3,4,10,14b-hexahydrodibenzo[c,f]pyrazino[1,2-a]azepine and N-(2-bromoethyl)phthalimide, the title compound was obtained in a yield of 91%.
Infrared Absorption Spectrum ($CHCl_3$) $\nu_{max}$ $cm^{-1}$:
2805, 1770, 1705, 1490, 1395.

26(b) (14bS)-2-(2-Aminoethyl)-8-fluoro-1,2,3,4,10,14b-hexahydrodibenzo[c,f]pyrazino[1,2-a]azepine

Following a procedure similar to that described in Preparation 14(b), but using (14bS)-2-(2-phthalimidoethyl)-8-fluoro-1,2,3,4,10,14b-hexahydrodibenzo[c,f]pyrazino[1,2-a]azepine [prepared as described in step (a) above], the title compound was obtained in a yield of 98%.
Infrared Absorption Spectrum ($CHCl_3$) $\nu_{max}$ $cm^{-1}$:
2940, 2805, 1595, 1490, 1445.

PREPARATION 27

(14bR)-2-(2-Aminoethyl)-1,2,3,4,10,14b-hexahydropyrazino[2,1-a]pyrido[2,3-c][2]benzoazepine

27(a) (14bR)-2-(2-Phthalimidoethyl)-1,2,3,4,10,14b-hexahydropyrazino[2,1-a]pyrido[2,3-c][2]benzoazepine

Following a procedure similar to that described in Preparation 14(a), but using (14bR)-1,2,3,4,10,14b-hexahydropyrazino[2,1-a]pyrido[2,3-c][2]benzoazepine and N-(2-bromoethyl)phthalimide, the title compound was obtained in a yield of 47%.
Infrared Absorption Spectrum ($CHCl_3$) $\nu_{max}$ $cm^{-1}$:
2800, 1770, 1710, 1590, 1440, 1395.

27(b) (14bR)-2-(2-Aminoethyl)-1,2,3,4,10,14b-hexahydropyrazino[2,1-a]pyrido[2,3-c][2]benzoazepine

Following a procedure similar to that described in Preparation 14(b), but using (14bR)-2-(2-phthalimidoethyl)-1,2,3,4,10,14b-hexahydropyrazino[2,1-a]pyrido[2,3-c][2]benzoazepine [prepared as described in step (a) above], the title compound was obtained in a quantitative yield.
Infrared Absorption Spectrum ($CHCl_3$) $\nu_{max}$ $cm^{-1}$:
2950, 2810, 1590, 1495, 1460, 1440.

PREPARATION 28

(14bS)-2-(2-Aminoethyl)-1,2,3,4,10,14b-hexahydropyrazino[2,1-a]pyrido[2,3-c][2]benzoazepine

28(a) (14bS)-2-(2-Phthalimidoethyl)-1,2,3,4,10,14b-hexahydropyrazino[2,1-a]pyrido[2,3-c][2]benzoazepine

Following a procedure similar to that described in Preparation 14(a), but using (14bS)-1,2,3,4,10,14b-hexahydropyrazino[2,1-a]pyrido[2,3-c][2]benzoazepine and N-(2-bromoethyl)phthalimide, the title compound was obtained in a yield of 48%.
Infrared Absorption Spectrum ($CHCl_3$) $\nu_{max}$ $cm^{-1}$:
2805, 1770, 1710, 1590, 1440, 1395.

28(b) (14bS)-2-(2-Aminoethyl)-1,2,3,4,10,14b-hexahydropyrazino[2,1-a]pyrido[2,3-c][2]benzoazepine

Following a procedure similar to that described in Preparation 14(b), but using (14bS)-2-(2-phthalimidoethyl)-1,2,3,4,10,14b-hexahydropyrazino[2,1-a]pyrido[2,3-c][2]benzoazepine [prepared as described in step (a) above], the title compound was obtained in a yield of 82%.
Infrared Absorption Spectrum ($CHCl_3$) $\nu_{max}$ $cm^{-1}$:

2950, 2805, 1590, 1490, 1460, 1440.

PREPARATION 29

1,{2-[(14bR)-1,2,3,4,10,14b-Hexahydrodibenzo[c,f]pyrazino[1,2-a]azepin-2-yl]ethyl}piperazine

29(a) (14bR)-2-(2-Chloroethyl)-1,2,3,4,10,14b-hexahydrodibenzo[c,f]pyrazino[1,2-a]azepine

A mixture of 3.1 g (10.5 mmole) of (14bR)-2-(2-hydroxyethyl)-1,2,3,4,10,14b-hexahydrodibenzo[c,f]-pyrazino[1,2-a]azepine, 1.5 ml of thionyl chloride and 50 ml of chloroform was heated under reflux for 2 hours. At the end of this time, the reaction mixture was poured into ice water, and sufficient sodium carbonate was added to the mixture to make it alkaline. The resulting mixture was extracted with chloroform, and then the solvent was removed from the extract by evaporation under reduced pressure. The resulting residue was subjected to column chromatography through silica gel, using a 1 : 5 by volume mixture of ethyl acetate and hexane as the eluent, to obtain 2.30 g (yield 70%) of the title compound.
Infrared Absorption Spectrum ($CHCl_3$) $\nu_{max}$ $cm^{-1}$:
3010, 2960, 2820, 1600, 1495, 1450.
Mass spectrum, m/z (%):
312 (46), 220 (31), 193 (100).

29(b) 1-{2-[(14bR)-1,2,3,4,10,14b-Hexahydrodibenzo[c,f]pyrazino[1,2-a]azepin-2-yl]ethyl}piperazine

A mixture of 1.3 g (15.5 mmole) of (14bR)-2-(2-chloroethyl)-1,2,3,4,10,14b-hexahydrodibenzo[c,f]-pyrazino[1,2-a]azepine [prepared as described in step (a) above], 3.6 g of piperazine and 30 ml of toluene was heated under reflux for 16 hours. At the end of this time, the mixture was cooled and water was added. Sufficient of a 10% w/v aqueous solution of acetic acid was then added to make it acidic. The aqueous layer was separated, neutralized with a 10% w/v aqueous solution of sodium hydroxide, and extracted with diethyl ether. The solvent was then removed by evaporation under reduced pressure, to give 1.38 g (yield 92%) of the title compound.
Infrared Absorption Spectrum ($CHCl_3$) $\nu_{max}$ $cm^{-1}$:
2960, 2830, 1600, 1490, 1450.
Mass spectrum, m/z (%):
362 (20), 263 (73), 99 (100).

## Claims

**1.** Compounds of formula (I):

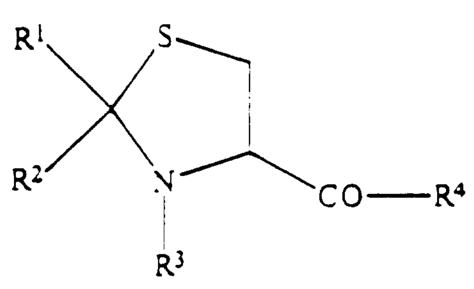

(I)

in which:
$R^1$ represents a pyridyl group which is unsubstituted or is substituted by at least one substituent selected from alkyl groups having from 1 to 4 carbon atoms and alkoxy groups having from 1 to 4 carbon atoms;
$R^2$ represents a hydrogen atom, or a pyridyl group which is unsubstituted or is substituted by at least one substituent selected from alkyl groups having from 1 to 4 carbon atoms and alkoxy groups having from 1 to 4 carbon atoms;

$R^3$ represents a hydrogen atom or an alkyl group having from 1 to 4 carbon atoms;
$R^4$ represents a group of formula (II) or (III):

$-A^1-B-X$ (II)

(wherein X represents

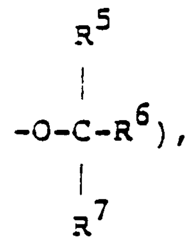

$- A^1-X-X'$ (III),

wherein X' represents

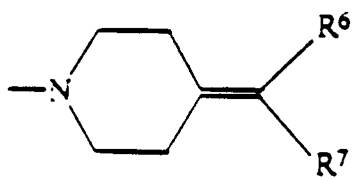

),

in which:
$R^5$ represents a hydrogen atom or an alkyl group having from 1 to 4 carbon atoms;
$R^6$ and $R^7$ are the same or different and each represents:
an unsubstituted phenyl group,
a substituted phenyl group which is substituted by at least one substituent selected from alkyl groups having from 1 to 4 carbon atoms, haloalkyl groups having from 1 to 4 carbon atoms and having at least one halogen atom, alkoxy groups having from 1 to 4 carbon atoms and halogen atoms, or
a cycloalkyl group having from 3 to 6 ring carbon atoms;
$A^1$ represents a group of formula (VII) or (VIII):

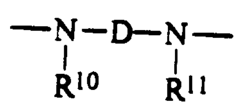

(VII)

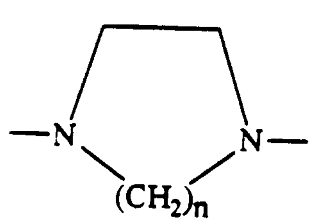

(VIII)

where:
$R^{10}$ and $R^{11}$ are the same or different and each represents a hydrogen atom or an alkyl group having from 1 to 4 carbon atoms;
D represents an alkylene or alkylidene group having from 2 to 5 carbon atoms; and
n is 2 or 3; and
B represents an alkylene or alkylidene group having from 2 to 4 carbon atoms;
and pharmaceutically acceptable salts thereof.

**2.** A compound according to Claim 1, in which $R^1$ represents a pyridyl group.

**3.** A compound according to Claim 1 or Claim 2, in which $R^2$ represents a hydrogen atom, a methyl group or a pyridyl group.

**4.** A compound according to Claim 3, in which $R^2$ represents a hydrogen atom.

**5.** A compound according to any one of Claims 1 to 4, in which $R^3$ represents a hydrogen atom or a methyl group.

**6.** A compound according to Claim 5, in which $R^3$ represents a hydrogen atom.

**7.** A compound according to any one of Claims 1 to 6, in which $A^1$ represents a group of formula (VII) or (VIII), in which $R^{10}$ and $R^{11}$ are the same or different, and each represents a methyl group or an ethyl group; D represents an ethylene, trimethylene or tetramethylene group; and n is 2 or 3.

**8.** A compound according to Claim 7, in which $R^{10}$ and $R^{11}$ are the same and each represents a methyl group; D represents an ethylene group or a trimethylene group; and n is 2.

**9.** A compound according to any one of Claims 1 to 8, in which B represents an ethylene group or a trimethylene group.

**10.** A compound according to Claim 9, in which B represents an ethylene group.

**11.** A compound according to any one of Claims 1 to 10, in which the group of formula X represents a bis-(o-, m- or p- fluorophenyl)methoxy, α-(o-, m- or p- chlorophenyl)benzyloxy, α,α-diphenylmethoxy, α-(o-, m-or p- fluoro- phenyl)benzyloxy, α-(o-, m- or p-methylphenyl)benzyloxy or α-(2-, 3- or 4-pyridyl)-benzyloxy group, or the group of formula X' represents a 4-[α-(2- or 3-thienyl)-α-phenylmethylene]-piperidinyl or 4-[α,α-bis(o-, m- or p- fluorophenyl)methylene]piperidinyl group.

**12.** A compound according to Claim 11, in which the group of formula X represents a bis(4-fluorophenyl)-methoxy, α-(4-chlorophenyl)benzyloxy, α-(4-fluorophenyl)benzyloxy, α-(4-methylphenyl)benzyloxy, diphenylmethoxy or α-(2-pyridyl)benzyloxy group.

**13.** A compound according to Claim 1, in which:
$R^1$ represents a pyridyl group;
$R^2$ represents a hydrogen atom, a methyl group or a pyridyl group;
$R^3$ represents a hydrogen atom or a methyl group;
$A^1$ represents a group of formula (VII) or (VIII), in which $R^{10}$ and $R^{11}$ are the same or different, and each represent a methyl group or an ethyl group; D represents an ethylene, trimethylene or tetramethylene group; and n is 2 or 3;
B represents an ethylene group or a trimethylene group; and
$R^4$ represents a group of formula (II), and the group of formula X represents a bis(o-, m- or p-fluorophenyl)methoxy, α-(o-, m- or p- chlorophenyl)benzyloxy, α,α-diphenylmethoxy, α-(o-, m- or p-fluorophenyl)benzyloxy, α-(o-, m- or p-methylphenyl)benzyl or α-(2-, 3- or 4-pyridyl)benzyloxy group, or $R^4$ represents a group of formula (III) and the group of formula X' represents a 4-[α-(2- or 3- thienyl)-α-phenylmethylene]piperidinyl or 4-[α,α-bis(o-, m- or p-fluorophenyl)methylene]piperidinyl group.

**14.** A compound according to Claim 13, in which the group of formula X represents a bis(4-fluorophenyl)-methoxy, α-(4-chlorophenyl)benzyloxy, α-(4-fluorophenyl)benzyloxy, α(4-methylphenyl)benzyloxy,

diphenylmethoxy or α-(2-pyridyl)benzyloxy group.

**15.** The following compounds according to Claim 1:
N-methyl-N-{3-{N-[2-bis(4-fluorophenyl)methoxyethyl]-N-methylamino}propyl}-2-(3-pyridyl)thiazolidine-4-carboxamide;
N-methyl-N-{3-[N-(2-diphenylmethoxyethyl)-N-methylamino]propyl}-2-(3-pyridyl)thiazolidine-4-carboxamide;
N-methyl-N-{2-[N-(2-diphenylmethoxyethyl)-N-methylamino]ethyl}-2-(3-pyridyl)thiazolidine-4-carboxamide;
1-[2-bis(4-fluorophenyl)methoxyethyl]-4-[2-(3-pyridyl)thiazolidin-4-ylcarbonyl]piperazine;
1-(2-diphenylmethoxyethyl)-4-[2-(3-pyridyl thiazolidin-4-ylcarbonyl]homopiperazine;
1-{2-[bis(4-fluorophenyl)methoxy]ethyl}-4-[2-(3-pyridyl)thiazolidin-4-ylcarbonyl]homopiperazine;
1-{2-[α-(4-methylphenyl)benzyloxy]ethyl}-4-[2-(3-pyridyl)thiazolidin-4-ylcarbonyl]piperazine;
1-[2-(α-cyclohexylbenzyloxy)ethyl]-4-[2-(3-pyridyl)thiazolidin-4-ylcarbonyl]piperazine;
1-(2-diphenylmethoxyethyl)-4-[2-(3-pyridyl)thiazolidin-4-ylcarbonyl]piperazine;
1-{2-[α-(4-chlorophenyl)benzyloxy]ethyl}-4-[2-(3-pyridyl)thiazolidin-4-ylcarbonyl]piperazine;
1-[2-(1,1-diphenylethoxy)ethyl]-4-[2-(3-pyridyl)thiazolidin-4-ylcarbonyl]homopiperazine;
1-{2-[1,1-bis(4-fluorophenyl)ethoxy]ethyl}-4-[2-(3-pyridyl)thiazolidin-4-ylcarbonyl]homopiperazine;
1-[2-(1,1-diphenylethoxy)ethyl]-4-[2-(3-pyridyl)thiazolidin-4-ylcarbonyl]piperazine;
1-{2-[1-(4-chlorophenyl)-1-phenylethoxy]ethyl}-4-[2-(3-pyridyl)thiazolidin-4-ylcarbonyl]piperazine;
1-{2-[1,1-bis(4-fluorophenyl)ethoxy]ethyl}-4-[2-(3-pyridyl)thiazolidin-4-ylcarbonyl]piperazine;
1-{2-[4-(diphenylmethylene)-1-piperidinyl]ethyl}-4-[2-(3-pyridyl)thiazolidin-4-ylcarbonyl]piperazine;
and pharmaceutically acceptable salts thereof.

**16.** A composition for the treatment or prophylaxis of histamine- or PAF- related allergic disorders in a mammal, which comprises an effective amount of an antihistamine or anti-PAF agent in admixture with a pharmaceutically acceptable carrier or diluent, in which the anti-histamine or anti-PAF agent is at least one compound of formula (I) or a pharmaceutically acceptable salt thereof, as claimed in any one of Claims 1 to 15.

**17.** A compound of formula (I) or a pharmaceutically acceptable salt thereof, as claimed in any one of Claims 1 to 15, for use in therapy.

**18.** The use of a compound of formula (I) or a pharmaceutically acceptable salt thereof, as claimed in any one of Claims 1 to 15, for the manufacture of a medicament for the treatment or prophylaxis of histamine-related disorders.

**19.** A process for preparing a compound according to any one of Claims 1 to 15, which comprises reacting a carboxylic acid compound of formula (XII):

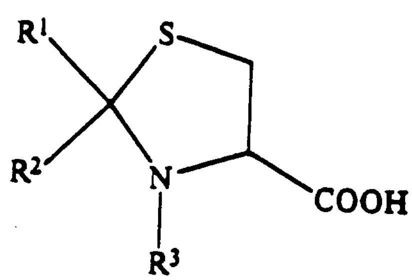

(XII)

(wherein $R^1$, $R^2$ and $R^3$ are as defined in Claim 1) or a reactive derivative thereof with an amine compound of formula (XIII):

$H\text{-}R^4$ (XIII)

(wherein $R^4$ is as defined in Claim 1).

European Patent Office

# EUROPEAN SEARCH REPORT

Application Number
EP 95 11 3591

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A<br>D | EP-A-0 279 681 (YAMANOUCHI PHARMACEUTICAL CO. LTD.)<br>& JP-A-2 000 179 (YAMANOUCHI PHARMACEUTICAL CO. LTD.)<br>--- | 1,16-18 | C07D417/04<br>C07D417/14<br>A61K31/44<br>//(C07D417/04, 277:00,213:00) |
| A | EP-A-0 350 145 (YAMANOUCHI PHARMACEUTICAL CO. LTD.)<br>--- | 1,16-18 | |
| D,A,<br>P | EP-A-0 463 873 (SANKYO COMPANY LIMITED)<br>----- | 1,16-18 | |

| TECHNICAL FIELDS SEARCHED (Int.Cl.5) |
|---|
| C07D<br>A61K |

The present search report has been drawn up for all claims

| Place of search | Date of completion of the search | Examiner |
|---|---|---|
| BERLIN | 12 October 1995 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)